# EUROPEAN PATENT APPLICATION

(11) **EP 2 301 936 A1**
(43) Date of publication of application: **30.03.2011**
(21) Application number: 09766580.6
(22) Date of filing: 11.06.2009
(51) Int. Cl.: C07D 471/20, A61K 31/4427, A61K 31/4439, A61K 31/444, A61K 31/497, A61K 31/498, A61K 31/501, A61K 31/506, A61K 31/517, A61P 1/16, A61P 3/04, A61P 3/06, A61P 3/10, A61P 25/14, A61P 25/22, A61P 25/24, A61P 43/00, C07D 487/10, C07D 519/00

(54) **SPIRODIAMINE-DIARYLKETOXIME DERIVATIVE**

(30) Priority: 19.06.2008 JP 2008160830
(71) Applicant: Banyu Pharmaceutical Co., Ltd., Chiyoda-ku Tokyo 102-8667 (JP)
(72) Inventor: ANDO Makoto, Tokyo 102-8667 (JP); HIROSE Etsuko, Tokyo 102-8667 (JP); MASUTANI Kouta, Tokyo 102-8667 (JP); MORIYA Minoru, Tokyo 102-8667 (JP); SUZUKI Takao, Tokyo 102-8667 (JP)
(74) Representative: Buchan, Gavin MacNicol
(86) International application number: PCT/JP2009/060687
(87) International publication number: WO 2009/154132

(57) **Abstract**

[Problem] To provide a melanin concentrating hormone receptor antagonist useful as medicines for central system disorders, cardiovascular disorders, metabolic disorders.

[Means for Resolution] Provided are compound of a formula (I):

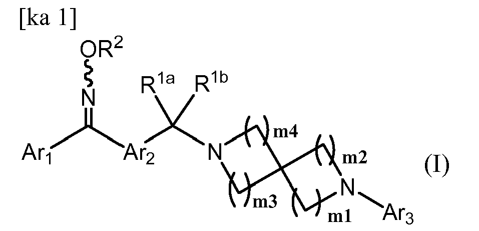

[wherein R^{1 a} and R^{1 b} each are a hydrogen atom, etc.; R² is a hydrogen atom, a C₁₋₆ alkyl, etc.; Ar₁ is a 6-membered aromatic carbocyclic group or a 6-membered aromatic nitrogen-containing heterocyclic group; Ar₂ is a group to be formed by removing two hydrogen atoms from a 6-membered aromatic carbon ring, a 6-membered aromatic nitrogen-containing hetero ring, etc.; Ar₃ is a mono- or bi-cyclic aromatic carbon ring or aromatic hetero ring; m1, m2, m3 and m4 each are independently 0, 1, 2, 3 or 4, provided that the total of m1 and m2 is from 2 to 6, the total of m3 and m4 is from 2 to 6]. The compounds are useful as medicines for central system disorders, cardiovascular disorders, metabolic disorders.

## Description

### TECHNICAL FIELD

The present invention relates to a novel spirodiamine-diaryl ketoxime derivative. The compound acts as a melanin concentrating hormone receptor antagonist, and is useful as a preventive or remedy for various circular system diseases, nervous system diseases, metabolic diseases, genital diseases, respiratory diseases, digestive diseases, etc.

### BACKGROUND ART

Melanin concentrating hormone (hereafter referred to as "MCH") is a cyclic peptide hormone/neuro-peptide, which was for the first time isolated by Kawauchi, et al., in 1983 from sermon hypophysis [see Nature, Vol. 305, 321 (1983)]. The hormone is known to functionally antagonize for melanin cell stimulating hormone in fishes, to cause concentration of melanin granules in melanophore and participate in body color change [see International Review of Cytology, Vol. 126, 1 (1991); Trends in Endocrinology and Metabolism, Vol. 5, 120 (1994)]. Also in mammals, MCH-containing neuron cells are localized in the hypothalamus lateral field and uncertain zone, but their nerve fibers project over a very wide scope in the brain [see The Journal of Comparative Neurology, Vol. 319, 218 (1992)], and MCH is considered to preside over various central functions in living bodies.

Hypothalamus lateral field is known of old as a feeding center, and furthermore, recently, molecular biological and pharmacological knowledges suggesting participation of MCH in controlling energetic homeostasis are much accumulated. That is, it is reported that expression of mRNA, which is an MCH precursor, is accelerated in the brains of ob/ob mice, db/db mice, KKAy mice, Zucker fatty rats which are model animals of hereditary obesity, and in the brains of fasting mice [see Nature, Vol. 380, 243 (1996); Diabetes, Vol. 47, 294 (1998); Biochemical and Biophysical Research Communications, Vol. 268, 88 (2000); Molecular Brain Research, Vol. 92, 43 (2001)].

Acute ventricular administration of MCH to rats was observed to induce accelerated feeding activity [Nature, Vol. 380, 243 (1996)] and chronic administration thereof invites obesity accompanied by polyphagy [see Proceedings of the National Academy of Sciences of the United States of America, Vol. 99, 3240 (2002)]. Moreover, MCH precursor gene-deficient mice show reduced feed ingestion or rise in oxygen consumption per body weight compared to wild type mice, and their low body weight due to decrease in body fat was observed [see Nature, Vol. 396, 670 (1998)].

On the contrary, transgenic mice which express excessive MCH precursor develop obesity accompanied by polyphagy and insulin resistance [see The Journal of Clinical Investigation, Vol. 107, 379 (2001)]. Consequently, it is suggested that MCH is an important factor for developing obesity and participates in diseases induced by metabolic disorders or respiratory diseases for which obesity is one risk factor. Besides, MCH is known to participate also in anxiety-causing action, epilepsy, memory, learning, diuretic action, sodium/potassium excretory action, oxytocin secreting action, reproduction and reproductive function [see Peptides, Vol. 17, 171 (1996); Peptides, Vol. 18, 1095 (1997); Peptides, Vol. 15, 757 (1994); Journal of Neuroendocrinology, Vol. 8, 57 (1996); Critical Reviews in Neurobiology, Vol. 8, 221 (1994)].

MCH causes versatile pharmacological actions through MCH receptors which are present mainly in the central nervous system. As receptors of MCH, at least two types of type 1 receptors (MCH-1R or SLC-1) and type 2 receptors (MCH-2R or SLT) are known [see Nature, Vol. 400, 261 (1999); Nature, Vol. 400, 265 (1999); Biochemical and Biophysical Research Communications, Vol. 261, 622 (1999); Nature Cell Biology, Vol. 1, 267 (1999); FEBS Letters, Vol. 457, 522 (1999); Biochemical and Biophysical Research Communications, Vol. 283, 1013 (2001); The Journal of Biological Chemistry, Vol. 276, 20125 (2001); Proceedings of the National Academy of Sciences of the United States of America, Vol. 98, 7564 (2001); Proceedings of the National Academy of Sciences of the United States of America, Vol. 98, 7576 (2001); The Journal of Biological Chemistry, Vol. 276, 34664 (2001); Molecular Pharmacology, Vol. 60, 632 (2001)].

Of those, the pharmacological action observed on rodents is induced mainly via MCH-1R [see Genomics, Vol. 79, 785 (2002)]. Because MCH-1R gene-deficient mice chronically administered with MCH do not develop polyphagy or obesity, it is known that controlling of energy metabolism by MCH is induced via MCH-1R. Furthermore, the deficiency of MCH-1R is known to promote the activity amount of mice [see Proceedings of the National Academy of Sciences of the United States of America, Vol. 99, 3240 (2002)], and its participation in central system diseases accompanied by behavioral disorders, for example, attention-deficit hyperactivity disorder, schizophrenia, depression and the like also is strongly suggested [see Molecular Medicine Today, Vol. 6, 43 (2000); Trends in Neuroscience, Vol. 24, 527 (2001)].

It is also reported that an autoantibody to MCH-1R is present in serum of vitiligo vulgaris patients [see The Journal of Clinical Investigation, Vol. 109, 923 (2002)]. Furthermore, expression of MCH-1R in certain species of cancer cells was reported, and in vivo MCH and MCH-1R expression sites also suggest MCH's participation in cancer, sleep, vigil, drug dependence and digestive disorders [see Biochemical and Biophysical Research Communications, Vol. 289, 44 (2001); Neuroendocrinology, Vol. 61, 348 (1995); Endocrinology, Vol. 137, 561 (1996); The Journal of Comparative Neurology, Vol. 435, 26 (2001)].

Functions of MCH are expressed upon its binding to MCH receptors. Therefore, when its binding to MCH receptor is inhibited, then expression of MCH action can be inhibited. In consequence, substances which are antagonists for binding of MCH with its receptor are expected to be useful as preventive or remedy for those various diseases in which MCH participates, for example, metabolic disorders such as obesity, diabetes, hormone disorder, hyperlipidemia, gout, fatty liver, etc.; cardiovascular disorders such as stenocardia, acute/congestive heart failure, myocardial infarction, coronary atherosclerosis, hypertension, renal diseases, electrolyte abnormality, etc.; central and peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism, etc.; reproductive disorders such as infertility, preterm labor, sexual dysfunction, etc.; and other digestive disorders, respiratory disorders, cancer or pigmentation.

As compounds having an MCH receptor-antagonistic effect, for example, WO03/004027 (Patent Reference 1) discloses many 4-phenylpiperidine derivatives. However, this patent reference does not disclose at all compounds having a spiro ring.

WO96/26196 (Patent Reference 2) discloses benzylpiperidine derivatives as a muscarinic antagonist. However, this patent reference does not disclose compounds having a spiro ring which is the key point of the present invention. Further, this patent reference has no description relating to MCH receptor-antagonistic effect.
Patent Reference 1: WO03/004027
Patent Reference 2: WO96/26196

### DISCLOSURE OF THE INVENTION

### PROBLEMS THAT THE INVENTION IS TO SOLVE

The present inventors have assiduously studied compounds having an MCH receptor-antagonistic effect and, as a result, have found that a compound in which two aryls bond to the carbon atom that forms an oxime and a spirodiamine skeleton bonds to one aryl via methylene is a novel compound unknown in literature, and that the compound has an MCH receptor-antagonistic effect and is useful for prevention or remedy for MCH receptor-related various diseases, and have completed the present invention.

Specifically, the invention provides the following:
(1) A compound of a formula (I) or a pharmaceutically-acceptable salt thereof:

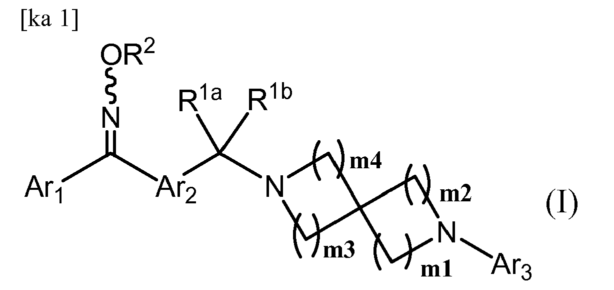

[wherein,
R^{1 a} and R^{1 b} each independently represent a hydrogen atom, or aC₁₋₆ alkyl optionally substituted with a halogen or a hydroxy, or R^{1 a} and R^{1 b}, taken together, form a cyclopropyl;
R² represents a hydrogen atom, a C₁₋₆ alkyl or a C₃₋₆ cycloalkyl, wherein the alkyl or the cycloalkyl may be optionally substituted with a substituent selected from a group consisting of a halogen, a hydroxy, a C₁₋₆ alkyloxy, a C₁₋₆ alkyloxycarbonyl, a C₁₋₆ alkylsulfonyl, a mono(C₁₋₆ alkyl)amino, a di(C₁₋₆ alkyl)amino, a carbamoyl, a mono(C₁₋₆ alkyl)carbamoyl, a di(C₁₋₆ alkyl)carbamoyl and cyano;
Ar₁ represents a 6-membered aromatic carbocyclic group optionally mono- to tetra-substituted with a substituent selected from the group α, or represents a 6-membered aromatic nitrogen-containing heterocyclic group optionally mono- to tetra-substituted with a substituent selected from the group α,
Ar₂ represents a group to be formed by removing two hydrogen atoms from a 6-membered aromatic carbon ring, a 6-membered aromatic nitrogen-containing hetero ring, a 5-membered aromatic hetero ring or a pyridone ring, and wherein the 6-membered aromatic carbon ring, the 6-membered aromatic nitrogen-containing hetero ring, the 5-membered aromatic hetero ring or the pyridone ring may be optionally substituted with a substituent selected from the group α;
Ar₃ represents a mono- or bi-cyclic aromatic carbocyclic group or aromatic heterocyclic group, or a pyridone, wherein the aromatic carbon ring or the aromatic hetero ring may form a fused ring with a non-aromatic cyclic hydrocarbon or a non-aromatic hetero ring, and wherein the aromatic carbocyclic group, the aromatic heterocyclic group or the pyridone may be optionally mono- to tetra-substituted with a substituent selected from a halogen, a C₁₋₆ alkyl, a halo-C₁₋₆ alkyl, a hydroxy-C₁₋₆ alkyl, a C₁₋₆ alkyloxy, a halo-C₁₋₆ alkyloxy, a C₃₋₆ cycloalkyl, a hydroxy-C₃₋₆ cycloalkyl, a cyano, a carbamoyl, a mono-C₁₋₆ alkylcarbamoyl, a di-C₁₋₆ alkylcarbamoyl, a C₁₋₆ alkylsulfonyl and a sulfonylamide;
m1, m2, m3 and m4 each independently indicate 0, 1, 2, 3 or 4, provided that the total of m1 and m2 is from 2 to 6, and the total of m3 and m4 is from 2 to 6, and any -CH₂- forming the spiro ring may be replaced by -O- and/or -C(O)-].

Substituents of group α:
a halogen, a cyano, a hydroxy, an amino, a mono-C₁₋₆ alkylamino, a di-C₁₋₆ alkylamino, a C₁₋₆ alkyl, a halo-C₁₋₆ alkyl, a C₁₋₆ alkyloxy, a halo-C₁₋₆ alkyloxy, a C₁₋₆ alkyloxy-C₁₋₆ alkyl, a C₁₋₆ alkyloxycarbonyl, a C₁₋₆ alkyloxycarbonylamino, a C₁₋₆ alkyloxycarbonyl(C₁₋₆ alkyl)amino, a C₁₋₆ alkylcarbonyl, a C₁₋₆ alkylcarbonyloxy, a C₁₋₆ alkylcarbonylamino, a C₁₋₆ alkylcarbonyl(C₁₋₆ alkyl)amino, a carbamoyl, a mono-C₁₋₆ alkylcarbamoyl, a di-C₁₋₆ alkylcarbamoyl, a carbamoylamino, a mono-C₁₋₆ alkylcarbamoylamino, a di-C₁₋₆ alkylcarbamoylamino, a mono-C₁₋₆ alkylcarbamoyl(C₁₋₆ alkyl)amino, a di-C₁₋₆ alkylcarbamoyl(C₁₋₆ alkyl)amino, a carbamoyloxy, a mono-C₁₋₆ alkylcarbamoyloxy, a di-C₁₋₆ alkylcarbamoyloxy, a C₁₋₆ alkylsulfonyl, a C₁₋₆ alkylsulonylamino, a C₁₋₆ alkylsulfonyl(C₁₋₆ alkyl)amino, a sulfamoyl, a mono-C₁₋₆ alkylsulfamoyl, a di-C₁₋₆ alkylsulfamoyl, a sulfamoylamino, a mono-C₁₋₆ alkylsulfamoylamino, a di-C₁₋₆ alkylsulfamoylamino, a mono-C₁₋₆ alkylsulfamoyl(C₁₋₆ alkyl)amino, and a di-C₁₋₆ alkylsulfamoyl(C₁₋₆ alkyl)amino.

Further, the invention provides the following:
(2) A melanin concentrating hormone receptor antagonist comprising the compound or the pharmaceutically-acceptable salt thereof of (1) as the active ingredient;
(3) A pharmaceutical composition containing a pharmaceutically-acceptable additive and the compound or the pharmaceutically-acceptable salt thereof of (1);
(4) A preventive or remedy comprising the compound or the pharmaceutically-acceptable salt thereof of (1) as the active ingredient, for metabolic disorders such as obesity, diabetes, hormone disorder, hyperlipidemia, gout, fatty liver, hepatitis, and cirrhosis; cardiovascular disorders such as stenocardia, acute/congestive heart failure, myocardial infarction, coronary atherosclerosis, hypertension, renal diseases and electrolyte abnormality; central and peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence and alcoholism; reproductive disorders such as infertility, preterm labor and sexual dysfunction; digestive disorders; respiratory disorders; cancer or pigmentation; especially for bulimia, obesity, diabetes, fatty liver, depression or anxiety;
(5) A pharmaceutical composition based on an MCH1R receptor antagonistic effect, comprising the compound or the pharmaceutically-acceptable salt thereof of (1) as the active ingredient.

The invention is described in more detail hereinunder.

In this description, the term "lower" means that the number of the carbon atoms constituting the group or the compound with the term is at most 6, preferably at most 4.

"C₁₋₆ alkyl" includes a linear alkyl having from 1 to 6 carbon atoms or a branched alkyl having from 3 to 6 carbon atoms, concretely, for example, methyl, ethyl, n-propyl, isopropyl, n-butyl, isobutyl, sec-butyl, tert-butyl, n-pentyl, isopentyl, neopentyl, tert-amyl, 2-propyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl, 1-ethyl-1-methylpropyl, etc.

In the definition of the above substituents, "halogen atom" includes a fluorine atom, a chlorine atom, a bromine atom and an iodine atom.

"C₃₋₆ cycloalkyl " means a cycloalkyl having from 3 to 6 carbon atoms, including cyclopropyl, cyclobutyl, cyclopentyl, cyclohexyl, etc.

"Halo-C₁₋₆ alkyl " includes a C₁₋₆ alkyl in which a part or all of the hydrogen atoms are substituted with halogen, for example, including fluoromethyl, difluoromethyl, trifluoromethyl, 2-fluoroethyl, 1,2-difluoroethyl, etc.

"Hydroxy-C₁₋₆ alkyl " includes a C₁₋₆ alkyl in which a part or all of the hydrogen atoms are substituted with hydroxy, preferably with one or two hydroxyls, for example, including hydroxymethyl, dihydroxymethyl, 2-hydroxyethyl, 2-hydroxymethylpropyl, etc.

"Hydroxy-C₃₋₆ cycloalkyl " includes the above-mentioned cycloalkyl in which a part or all of the hydrogen atoms are substituted with hydroxy, preferably with one or two hydroxyls, for example, including hydroxycyclopropyl, hydroxycyclobutyl, etc.

"C₁₋₆ alkyl optionally substituted with a halogen or a hydroxy" includes the above-mentioned C₁₋₆ alkyl, the above-mentioned halo-C₁₋₆ alkyl, and the above-mentioned hydroxy-C₁₋₆ alkyl.

"C₁₋₆ alkyloxy" includes a group of a C₁₋₆ alkyl bonding to an oxygen atom, concretely including, for example, methoxy, ethoxy, n-propyloxy, isopropyloxy, n-butoxy, n-pentyloxy, etc.

"Halo-C₁₋₆ alkyloxy" includes a group of a halo-C₁₋₆ alkyl bonding to an oxygen atom, concretely including, for example, fluoromethoxy, difluoromethoxy, trifluoromethoxy, 1,2-difluoroethoxy, etc.

"Mono-C₁₋₆ alkylamino" is a group of an amino (-NH₂) in which one hydrogen atom is substituted with a C₁₋₆ alkyl, and concretely includes, for example, methylamino, ethylamino, n-propylamino, isopropylamino, n-butylamino, etc.

"Di-C₁₋₆ alkylamino" is a group of an amino in which two hydrogen atoms are substituted with a C₁₋₆ alkyl, and concretely includes, for example, dimethylamino, diethylamino, ethylmethylamino, di(n-propyl)amino, methyl(n-propyl)amino, diisopropylamino, etc.

"C₁₋₆ alkyloxy-C₁₋₆ alkyl" is a C₁₋₆ alkyl substituted with a C₁₋₆ alkyloxy, and concretely includes, for example, methoxymethyl, ethoxymethyl, n-propyloxymethyl, isopropyloxymethyl, 1-methoxyethyl, 2-methoxyethyl, etc.

"C₁₋₆ alkyloxycarbonyl" is a C₁₋₆ alkyloxy bonding to a carbonyl (-CO-) and includes an alkyloxycarbonyl having from 1 to 6 carbon atoms, concretely, for example, methoxycarbonyl, ethoxycarbonyl, n-propyloxycarbonyl, isopropyloxycarbonyl, n-butoxycarbonyl, etc.

"C₁₋₆ alkyloxycarbonylamino" is a group of an amino in which one hydrogen atom is substituted with a C₁₋₆ alkyloxycarbonyl, and includes an alkyloxycarbonylamino having from 1 to 6 carbon atoms, concretely, for example, methoxycarbonylamino, ethoxycarbonylamino, n-propyloxycarbonylamino, isopropyloxycarbonylamino, n-butoxycarbonylamino, n-pentyloxycarbonylamino, etc.

"C₁₋₆ alkyloxycarbonyl(C₁₋₆ alkyl)amino" is a group of a mono-C₁₋₆ alkylamino in which the hydrogen atom on the nitrogen atom is substituted with a C₁₋₆ alkyloxycarbonyl, and concretely includes, for example, methoxycarbonyl(methyl)amino, ethoxycarbonyl(methyl)amino, n-propyloxycarbonyl(methyl)amino, etc.

"C₁₋₆ alkylcarbonyl" is a group of a C₁₋₆ alkyl bonding to a carbonyl, and includes an alkylcarbonyl having from 1 to 6 carbon atoms, concretely, for example, acetyl, propionyl, butyryl, isobutyryl, valeryl, isovaleryl, pivaloyl, etc.

"C₁₋₆ alkylcarbonyloxy" is a C₁₋₆ alkylcarbonyl bonding to an oxygen atom, and concretely includes, for example, acetoxy, propionyloxy, valeryloxy, isovaleryloxy, pivaloyloxy, etc.

"C₁₋₆ alkylcarbonylamino " is a group of an amino in which one hydrogen atom is substituted with a C₁₋₆ alkylcarbonyl, and concretely includes, for example, acetylamino, propionylamino, isobutyrylamino, valerylamino, isovalerylamino, pivaloylamino, etc.

"C₁₋₆ alkylcarbonyl(C₁₋₆ alkyl)amino " is a mono-C₁₋₆ alkylamino in which the hydrogen atom on the nitrogen atom is substituted with a C₁₋₆ alkylcarbonyl, and includes, for example, methylcarbonyl(methyl)amino, ethylcarbonyl(methyl)amino, n-propylcarbonyl(methyl)amino, etc.

"Mono-C₁₋₆ alkylcarbamoyl" is a carbamoyl (-CONH₂) in which one hydrogen atom is substituted with a C₁₋₆ alkyl, and concretely includes, for example, methylcarbamoyl, ethylcarbamoyl, n-propylcarbamoyl, isopropylcarbamoyl, n-butylcarbamoyl, etc.

"Di-C₁₋₆ alkylcarbamoyl " is a carbamoyl in which two hydrogen atoms are substituted with a C₁₋₆ alkyl, and concretely includes, for example, dimethylcarbamoyl, diethylcarbamoyl, ethylmethylcarbamoyl, di(n-propyl)carbamoyl, methyl(n-propyl)carbamoyl, diisopropylcarbamoyl, etc.

"Mono-C₁₋₆ alkylcarbamoylamino" is an amino in which one hydrogen atom is substituted with a mono-C₁₋₆ alkylcarbamoyl, and concretely includes, for example, methylcarbamoylamino, ethylcarbamoylamino, n-propylcarbamoylamino, isopropylcarbamoylamino, n-butylcarbamoylamino, etc.

"Di-C₁₋₆ alkylcarbamoylamino" is an amino in which one hydrogen atom is substituted with a di-C₁₋₆ alkylcarbamoyl, and concretely includes, for example, dimethylcarbamoylamino, diethylcarbamoylamino, di(n-propyl)carbamoylamino, diisopropylcarbamoylamino, etc.

"Mono-C₁₋₆ alkylcarbamoyl(C₁₋₆ alkyl)amino " is a mono-C₁₋₆ alkylamino in which a hydrogen atom on the nitrogen atom is substituted with a mono-C₁₋₆ alkylcarbamoyl, and concretely includes, for example, monomethylcarbamoyl(methyl)amino, monoethylcarbamoyl(methyl)amino, [mono(n-propyl)carbamoyl](methyl)amino, etc.

"Di-C₁₋₆ alkylcarbamoyl(C₁₋₆ alkyl)amino" is a mono-C₁₋₆ alkylamino in which the hydrogen atom on the nitrogen atom is substituted with a di-C₁₋₆ alkylcarbamoyl, and concretely includes, for example, dimethylcarbamoyl(methyl)amino, diethylcarbamoyl(methyl)amino, [di(n-propyl)carbamoyl](methyl)amino, etc.

"Mono-C₁₋₆ alkylcarbamoyloxy" is a mono-C₁₋₆ alkylcarbamoyl bonding to an oxygen atom, and concretely includes, for example, methylcarbamoyloxy, ethylcarbamoyloxy, n-propylcarbamoyloxy, isopropylcarbamoyloxy, n-butylcarbamoyloxy, etc.

"Di-C₁₋₆ alkylcarbamoyloxy" is a di-C₁₋₆ alkylcarbamoyl bonding to an oxygen atom, and concretely includes, for example, dimethylcarbamoyloxy, diethylcarbamoyloxy, ethylmethylcarbamoyloxy, di(n-propyl)carbamoyloxy, methyl(n-propyl)carbamoyloxy, diisopropylcarbamoyloxy, etc.

"C₁₋₆ alkylsulfonyl " is a C₁₋₆ alkyl bonding to a sulfonyl (-SO₂-), and concretely includes, for example, methanesulfonyl, ethanesulfonyl, n-propanesulfonyl, isopropanesulfonyl, n-butanesulfonyl, etc.

"C₁₋₆ alkylsulfonylamino " is an amino in which one hydrogen atom is substituted with a C₁₋₆ alkylsulfonyl, and concretely includes, for example, methanesulfonylamino, ethanesulfonylamino, n-propanesulfonylamino, isopropanesulfonylamino, n-butanesulfonylamino, etc.

"C₁₋₆ alkylsulfonyl(C₁₋₆ alkyl)amino" is a group of a mono-C₁₋₆ alkylamino in which the hydrogen atom on the nitrogen atom is substituted with a C₁₋₆ alkylsulfonyl, and concretely includes, for example, methanesulfonyl(methyl)amino, ethanesulfonyl(methyl)amino, n-propanesulfonyl(methyl)amino, isopropanesulfonyl(methyl)amino, etc.

"Mono-C₁₋₆ alkylsulfamoyl" is a group of a sulfamoyl (-SO₂NH₂) in which one hydrogen atom is substituted with a C₁₋₆ alkyl, and concretely includes, for example, monomethylsulfamoyl, monoethylsulfamoyl, mono(n-propyl)sulfamoyl, monoisopropylsulfamoyl, mono(n-butyl)sulfamoyl, etc.

"Di-C₁₋₆ alkylsulfamoyl " is a group of a sulfamoyl in which two hydrogen atoms are substituted with a C₁₋₆ alkyl, and concretely includes, for example, dimethylsulfamoyl, diethylsulfamoyl, di(n-propyl)sulfamoyl, diisopropylsulfamoyl, di(n-butyl)sulfamoyl, etc.

"Mono-C₁₋₆ alkylsulfamoylamino" is a group of an amino in which one hydrogen atom is substituted with a mono-C₁₋₆ alkylsulfamoyl, and concretely includes, for example, (monomethylsulfamoyl)amino, (monoethylsulfamoyl)amino, [mono(n-propyl)sulfamoyl] amino, (monoisopropylsulfamoyl)amino, [mono(n-butyl)sulfamoyl] amino, etc.

"Di-C₁₋₆ alkylsulfamoylamino" is a group of an amino in which one hydrogen atom is substituted with a di-C₁₋₆ alkylsulfamoyl, and concretely includes, for example, (dimethylsulfamoyl)amino, (diethylsulfamoyl)amino, (ethylmethylsulfamoyl)amino, [di(n-propyl)sulfamoyl]amino, [methyl(n-propyl)sulfamoyl]amino, (diisopropylsulfamoyl)amino, etc.

"Mono-C₁₋₆ alkylsulfamoyl(C₁₋₆ alkyl)amino" is a group of a mono-C₁₋₆ alkylamino in which the hydrogen atom on the nitrogen atom is substituted with a mono-C₁₋₆ alkylsulfamoyl, and concretely includes, for example, monomethylsulfamoyl(methyl)amino, monoethylsulfamoyl(methyl)amino, [mono(n-propyl)sulfamoyl](methyl)amino, etc.

"Di-C₁₋₆ alkylsulfamoyl(C₁₋₆ alkyl)amino " is a group of a mono-C₁₋₆ alkylamino in which the hydrogen atom on the nitrogen atom is substituted with a di-C₁₋₆ alkylsulfamoyl, and concretely includes, for example, dimethylsulfamoyl(methyl)amino, diethylsulfamoyl(methyl)amino, [di(n-propyl)sulfamoyl](methyl)amino, etc.

"Pharmaceutically-acceptable salts" of a compound of formula [I] mean ordinary salts that are acceptable as medicines. Their examples are acid-addition salts to the amine moiety of the compound of formula (I) or acid-addition salts to the nitrogen-containing hetero ring thereof, or base-addition salts to the acidic substituent, if any, of the compound of formula (I).

The acid-addition salts include inorganic acid salts such as hydrochlorides, sulfates, nitrates, phosphates, perchlorates; organic acid salts such as maleates, fumarates, tartrates, citrates, ascorbates, trifluoroacetates; and sulfonates such as methanesulfonates, isothiocyanates, benzenesulfonates, p-toluenesulfonates.

The base-addition salts include alkali metal salts such as sodium salts, potassium salts; alkaline earth metal salts such as calcium salts, magnesium salts; ammonium salts; and organic amine salts such as trimethylamine salts, triethylamine salts, dicyclohexylamine salts, ethanolamine salts, diethanolamine salts, triethanolamine salts, procaine salts, N,N'-dibenzylethylenediamine salts.

For the purpose of more concretely disclosing the compounds of the invention hereinunder, various symbols used in formula [I] are described in detail with reference to their examples.

R^{1a} and R^{1b} are the same or different, each representing a hydrogen atom, or a C₁₋₆ alkyl optionally substituted with a halogen or a hydroxy; or R^{1a} and R^{1b}, taken together, form a cyclopropyl.

Concretely, R^{1a} and R^{1b} are the same or different, each representing a hydrogen atom, methyl, ethyl, n-propyl, hydroxymethyl, chloromethyl, fluoromethyl, etc., preferably a hydrogen atom or methyl.

R² represents a hydrogen atom, a C₁₋₆ alkyl or a C₃₋₆ cycloalkyl, wherein the alkyl or the cycloalkyl may be optionally substituted with a substituent selected from a group consisting of a halogen, a hydroxy, a C₁₋₆ alkyloxy, a C₁₋₆ alkyloxycarbonyl, a C₁₋₆ alkylsulfonyl, a mono(C₁₋₆ alkyl)amino, a di(C₁₋₆ alkyl)amino, a carbamoyl, a mono(C₁₋₆ alkyl)carbamoyl, a di(C₁₋₆ alkyl)carbamoyl and cyano. The C₁₋₆ alkyl or the C₃₋₆ cycloalkyl may be independently substituted with one or more, preferably from 1 to 3 of these substituents.

Concretely, R² include a hydrogen atom, methyl, ethyl, n-propyl, isopropyl, fluoromethyl, difluoromethyl, trifluoromethyl, 2-fluoroethyl, 2-chloroethyl, 2,2-difluoroethyl, 2-methoxyethyl, 2-hydroxyethyl, 2-hydroxy-2-methylpropyl, methoxycarbonylmethyl, carbamoylmethyl, methylcarbamoylmethyl, dimethylcarbamoylmethyl, 2-dimethylaminoethyl, cyanomethyl, cyanoethyl, cyclopropyl, etc.

Preferably, R² is, for example, a hydrogen atom, methyl, ethyl, n-propyl, isopropyl, 2-fluoroethyl, 2,2-difluoroethyl, 2-hydroxyethyl, dimethylcarbamoylmethyl, difluoromethyl, 2-hydroxy-2-methylpropyl, methanesulfonylmethyl or cyanomethyl, more preferably a hydrogen atom, methyl, ethyl, 2-fluoroethyl, difluoromethyl, 2-hydroxy-2-methylpropyl, methanesulfonylmethyl or cyanomethyl.

m1, m2, m3 and m4 each independently indicate 0, 1, 2, 3 or 4, provided that the total of m1 and m2 is from 2 to 6, and the total of m3 and m4 is from 2 to 6, and any -CH₂- forming the spiro ring may be replaced by -O- and/or -C(O)-. That is, when m1, m2, m3 or m4 is from 1 to 4, any -CH₂- may be replaced by -O- and/or -C(O)-.

The total of m1 and m2 is preferably from 2 to 4, and the total of m3 and m4 is preferably from 2 to 3.

In the invention, the group of a formula (A):

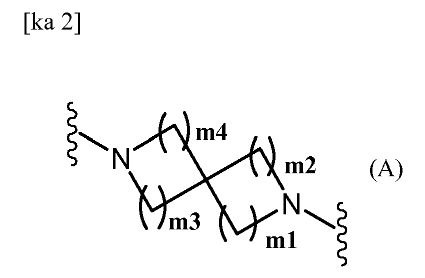

includes, for example, the following:

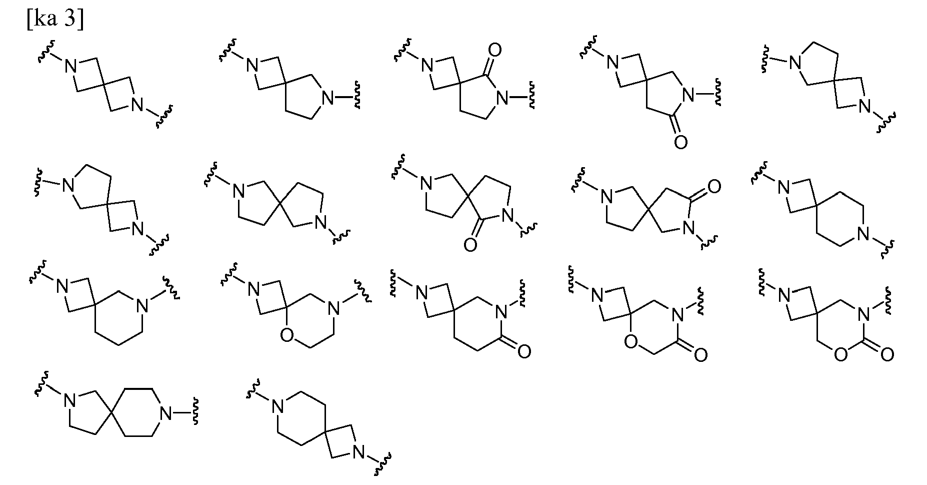

Above all, the following are recommended:

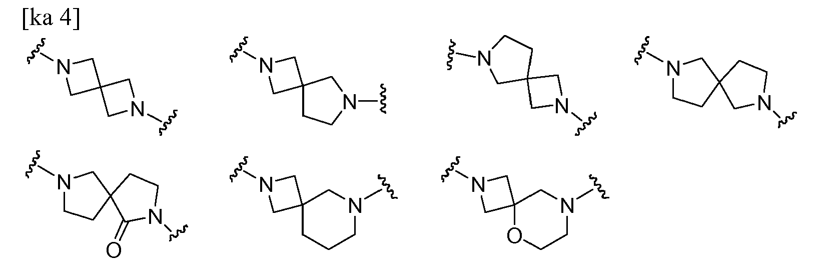

Ar₁ represents a 6-membered aromatic carbocyclic group optionally substituted with a substituent selected from the group α, or represents a 6-membered aromatic nitrogen-containing heterocyclic group optionally substituted with a substituent selected from the group α. Ar₁ may be substituted with the same or different, from 1 to 4, preferably from 1 to 3 substituents selected from the group α.

The 6-membered aromatic carbon ring for Ar₁ includes a benzene ring; and the 6-membered aromatic nitrogen-containing hetero ring includes a pyridine ring, a pyrazine ring, a pyrimidine ring, a pyridazine ring, etc.

The substituent selected from the group α for Ar₁ is preferably a halogen, especially a fluorine atom or a chlorine atom.

Concretely, the 6-membered aromatic carbocyclic group for Ar₁ includes phenyl, 4-fluorophenyl, 3,4-difluorophenyl, 4-chloro-3,5-difluorophenyl, 3,4,5-trifluorophenyl, etc.; and the 6-membered aromatic nitrogen-containing heterocyclic group includes pyridyl, 5-fluoropyridin-2-yl, 5-chloropyridin-2-yl, etc. Preferred is the 6-membered aromatic carbocyclic group substituted with from 1 to 3 fluorine atoms or chlorine atoms, or the 6-membered aromatic nitrogen-containing heterocyclic group substituted with from 1 to 3 fluorine atoms or chlorine atoms; and more preferred is 3,4-difluorophenyl or 5-chloropyridin-2-yl.

Ar₂ represents a group to be formed by removing two hydrogen atoms from a 6-membered aromatic carbon ring, a 6-membered aromatic nitrogen-containing hetero ring, a 5-membered aromatic hetero ring or a pyridone ring, wherein the 6-membered aromatic carbon ring, the 6-membered aromatic nitrogen-containing hetero ring, the 5-membered aromatic hetero ring or the pyridone ring may be optionally substituted with a substituent selected from the group α. Ar₂ may be substituted with the same or different, from 1 to 4, preferably from 1 or 2 substituents selected from the group α.

The 6-membered aromatic carbon ring for Ar₂ includes a benzene ring; the 6-membered aromatic nitrogen-containing heterocyclic group includes a pyridine ring, a pyrazine ring, a pyrimidine ring, a pyridazine ring; and the 5-membered aromatic hetero ring includes a thiophene ring, a thiazole ring, an oxazole ring, a thiadiazole ring, an oxadiazole ring, etc. These may be optionally substituted with the substituent selected from the group α.

The substituent selected from the group α for Ar₂ preferably includes a halogen such as fluorine, chlorine, etc.; a C₁₋₆ alkyl such as methyl, ethyl, etc.; a C₁₋₆ alkyloxy such as methoxy, ethoxy, etc.; a C₁₋₆ alkylsulfonyl such as methanesulfonyl, ethanesulfonyl, etc.

More concretely, Ar₂ preferably includes 1,4-phenylenediyl, 3-methoxyphenylene-1,4-diyl, 3-methanesulfonylphenylene-1,4-diyl, 2-fluorophenylene-1,4-diyl, 3-fluorophenylene-1,4-diyl, 2-methylphenylene-1,4-diyl, pyridine-2,5-diyl, pyrimidine-2,5-diyl, pyrazine-2,5-diyl, pyridazine-3,6-diyl, thiophene-2,5-diyl, pyridonediyl (especially pyridone-3,6-diyl), etc.

Ar₃ represents a mono- or bi-cyclic aromatic carbocyclic group or aromatic heterocyclic group, or a pyridone, wherein the aromatic carbon ring or the aromatic hetero ring may form a fused ring with a non-aromatic cyclic hydrocarbon or a non-aromatic hetero ring, and wherein the aromatic carbocyclic group, the aromatic heterocyclic group or the pyridone may be optionally substituted with the same or different, from 1 to 4 substituents selected from a halogen, a C₁₋₆ alkyl, a halo-C₁₋₆ alkyl, a hydroxy-C₁₋₆ alkyl, a C₁₋₆ alkyloxy, a halo-C₁₋₆ alkyloxy, a C₃₋₆ cycloalkyl, a hydroxy-C₃₋₆ cycloalkyl, a cyano, a carbamoyl, a mono-C₁₋₆ alkylcarbamoyl, a di-C1-6 alkylcarbamoyl, a C₁₋₆ alkylsulfonyl and a sulfonylamide.

The substituent for Ar₃ includes a halogen such as fluorine, chlorine, etc.; a C₁₋₆ alkyl such as methyl, ethyl, n-propyl, isopropyl, n-butyl, t-butyl, etc.; a halo-C₁₋₆ alkyl such as chloromethyl, trichloromethyl, fluoromethyl, trifluoromethyl, etc.; a hydroxy-C₁₋₆ alkyl such as hydroxymethyl, hydroxyethyl, etc.; a C₁₋₆ alkyloxy such as methoxy, ethoxy, n-propyloxy, isopropyloxy, etc.; a halo-C₁₋₆ alkyloxy such as chloromethoxy, fluoromethoxy, trifluoromethoxy, etc.; a C₃₋₆ cycloalkyl such as cyclopropyl, cyclobutyl, cyclopentyl, etc.; a hydroxy-C₃₋₆ cycloalkyl such as hydroxycyclopropyl, hydroxycyclobutyl, etc.; a cyano; a carbamoyl; a mono-C₁₋₆ alkylcarbamoyl such as methylcarbamoyl, ethylcarbamoyl, etc.; a di-C₁₋₆ alkylcarbamoyl such as dimethylcarbamoyl, diethylcarbamoyl, etc.; a C₁₋₆ alkylsulfonyl such as methanesulfonyl, ethanesulfonyl, etc.; and a sulfonylamide. Ar₃ may be substituted with the same or different, from 1 to 4, preferably 1 or 2 these substituents.

The mono- or bi-cyclic aromatic carbon ring includes a benzene ring, a naphthalene ring, etc.

The monocyclic aromatic hetero ring includes a pyridine ring, a pyrazine ring, a pyridazine ring, a pyrimidine ring, a thiadiazole ring, an isothiazole ring, etc.

The bicyclic aromatic hetero ring which may form a fused ring with a non-aromatic cyclic hydrocarbon or a non-aromatic hetero ring includes an imidazopyridine ring, a naphthyridine ring, a quinoxaline ring, a quinazoline ring, etc., and the following:

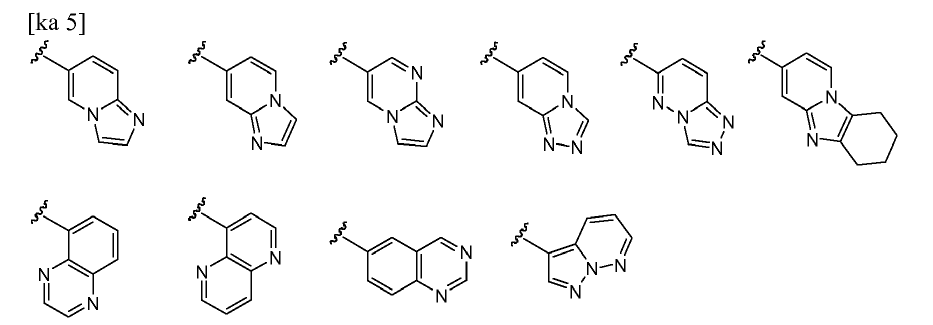

Ar₃ preferably includes the following:

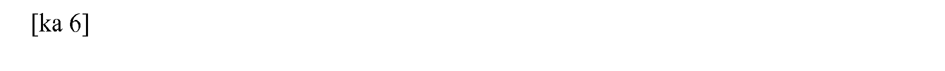

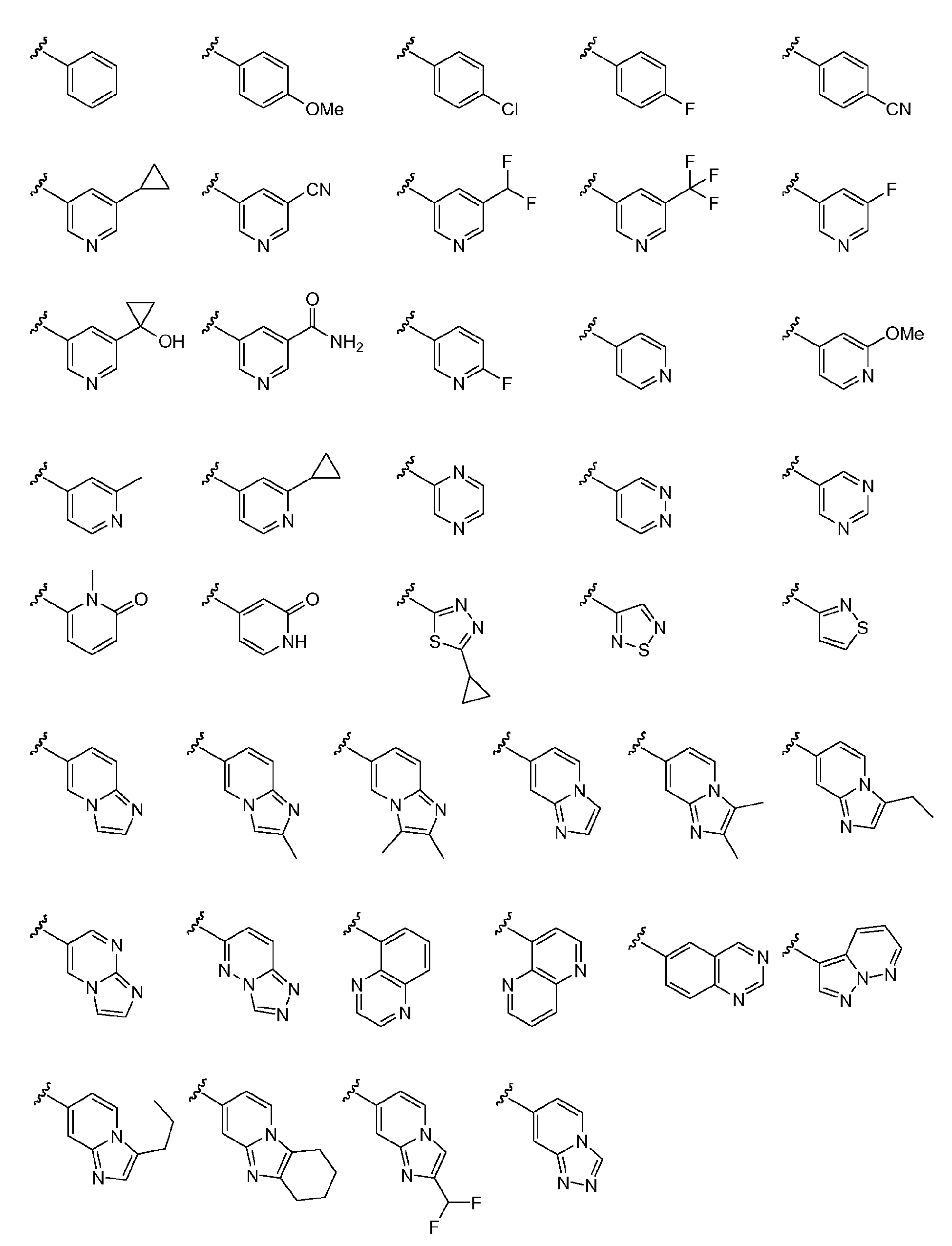

Above all, more preferred are the following:

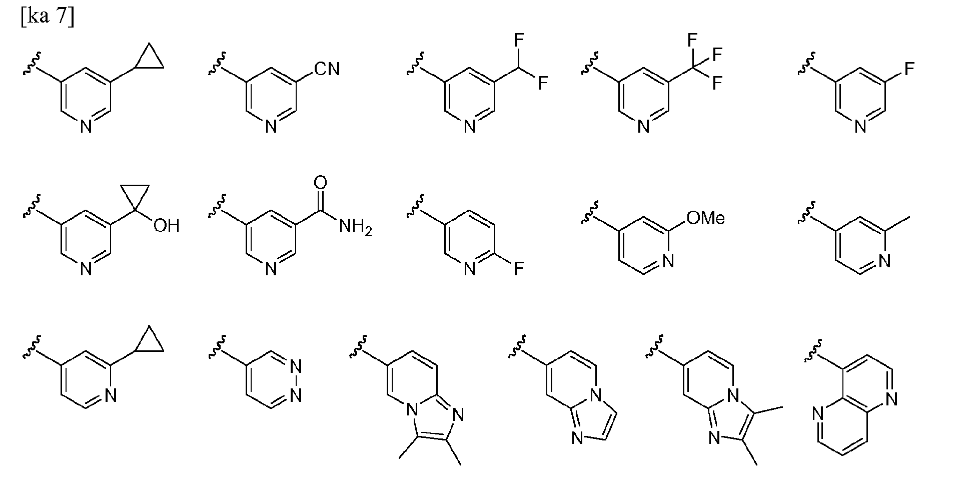

The compounds of formula (I) of the invention may form geometric isomers at the site of =N-OR² therein, as follows. The invention encompasses all isomers, and the structure of formula (I) means that it includes both two. Geometric isomers may be expressed by (E) and (Z), but depending on the type of Ar₁ and Ar₂, the expression of (E) and (Z) may vary. Accordingly, for convenience' sake, the case where Ar₁ and the oxime substituent are on the same side of the double bond is defined as syn; and the case where they are on different sides is defined as anti.

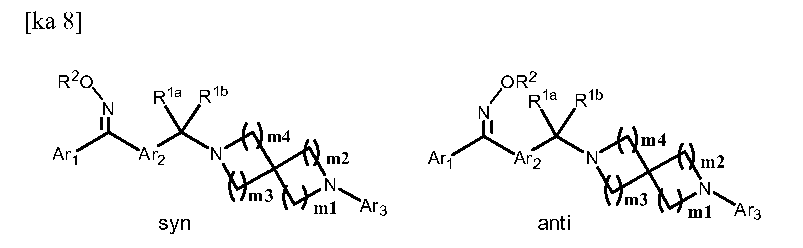

[In the formulae, the symbols are the same as above.]

Further, the invention provides a compound of a formula (I-x):

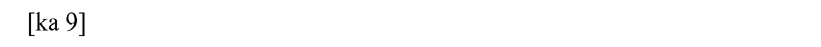

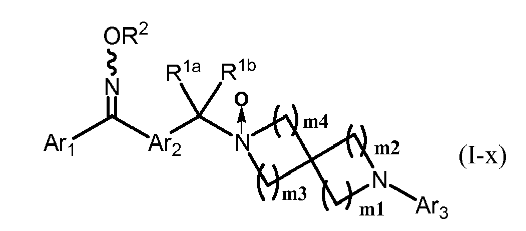

[wherein the symbols are the same as above].

The compound of formula (I-x) can be easily prepared by oxidizing the compound of formula (I).

Of the compounds of formula (I), preferred are those selected from the following group:
(E)-(3,4-difluorophenyl) {5-[(7-phenyl-2,7-diazaspiro[4.4]non-2-yl)methyl]-2-pyridinyl}methanone O-ethyloxime,
5-{6-[(6-{(E)-(3,4-difluorophenyl)[(ethoxy)imino]methyl}-3-pyridinyl)methyl]-2,6-diazaspiro[3.3]hept-2-yl}-3-pyridinecarbonitrile,
(E)-(5-{[6-(2-cyclopropyl-4-pyridinyl)-2,6-diazaspiro]3.3]hept-2-yl]methyl}-2-pyridinyl)-(3,4-difluorophenyl)methanone O-ethyloxime,
(E)-(3,4-difluorophenyl)(5-{[6-(6-fluoro-3-pyridinyl)-2,6-diazaspiro[3.4]oct-2-yl]methyl}-2-pyridinyl)methanone O-ethyloxime,
(E)-(3,4-difluorophenyl)(5-{[2-(6-fluoro-3-pyridinyl)-2,6-diazaspiro[3.4]oct-6-yl]methyl}-2-pyridinyl)methanone O-ethyloxime,
5-(6-{[6-((E)-(3,4-difluorophenyl){[(2-hydroxy-2-methylpropyl)oxy]imino}methyl)-3-pyridinyl]methyl}-2,6-diazaspiro[3.3]hept-2-yl)-3-pyridinecarbonitrile,
(E)-(3,4-difluorophenyl){5-[(6-imidazo[1.2-a]pyridin-7-yl-2,6-diazaspiro[3.3]hept-2-yl)methyl]-2-pyridinyl}methanone O-(2-hydroxy-2-methylpropyl)oxime,
(E)-(3,4-difluorophenyl){5-[(7-pyrazolo[1,5-b]pyridazin-3-yl-2,7-diazaspiro[4.4]non-2-yl)methyl]-2-pyridinyl}methanone O-ethyloxime,
(E)-(3,4-difluorophenyl)(5-{[7-(4-pyridazinyl)-2,7-diazaspiro[4.4]non-2-yl]methyl}-2-pyridinyl)methanone O-ethyloxime,
(E)-(3,4-difluorophenyl)(5-{[6-(3-pyridinyl)-2,6-diazaspiro[3.3]hept-2-yl]methyl}-2-pyridinyl)methanone O-ethyloxime,
(E)-(3,4-difluorophenyl)(5-{[6-(4-pyridinyl)-2,6-diazaspiro[3.3]hept-2-yl]methyl}-2-pyridinyl)methanone O-ethyloxime,
(E)-(3,4-difluorophenyl)(5-{[6-(5-fluoro-3-pyridinyl)-2,6-diazaspiro[3.3]hept-2-yl]methyl}-2-pyridinyl)methanone O-ethyloxime,
(E)-[5-({6-[5-(difluoromethyl)-3-pyridinyl]-2,6-diazaspiro[3.3]hept-2-yl}methyl)-2-pyridinyl](3,4-difluorophenyl)methanone O-ethyloxime,
(E)-(5-{[6-(5-cyclopropyl-3-pyridinyl)-2,6-diazaspiro[3.3]hept-2-yl]methyl}-2-pyridinyl)(3,4-difluorophenyl)methanone O-ethyloxime,
(E)-(3,4-difluorophenyl)[5-({6-[5-(1-hydroxycyclopropyl)-3-pyridinyl]-2,6-diazaspiro[3.3]hept-2-yl}methyl)-2-pyridinyl]methanone O-ethyloxime,
(E)-(3,4-difluorophenyl)(5-{[6-(2-methyl-4-pyridinyl)-2,6-diazaspiro[3.3]hept-2-yl]methyl}-2-pyridinyl)methanone O-ethyloxime,
(E)-(3,4-difluorophenyl)[5-({6-[2-(methyloxy)-4-pyridinyl]-2,6-diazaspiro[3.3]hept-2-yl}methyl)-2-pyridinyl]methanone O-ethyloxime,
(E)-(3,4-difluorophenyl)(5-{[6-(3-isothiazolyl)-2,6-diazaspiro[3.3]hept-2-yl]methyl}-2-pyridinyl)methanone O-ethyloxime,
(E)-(3,4-difluorophenyl)(5-{[6-(1,5-naphthyridin-4-yl)-2,6-diazaspiro[3.3]hept-2-yl]methyl}-2-pyridinyl)methanone O-ethyloxime,
(E)-(3,4-difluorophenyl){5-[(6-imidazo[1,2-a]pyridin-6-yl-2,6-diazaspiro[3.3]hept-2-yl)methyl]-2-pyridinyl}methanone O-ethyloxime,
(E)-(3,4-difluorophenyl)(5-{[6-(2,3-dimethylimidazo[1,2-a]pyridin-6-yl)-2,6-diazaspiro[3.3]hept-2-yl]methyl}-2-pyridinyl)methanone O-ethyloxime,
(E)-(3,4-difluorophenyl){5-[(6-imidazo[1,2-a]pyridin-7-yl-2,6-diazaspiro[3.3]hept-2-ylmethyl]-2-pyridinyl}methanone O-ethyloxime,
(E)-(3,4-difluorophenyl)(5-{[6-(3-propylimidazo[1,2-a]pyridin-7-yl)-2,6-diazaspiro[3.3]hept-2-yl]methyl}-2-pyridinyl)methanone O-ethyloxime,
(E)-(3,4-difluorophenyl)(5-{[6-(6,7,8,9-tetrahydropyrido[1,2-a]benzimidazol-3-yl)-2,6-diazaspiro[3.3]hept-2-yl]methyl}-2-pyridinyl)methanone O-ethyloxime,
5-(6-{[6-((E)-(4-chloro-3,5-difluorophenyl){[(2-hydroxy-2-methylpropyl)oxy]imino}methyl)-3-pyridinyl]methyl}-2,6-diazaspiro[3.3]hept-2-yl)-3-pyridinecarbonitrile,
(E)-(3,4-difluorophenyl)(5-{[(5R) or (5S)-7-(2-methyl-4-pyridinyl)-2,7-diazaspiro[4.4]non-2-yl]methyl}-2-pyridinyl)methanone O-(2-hydroxy-2-methylpropyl)oxime, and
(E)-(3,4-difluorophenyl)(5-{[6-(6-fluoro-3-pyridinyl)-2,6-diazaspiro[3.5]non-2-yl]methyl}-2-pyridinyl)methanone O-ethyloxime.

### Methods for Preparing Compounds of Formula (I)

The compounds of formula [I] may be prepared, for example, according to the following production methods, to which, however, the invention should not be limited.

### Production Method 1-1:

The production method 1-1 is for obtaining a compound of formula (I) starting from a compound of formula (II).

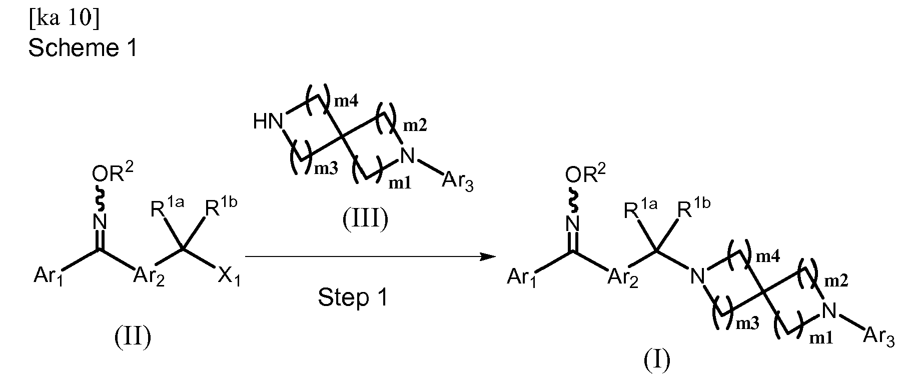

[In the formulae, X1 represents a leaving group such as methanesulfonyloxy, p-toluenesulfonyloxy, halogen, etc.; and the other symbols are the same as above.]

### Step 1:

A compound of formula (II) is reacted with a compound of formula (III) in a reaction solvent, preferably in the presence of a base to give a compound of formula (I).

The amount of the compound of formula (III) to be used may be from 1.0 to 1.5 mols per mol of the compound of formula (II), preferably from 1.0 to 1.3 mols.

The base includes inorganic bases such as sodium carbonate, sodium hydrogencarbonate, potassium carbonate, potassium hydrogencarbonate, lithium carbonate, etc.; organic bases such as triethylamine, diisopropylethylamine, pyridine, etc. The amount of the base to be used may be from 1.0 to 5.0 mols per mol of the compound of formula (II), preferably from 1.1 to 1.5 mols.

The reaction solvent includes halogenohydrocarbons such as methylene chloride, chloroform, carbon tetrachloride, etc.; ethers such as diethyl ether, tetrahydrofuran (hereinafter referred to as "THF"), dioxane, etc.; N,N-dimethylformamide (hereinafter referred to as "DMF"), dimethyl sulfoxide (hereinafter referred to as "DMSO"), etc.

The reaction temperature may be from 0 to 100°C, preferably from 10 to 40°C, and the reaction may complete, generally from 1 to 24 hours.

The compound of formula (II) can be prepared according to the method described in WO2008/038692, and the compound of formula (III) can be prepared according to the method described below.

### Production Method 1-2:

The production method 1-2 is an alternative method for preparing the compound of formula (I).

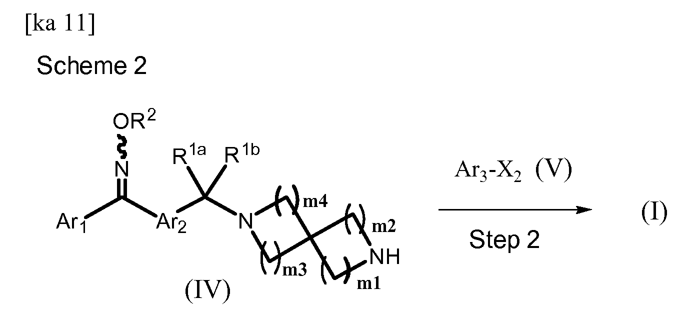

[In the formula, X2 has the same meaning as that of X1, and the other symbols are the same as above.]

### Step 2:

In the presence of a basic catalyst, a ligand and a metal catalyst, a compound of formula (IV) is condensed with a compound of formula (V) in a reaction solvent to give a compound of formula (I).

The amount of the compound of formula (V) to be used may be from 1 to 5 mols per mol of the compound of formula (IV), preferably from 1 to 3 mols.

The basic catalyst includes potassium carbonate, cesium carbonate, potassium phosphate, potassium acetate, sodium t-butoxide, lithium hexamethyldisilazane, etc., preferably sodium t-butoxide, cesium carbonate, etc. The amount of the base to be used may be from 1 to 5 mols per mol of the compound of formula (IV), preferably from 1 to 1.5 mols.

The ligand includes BINAP, tri-t-butyl phosphine, N,N'-dimethylcyclohexanediamine, proline, etc., preferably BINAP, N,N'-dimethylcyclohexanediamine, proline, etc. The amount of the ligand to be used may be from 1 to 0.01 mols per mol of the compound of formula (IV), preferably from 0.1 to 0.01 mols.

The metal catalyst includes Pd₂ dba₃, Pd(dba)₂, Pd(OAc)₂, Cul, etc., preferably Pd₂ dba₃, Pd(OAc)₂, CuI, etc. The amount of the metal catalyst to be used may be from 1 to 0.01 mols per mol of the compound of formula (IV), preferably from 0.1 to 0.01 mols.

The reaction solvent includes dioxane, THF, toluene, xylene, benzene, DMSO, etc.

The reaction temperature may be from room temperature to 160°C, preferably from room temperature to 100°C, and the reaction may complete, generally from 1 to 24 hours.

For promoting the reaction, the reaction system may be irradiated with microwaves.

The compound of formula (IV) can be prepared according to the method mentioned below. The compound of formula (V) includes bromobenzene, 1-bromo-2-fluorobenzene, 4-bromobenzonitrile, 4-bromopyridine, 3-bromo-5-(difluoromethyl)pyridine, 3-bromo-5-(difluoromethyl)pyridine, 3-bromo-5-(trifluoromethyl)pyridine, 3-bromo-5-cyclopropylpyridine, 3-bromo-5-(1-{[(1,1-dimethylethyl)(dimethyl)silyl]oxy}cyclopropyl)pyridine, 2-iodopyrazine, 4-bromopyridazine, 5-bromopyrimidine, 6-bromo-1-methyl-2(1H)-pyridone, 4-iodo-2(1H)-pyridine. 4-bromo-2-methylpyridine, 2-bromo-5-cyclopropyl-1,3,4-thiadiazole, 3-bromo-1,2,5-thiadiazole, 3-bromo-isothiazole, 4-bromo-1,5-naphthyridine, 5-bromoquinoxaline, 7-bromoquinoxaline, 6-bromoimidazo[1,2-a]pyridine, 7-bromoimidazo[1,2-a]pyridine, 7-bromo-2,3-dimethylimidazo[1,2-a]pyridine, 3-bromo-6,7,8,9-tetrahydropyrido[1,2-a]benzimidazole, 7-bromo-2-(difluoromethyl)imidazo[1,2-a]pyridine, 6-bromoimidazo[1,2-a]pyrimidine, 3-bromopyrazolo[1,5-b]pyridazine, etc.

### Production Method 1-3:

The production method 1-3 is another alternative method for preparing the compound of formula (I).

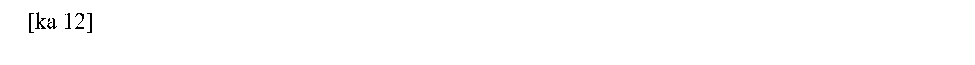

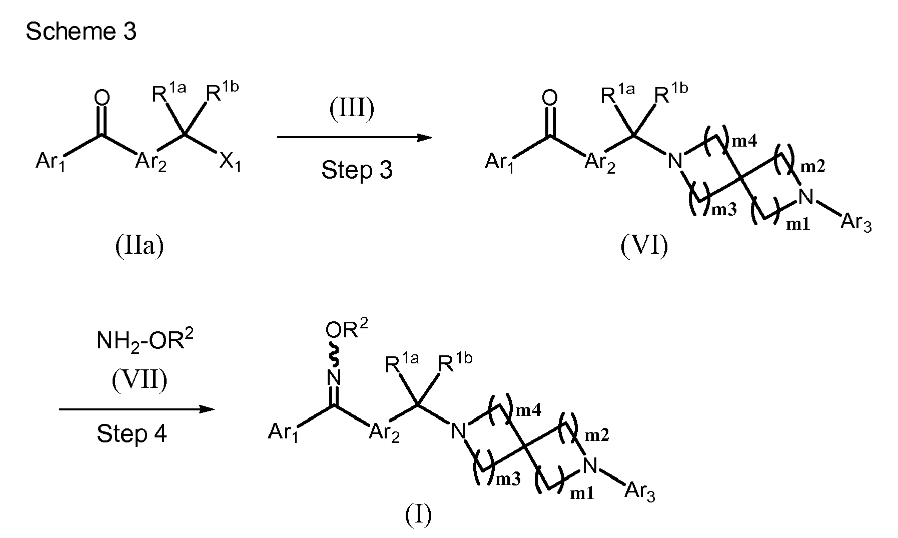

[In the formulae, the symbols are the same as above.]

### Step 3:

A compound of formula (IIa) and a compound of formula (III) are reacted according to the step 1 to give a compound of formula (VI). As the compound of formula (IIa), usable are those described in WO2008/386921 or conventional known compounds.

### Step 4:

The compound of formula (VI) is oximated with a compound of formula (VII) according to a known method (for example, in WO2008/386921) to give a compound of formula (I).

The amount of the compound of formula (VII) to be used may be from 1.0 to 5.0 mols per mol of the compound of formula (VI), preferably from 1.0 to 1.5 mols.

The reaction solvent includes lower alcohols such as methanol, ethanol, n-butanol, isopropyl alcohol, etc., and pyridine, etc.

The reaction temperature may be from 0 to 100°C, preferably from 10 to 30°C, and the reaction may complete, generally from 0.5 to 24 hours.

The compound of formula (VII) includes hydroxylamine hydrochloride, O-methylhydroxylamine hydrochloride, O-ethylhydroxylamine hydrochloride, O-cyclopropylhydroxylamine hydrochloride, O-methylsulfonylhydroxylamine hydrochloride, 2-fluoroethylhydroxylamine hydrochloride, O-(2-hydroxy-2-methylpropyl)hydroxylamine hydrochloride, etc.

### Production Method 2-1:

The production method 2-1 is method for preparing a compound of formula (III).

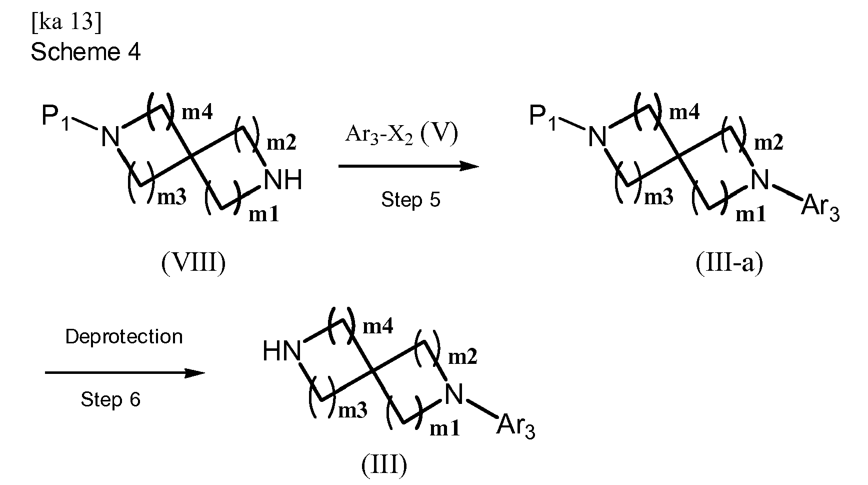

[In the formulae, P1 represents an amino-protective group, and the other symbols are the same as above.]

### Step 5:

A compound of formula (VIII) is reacted with a compound of formula (V) according to the step 2 to give a compound of formula (III-a). As the compound of formula (VIII), usable are conventional known compounds, or the compound may be prepared by introducing a protective group P1 into the corresponding known spirodiamine.

### Step 6:

The protective group in the compound of formula (III-a) is removed to give a compound of formula (III). The deprotection may be attained according to methods described in literature [see Protective Groups in Organic Synthesis, T. W. Greene, John Wiley & Sons (1981)], or according to methods similar thereto.

For example, in case where P1 is a tert-butyloxycarbonyl (t-Boc) group, the compound may be deprotected by treatment with trifluoroacetic acid, hydrochloric acid or the like at room temperature to 100°C, preferably room temperature to 60°C, for 10 minutes to 6 hours, preferably for 0.5 to 2 hours.

### Production Method 2-2:

The production method 2-2 is method for preparing a compound of formula (II) or a compound of formula (IV).

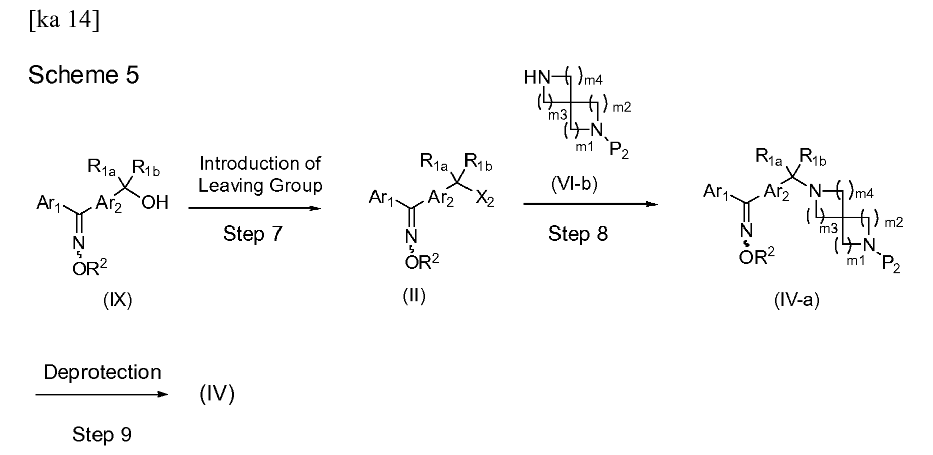

[In the formulae, P2 represents an amino-protective group, and the other symbols are the same as above.]

### Step 7:

A leaving group is introduced into the hydroxyl group of the compound of formula (IX) to give a compound of formula (II). For example, in case where X₂ is a methanesulfonyloxy group, the compound of formula (IX) is reacted with methanesulfonyl chloride in an organic solvent in the presence of a base catalyst to give a compound of formula (II).

The amount of methanesulfonyl chloride to be used may be from 1.0 to 1.5 mols per mol of the compound of formula (IX), preferably from 1.0 to 1.3 mols.

The base includes triethylamine, diisopropylethylamine, pyridine, etc. The amount of the base to be used may be from 0.1 to 3 mols per mol of the compound of formula (IX), preferably from 1.0 to 2.0 mols.

The reaction solvent includes ethyl acetate, methylene chloride, chloroform, THF, DMF, etc.

The reaction temperature may be from 0 to 100°C, preferably from 10 to 40°C, and the reaction may complete, generally from 1 to 24 hours.

### Step 8:

The compound of formula (II) is condensed with a compound of formula (VI-b) to give a compound of formula (IV-a). The reaction may be attained according to the step 1. As the compound of formula (VI-b), usable are conventional known compounds, and the compound may be prepared by introducing a protective group P2 into the corresponding known spirodiamine. The protective group is preferably a t-Boc group, in consideration of the stability of the compound in deprotection.

### Step 9:

The amino-protective group is removed from the compound of formula (IV-a) to give a compound of formula (IV).

The deprotection may be attained according to the step 6.

### Production Method 3-1:

The production method 3-1 is a stereoselective production method for a compound of formula (II).

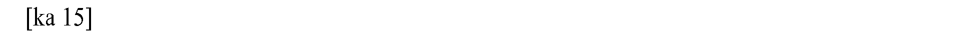

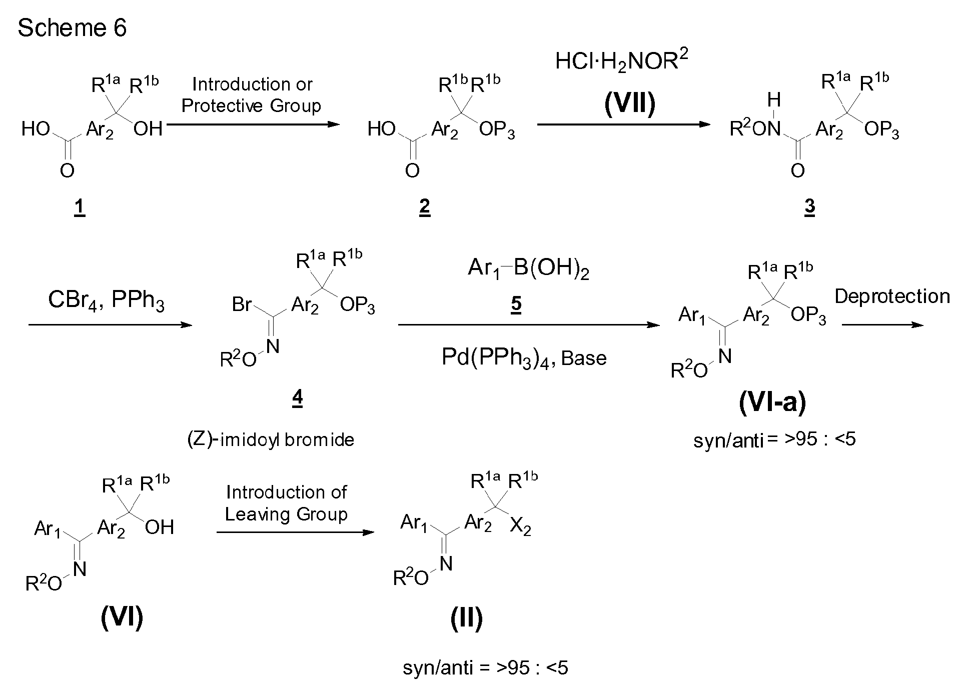

[In the formulae, P3 represents a hydroxy-protective group, and the other symbols are the same as above.]

The hydroxyl group on the compound 1 is protected according to a known method (Protective Groups in Organic Synthesis) to give a compound 2. The protective group includes an acetyl group, a t-butyldimethylsilyl group, etc.

Next, the compound 2 is reacted with a compound of formula (VII) in an alcohol such as methanol, ethanol or the like at 0 to 100°C, preferably at 10 to 30°C to give a compound 3. Next, the compound 3 is reacted under reflux in the presence of tetrabromomethane and triphenyl phosphine in a solvent such as acetonitrile or the like to give a compound 4.

The amount oftetrabromomethane to be used may be from 1.5 to 3.0 mols pre mol of the compound 3, preferably 1.5 mol; and the amount of triphenyl phosphine to be used may be from 1.5 to 2.0 mols per mol of the compound 3, preferably 2.0 mols.

The obtained compound 4 is reacted with a compound 5 in a solvent such as toluene or the like in the presence of tetrakis(triphenylphosphine)palladium and a base to give a compound of formula (VI-a). The compound of formula (VI-a) is produced, still holding the stereochemical configuration of the compound 4 (imidoyl bromide 4).

The base includes sodium carbonate, potassium carbonate, sodium hydrogencarbonate, sodium hydroxide, potassium hydroxide, etc.

The amount of the compound 5 to be used may be from 1.5 to 2.0 mols per mol of the compound 4, preferably 2.0 mols.

Regarding the amount of tetrakis(triphenylphosphine)palladium and the base to be used, the amount of tetrakis(triphenylphosphine)palladium may be from 0.05 to 0.10 mols per mol of the compound 4, preferably 0.05 mols, and the base may be from 1.5 to 2.0 mols, preferably 2.0 mols.

Next, the hydroxy-protective group P3 in the compound of formula (VI-a) is removed according to a known method to give a compound of formula (VI), and then the hydroxy group in the compound of formula (VI) is converted into a leaving group X2 (e.g., methanesulfonyloxy group, p-toluenesulfonyloxy group, halogen atom, etc.) according to a known method to give a compound of formula (II).

The compound 1 includes the following:

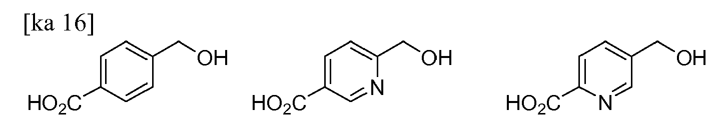

The compound 5 includes the following:

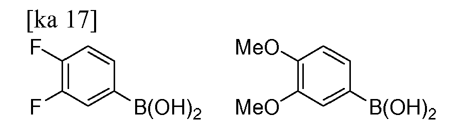

### Production Method 3-2:

The production method 3-2 is a stereoselective production method for a compound of formula (IX).

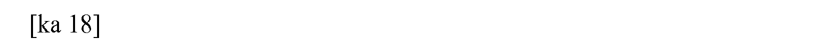

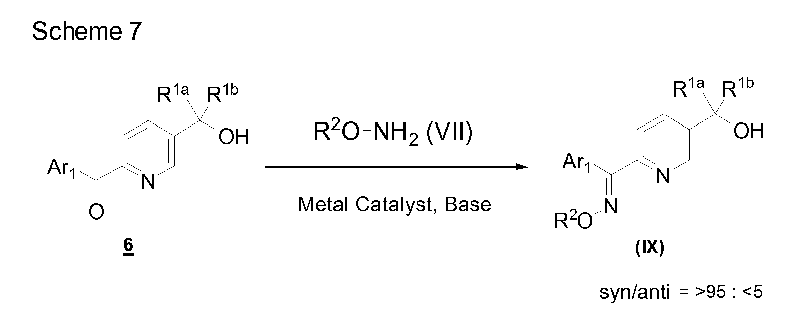

[In the formulae, the symbols are the same as above.]

A compound 6 is reacted with a compound of formula (VII) in an organic solvent in the presence of a base and a metal catalyst to give syn-selectively a compound of formula (II).

The amount of the compound of formula (VII) to be used may be from 1.0 to 5.0 mols per mol of the compound 6, preferably from 2.0 to 3.0 mols.

The base includes sodium methoxide, etc.; and the amount of the base to be used may be from 1.0 to 5.0 mols per mol of the compound 6, preferably from 2.0 to 3.0 mols.

The metal catalyst includes copper(I) triflate-toluene complex, copper(I) oxide, zinc(II) triflate, etc.; and the amount of the metal catalyst to be used may be from 5.0 to 0.001 mols per mol of the compound 6, preferably from 1.0 to 0.005 mols.

The organic solvent includes methanol, ethanol, etc.

The reaction temperature may be from 0 to 100°C, preferably from 10 to 50°C, more preferably from 20 to 30°C, and the reaction may complete, generally from 24 to 72 hours.

As the compound 6, usable are commercial products.

In the above-mentioned production methods where the reactant substances have an amino, imino, hydroxyl, carboxyl, oxo group, carbonyl or the like group not participating in the reaction, the amino, hydroxyl, carboxyl, oxo and carbonyl groups may be suitably protected with an amino-protective group, a hydroxy-protective group, a carboxyl-protective group, or an oxo or carbonyl-protective group, then the reaction in the production method is attained, and after the reaction, the protective group may be removed.

The introduction and the removal of the protective group, through differing depending on the type of the protective group and the stability of the product compound, may be attained, for example, through solvolysis with acid or base, for example, according to the methods described in the above-mentioned Protective Groups in Organic Synthesis, or according to methods similar thereto, concretely, for example, according to a method of treating with from 0.01 mol to a large excessive amount of an acid, preferably trifluoroacetic acid, formic acid, hydrochloric acid or the like, or with from an equimolar amount to a large excessive amount of a base, preferably potassium hydroxide, calcium hydroxide or the like; or through chemical reduction with a metal hydride complex or the like, or through catalytic reduction with a palladium-carbon catalyst, a Raney-nickel catalyst or the like.

Not specifically limited, the amino and imino-protective group may be any one having its function, and includes, for example, an aralkyl such as benzyl, p-methoxybenzyl, 3,4-dimethoxybenzyl, benzhydryl, trityl, etc.; a lower alkanoyl such as formyl, acetyl, pivaloyl, etc.; benzoyl; an arylalkanoyl such as phenylacetyl, etc.; a lower alkyloxycarbonyl such as methoxycarbonyl, ethoxycarbonyl, tert-butoxycarbonyl, etc.; an alkyloxycarbonyl such as benzyloxycarbonyl, etc.; a lower alkylsilyl such as trimethylsilyl, tert-butyldimethylsilyl, etc.; tetrahydropyranyl; trimethylsilylethoxymethyl; a lower alkylsulfonyl such as methylsulfonyl, ethylsulfonyl, etc; an arylsulfonyl such as benzenesulfonyl, toluenesulfonyl, etc. Especially preferred are acetyl, benzoyl, tert-butoxycarbonyl, trimethylsilylethoxymethyl, methylsulfonyl, etc.

Not specifically limited, the hydroxyl-protective group may be any one having its function, and includes, for example, a lower alkyl such as methyl, ethyl, tert-butyl, etc.; a lower alkylsilyl such as trimethylsilyl, tert-butyldimethylsilyl, etc.; a lower alkyloxymethyl such as methoxymethyl, 2-methoxyethoxymethyl, etc.; tetrahydropyranyl; trimethylsilylethoxymethyl; an aralkyl such as benzyl, p-methoxybenzyl, 2,3-dimethoxybenzyl, trityl, etc.; an acyl such as formyl, acetyl, etc. Especially preferred are methyl, methoxymethyl, tetrahydropyranyl, trityl, trimethylsilylethoxymethyl, tert-butyldimethylsilyl, acetyl, etc.

Not specifically limited, the carboxyl-protective group may be any one having its function, and includes, for example, a lower alkyl such as methyl, ethyl, tert-butyl, etc.; a halo-lower alkyl such as 2,2,2-trichloroethyl, etc.; a lower alkenyl such as 2-propenyl, etc.; an aralkyl such as benzyl, p-methoxybenzyl, benzhydryl, trityl, etc. Especially preferred are methyl, ethyl, tert-butyl, 2-propenyl, benzyl, p-methoxybenzyl, benzhydryl, etc.

Not specifically limited, the carbonyl-protective group may be any one having its function, and includes, for example, acetals and ketals such as ethylene ketal, dimethyl ketal, S,S'-dimethyl ketal, etc.

The compounds of formula (I) obtained in the manner as above may be readily isolated and purified in any ordinary separation method of, for example, solvent extraction, recrystallization, column chromatography, preparative thin-layer chromatography or the like.

The compounds may be formed into pharmaceutically-acceptable salts thereof in an ordinary manner, or on the contrary, the salts may be converted into free compounds in an ordinary manner.

The effect of the compounds of the invention as an MCH receptor antagonist is shown, for example, by the following pharmacological test example.

### Pharmacological Test Example: MCH binding inhibition test

A human MCH-1R encoding cDNA sequence [FEBS Letters, Vol. 398, 253 (1996); Biochimica et Biophisica Acta, Vol. 1401, 216 (1998)] was cloned to a plasmid vector pEF/myc/cyto (by Invitrogen Corporation). The obtained expression vector was transfected to host cells CHO-K1 (American Type Culture Collection) using Lipofectamine Plus Reagent (by Life Technology Inc.) to provide MCH-1R expression cells.

Membrane samples prepared from the MCH-1R expression cells were incubated with each test compound and 50 pM of [¹²⁵I]MCH (by NEN Co.), in an assay buffer (50 mM Tris buffer comprising 10 mM magnesium chloride, 2 mM ethylenediamine tetraacetate, 0.01 % bacitracin and 0.2 % bovine serum albumin; pH 7.4) at 25°C for an hour, followed by filtration through a glass filter GF/C (by Wattman Co.). After washing the glass filter with 50 mM Tris buffer (pH 7.4) comprising 10 mM magnesium chloride, 2 mM ethylenediamine tetraacetate and 0.04 % Tween-20, the radioactive activity on the glass filter was measured. The non-specific binding was measured in the presence of 1 µM human MCH, and the 50 % inhibition concentration (IC₅₀ value) of each test compound to the specific [¹²⁵I]MCH binding was determined. The results are shown in Table 1.

**[Table 1]**

| Example | Structure | **IC50(**n**M)** |
|---|---|---|
| **1-10** | 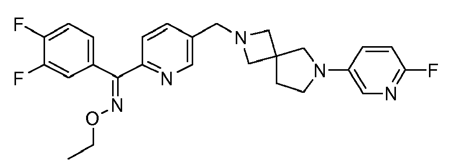 | **7.6** |
| **2-3** | 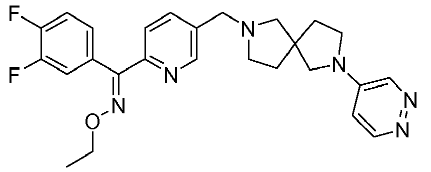 | **3.2** |
| **2-24** | 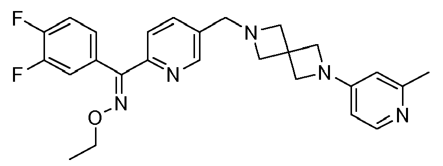 | **2.4** |
| **2-40** | 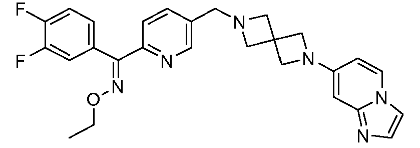 | **0.90** |
| **2-44** | 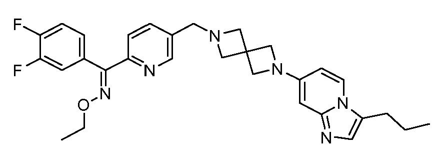 | **0.36** |

As in the above, the compounds of the invention strongly inhibit the binding of MCH to MCH-1R, and therefore exhibit an excellent effect as an MCH-IR antagonist.

Accordingly, the compounds of the invention are useful as a preventive or a remedy for various MCH-related diseases, for example, metabolic disorders such as obesity, diabetes, hormone disorder, hyperlipidemia, gout, fatty liver, etc.; cardiovascular disorders such as stenocardia, acute/congestive heart failure, myocardial infarction, coronary atherosclerosis, hypertension, renal diseases, electrolyte abnormality, etc.; central or peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism, etc.; reproductive disorders such as infertility, preterm labor, sexual dysfunction, etc.; and other digestive disorders; respiratory disorders; cancer or pigmentation; especially as a preventive or a remedy for bulimia, obesity, diabetes, fatty liver, depression, anxiety.

### Pharmaceutical Composition Comprising Compound of Formula [I]

The compound of the invention can be orally or parenterally administered, and can be formulated into preparations suitable to the administration thereof, which may be used as pharmaceutical compositions for prevention or treatment for the above-mentioned diseases.

In its clinical use, the compound of the invention may be formulated into various preparations along with a pharmaceutically-acceptable carrier added thereto generally in accordance with the administration route thereof, and the thus-formulated pharmaceutical composition may be administered. Various ordinary additives used in the field of pharmaceutical preparations can be used. For example, they include gelatin, lactose, white sugar, titanium oxide, starch, crystalline cellulose, hydroxypropylmethyl cellulose, carboxymethyl cellulose, corn starch, microcrystalline wax, white petrolatum, magnesium aluminate metasilicate, anhydrous calcium phosphate, citric acid, trisodium citrate, hydroxypropyl cellulose, sorbitol, sorbitan fatty acid esters, polysorbate, sucrose fatty acid esters, polyoxyethylene, hardened castor oil, polyvinylpyrrolidone, magnesium stearate, light silicic anhydride, talc, vegetable oils, benzyl alcohol, gum arabic, propylene glycol, polyalkylene glycol, cyclodextrin, hydroxypropylcyclodextrin, etc.

Preparations to be formed with those additives include, for example, solid preparations such as tablets, capsules, granules, powders, suppositories, etc.; and liquid preparations such as syrups, elixirs, injections, etc. These may be formulated according to ordinary methods known in the field of pharmaceutical preparations. The liquid preparations may also be in such a form that may be dissolved or suspended in water or in any other suitable medium in their use. Especially for injections, if desired, the preparations may be dissolved or suspended in physiological brine or glucose liquid, and a buffer or a preservative may be optionally added thereto.

The pharmaceutical compositions may contain the compound of the invention in an amount of from 1 to 99.9 % by weight, preferably from 1 to 60 % by weight of the composition. The compositions may further contain any other therapeutically-effective compound.

Specifically, the invention provides a pharmaceutical composition containing a medicinally-acceptable additive, and a therapeutically-effective amount of a compound of the invention or the pharmaceutically-acceptable salt thereof.

The wording, therapeutically-effective amount as referred to herein means the amount of a medicine to induce biological or medical phenomena in animals and humans, that is found by researchers, veterinaries, medical doctors or any other clinicians.

In case where the compounds of the invention are used for prevention or treatment for the above-mentioned diseases, then the dose and the dosing frequency may be varied, depending on the sex, the age, the body weight and the disease condition of the patient, on the type and the range of the intended remedial effect, etc. In general, in oral administration, the dose may be from 0.001 to 50 mg/kg of body weight/day, and it may be administered at a time or in a few times. The dose is preferably from about 0.01 to about 25 mg/kg/day, more preferably from about 0.05 to about 10 mg/kg/day.

As combination therapy, the compounds of the invention can be used in combination with drugs effective for hypertension, obesity-associated hypertension, hypertension-associated diseases, hypertrophy, left ventricular hypertrophy, metabolic disorders, obesity, obesity-associated diseases and the like (hereafter referred to as "co-drugs"). Such drugs can be administered simultaneously, separately or in succession, for prevention or treatment of the above-mentioned diseases. When a compound of the invention is used simultaneously with one, two or more of co-drugs, they may be formulated into a medical preparation suited for single administration form. Whereas, in combination therapy, a composition containing the compound of the invention and co-drugs may be administered to the object of medication in different packages, either simultaneously, separately or successively. They may be administered at time intervals.

The dose of the co-drug may be determined in accordance with the clinically adopted dose thereof, which can be suitably selected according to the individual object of medication, the administration route, the specific disease, the combination of drugs, and the like. The form of the co-drug for administration is not specifically defined, and it may be combined with the compound of the invention when they are administered.

The administration mode includes, for example, the following: (1) A compound of the invention is combined with a co-drug to give a single preparation for single administration; (2) a compound of the invention and a co-drug are separately formulated into different two preparations, and the two preparations are simultaneously administered in one administration route; (3) a compound of the invention and a co-drug are separately formulated into different two preparations, and they are administered at different times in one and the same administration route; (4) a compound of the invention and a co-drug are separately formulated into different two preparations, and they are administered at the same time in two different administration routes; (5) a compound of the invention and a co-drug are separately formulated into different two preparations, and they are administered at different times in different administration routes (for example, a compound of the invention and a co-drug are administered in that order, or in an order contrary to this). The blend ratio of the compound of the invention and the co-drug may be suitably determined depending on the administration object, the administration route, the disease for the administration, etc.

The co-drugs usable in the invention include, for example, drugs for diabetes, drugs for hyperlipidemia, drugs for hypertension, anti-obesity drugs. Two or more such co-drugs may be combined in an adequate ratio and used.

The remedy for diabetes include, for example, 1) PPAR-γ agonists such as glitazones (e.g., ciglitazone, darglitazone, englitazone, isaglitazone (MCC-555) et al), pioglitazone, rosiglitazone, troglitazone, BRL49653, CLX-0921, 5-BTZD, GW-0207, LG-100641, LY-300512et al; 2) biguanides such as metformin, buformin, phenformin et al; 3) protein tyrosine phosphatase 1B inhibitors; 4) sulfonylureas such as acetohexamide, chloropropamide, diabinese, glibenclamide, glipizide, glyburide, glimepiride, gliclazide, glipentide, gliquidone, glisolamide, trazamide, tolubutamide et al; 5) meglitinides such as repaglinide, nateglinide et al; 6) α-glucoside hydroxylase inhibitors such as acarbose, adiposine, camiglibose, emiglitate, miglitol, voglibose, pradimicin-Q, salbostatin, CKD-711, MDL-25, 673, MDL-73, 945, MOR14 et al; 7) α-amylase inhibitors such as tendamistat, trestatin, A13688 et al; 8) insulin secretion promoters such as linogliride, A-4166 et al; 9) fatty acid oxidation inhibitors such as clomoxir, etomoxir et al; 10) A2 antagonists such as midaglizole, isaglidole, deriglidole, idazoxan, earoxan, fluparoxan et al; 11) insulin or insulin mimetics such as biota, LP-100, novalapid, insulindetermir, insulin lispro, insulin glargine, insulin zinc, Lys-Pro-insulin, GLP-1 (73-7), GLP1 amide (7-36) et al; 12) non-thiazolidinediones such as JT-501, farglitazar et al; 13) PPARα/γ dual-agonists such as MK-0767, CLX-0940, GW-1536, GW-1929, GW-2433, KRP-297, L-796449, LR-90 and SB219994 et al.

The remedy for hyperlipidemia include, for example, 1) bile acid absorption promoters such as cholesterylamine, colesevelem, colestipol, crosslinked dextran dialkylaminoalkyl derivatives, Colestid^{™}, LoCholest^{™}, Questran^{™} et al; 2) HMG-CoA reductase inhibitors such as atorvastatin, itavastatin, fluvastatin, lovastatin, pravastatin, rivastatin, rosuvastatin, simvastatin, ZD-4522 et al; 3) HMG-CoA synthase inhibitors; 4) cholesterol absorption inhibitors such as snatol ester, β-sitosterol, sterol glucoside, ezetimibe et al; 5) acylcoenzyme A-cholesterol acyl transferase inhibitors such as avasimibe, eflucimibe, KY-505, SMP-709 et al; 6) CETP inhibitors such as JTT705, torcetrapib, CP532632, BAY-63-2149, SC-591, SC-795 et al; 7) squalane synthesis inhibitors; 8) antioxidants such as probucol; 9) PPAR-α agonists such as beclofibrate, benzafibrate, ciprofibrate, clofibrate, etofibrate, fenofibrate, gemcabene, gemfibrozil, GW-7647, BM-170744, LY-518674, fibric acid derivatives (e.g., Atromid^{™}, Lopid^{™}, Tricor^{™}) et al; 10) FXR receptor antagonists such as GW-4064, SR-103912 et al; 11) LXR receptor agonists such as GW3965, T9013137, XTCO-179628 et al; 12) lipoprotein synthesis inhibitors such as niacin; 13) renin-angiotensin system inhibitors; 14) microsome-triglyceride transport inhibitors; 15) bile acid resorption inhibitors such as BARA1453, SC435, PHA384640, S-435, AZD7706 et al; 16) PPAR-δ agonists such as GW501516, GW590735 et al; 17) triglyceride synthesis inhibitors; 18) MTTP inhibitors such as LAB687, CP346086 et al; 19) low-density lipoprotein receptor inducers; 20) squalane epoxidase inhibitors; 21) platelet agglutination inhibitors; 22) 5-lipoxygenase activated protein inhibitors such as MK-591.

The remedy for hypertension include, for example, 1) thiazide diuretics such as chlorothialidon, chlorothiazide, dichlorofenamide, hydrofluorothiazide, indapamide, hydrochlorothiazide et al; loop diuretics such as bumetanide, ethacrynic acid, flosemide, tolusemide et al; sodium diuretics such as amyloride, triamterene et al; aldosterone antagonist diuretics such as spironolactone, epilenone et al; 2) β-adrenaline blockers such as acebutolol, atenolol, betaxolol, bevantolol, bisoprolol, bopindolol, carteolol, carvedilol, celiprolol, esmolol, indenolol, metaprolol, nadolol, nebivolol, penbutolol, pindolol, probanolol, sotalol, tertatolol, tilisolol, timolol et al; 3) calcium channel blockers such as amlodipine, aranidipine, azelnidipine, barnidipine, benidipine, bepridil, cinaldipine, clevidipine, diltiazem, efonidipine, felodipine, gallopamil, isradipine, lacidipine, lemildipine, lercanidipine, nicardipine, nifedipine, nilvadipine, nimodepine, nisoldipine, nitrendipine, manidipine, pranidipine, verapamil et al; 4) angiotensin converting enzyme inhibitors such as benazepril, captopril, cilazapril, delapril, enalapril, fosinopril, imidapril, rosinopril, moexipril, quinapril, quinaprilat, ramipril, perindopril, perindoropril, quanipril, spirapril, tenocapril, trandolapril, zofenopril et al; 5) neutral endopeptidase inhibitors such as omapatrilat, cadoxatril, ecadotril, fosidotril, sampatrilat, AVE7688, ER4030 et al; 6) endotheline antagonists such as tezosentan, A308165, YM62899 et al; 7) vasodilators such as hydralazine, clonidine, minoxidil, nicotinyl alcohol et al; 8) angiotensin II antagonists such as candesartan, eporsartan, iribesartan, losartan, pratosartan, tasosartan, telmisartan, valsartan, EXP-3137, FI6828K, RNH6270 et al; 9) α/β adrenaline blockers such as nipradilol, arotinolol, amoslalol et al; 10) α1 blockers such as terazosin, urapidil, prazosin, bunazosin, trimazosin, doxazosin, naphthopidil, indolamin, WHIP164, XEN010 et al; 11) α2 agonists such as lofexidine, tiamenidine, moxonidine, rilmenidine, guanobenz et al; and 12) aldosterone inhibitors.

The anti-obesity drugs include, for example, 1) 5HT (serotonin) transporter inhibitors such as paroxetine, fluoxetine, fenfluramine, fluvoxamine, sertraline, imipramine et al; 2) norepinephrine transporter inhibitors such as GW320659, desipramine, talsupram, nomifensin et al; 3) cannabinoid-1 receptor 1 (CB-1) antagonists/inverse-agonists such as limonabant (Sanofi Synthelabo), SR-147778 (Sanofi Synthelabo), BAY-65-2520 (Bayer), SLV-319 (Solvey), as well as compounds disclosed in USP 5,532,237, USP 4,973,587, USP 5,013,837, USP 5,081,122, USP 5,112,820, USP 5,292,736, USP 5,624,941, USP 6,028,084, WO96/33159, WO98/33765, WO98/43636, WO98/43635, WO01/09120, WO01/96330, WO98/31227, WO98/41519, WO98/37061, WO00/10967, WO00/10968, WO97/29079, WO99/02499, WO01/58869, WO02/076949, WO01/64632, WO01/64633, WO01/64634, WO03/006007, WO03/007887 and EP-658546 et al; 4) ghrelin antagonists such as compounds disclosed in WO01/87355, WO02/08250 et al; 5) histamine(H3) antagonists/inverse-agonists such as thioperamide, 3-(1H-imidazol-4-yl)propyl N-(pentenyl)carbonate, clobenpropit, iodofenpropit, imoproxyfen, GT2395, A331440, compounds disclosed in WO02/15905, O-[3-(1H-imidazol-4-yl)propanol] carbamate, piperazine-containing H3-receptor antagonists (Lazewska, D. et al., Pharmazie, 56: 927-32 (2001), benzophenone derivatives (Sasse, A. et al., Arch. Pharm. (Weinheim) 334: 45-52 (2001)), substituted N-phenylcarbamates (Reidemeister, S. et al., Pharmazie, 55: 83-6 (2000)), proxyfen derivatives (Sasse, A. et al., J. Med. Chem., 43: 3335-43 (2000)) et al; 6) MCH-1R antagonists such as T-226296 (Takeda), SNP-7941 (Synaptic), other compounds disclosed in WO01/82925, WO01/87834, WO02/051809, WO02/06245, WO02/076929, WO02/076947, WO02/04433, WO02/51809, WO02/083134, WO02/094799, WO03/004027 and JP-A-2001-226269 et al; 7) MCH-2R agonists/antagonists; 8) NPY1 antagonists such as isopropyl 3-chloro-5-(1-(6-[2-(5-ethyl-4-methyl-thiazol-2-yl)-ethyl]-4-morpholinyl-4-yl-piridin-2-ylamino)-ethyl)phenyl]carbamate, BIBP3226, BIBO3304, LY-357897, CP-671906, GI-264879, and other compounds disclosed in USP 6,001,836, WO96/14307, WO01/23387, WO99/51600, WO01/85690, WO01/85098, WO01/85173 and WO01/89528 et al; 9) NPY5 antagonists such as 152804, GW-569180A, GW-594884A, GW-587081X, GW-548118X, FR235,208, FR226928, FR240662, FR252384, 1229U91, GI-264879A, CGP71683A, LY-377897, LY366377, PD-160170, SR-120562A, SR-120819A, JCF-104, H409/22, and other compounds disclosed in USP 6,140,354, USP 6,191,160, USP 6,258,837, USP 6,313,298, USP 6,337,332, USP 6,329,395, USP 340,683, USP 6,326,375, USP 6,329,395, USP 6,337,332, USP 6,335,345, EP-01010691, EP-01044970, WO97/19682, WO97/20820, WO97/20821, WO97/20822, WO97/20823, WO98/27063, WO00/107409, WO00/185714, WO00/185730, WO00/64880, WO00/68197, WO00/69849, WO01/09120, WO01/14376, WO01/85714, WO1/85730, WO01/07409, WO01/02379, WO01/02379, WO01/23388, WO01/23389, WO01/44201, WO01/62737, WO01/62738, WO01/09120, WO02/20488, WO02/22592, WO02/48152, WO02/49648, WO02/094789, and compounds disclosed in Norman et al., J. Med. Chem., 43:4288-4312(2000) et al; 10) leptins such as human recombinant leptin (PEG-OB, Hoffman La Roche), recombinant methionylleptin (Amgen) et al; 11) leptin derivatives such as compounds disclosed in USP 5,552,524, USP 5,552,523, USP 5,552,522, USP 5,521,283, WO96/23513, WO96/23514, WO96/23515, WO96/23516, WO96/23517, 96/23518, WO96/23519 and WO96/23520 et al; 12) opioid antagonists such as nalmefen (Revex^{™}), 3-methoxynaltorexone, naloxone, naltorexone, compounds disclosed in WO00/21509 et al; 13) orexin antagonists such as SB-334867A, and other compounds disclosed in WO01/96302, WO01/68609, WO02/51232, WO02/51838 and WO03/023561 et al; 14) bombesin receptor subtype-3 agonists; 15) cholecystokinin A (CCK-A) agonists such as AR-R15849, GI-181771, JMV-180, A-71378, A-71623, SR-146131, and other compounds disclosed in USP 5,739,106 et al; 16) CNTF (ciliary neurotrophic factors) such as GI-181771 (Glaxo-Smith Kline), SR146131 (Sanofi Synthelabo), butabindide, PD170,292, PD149164 (Pfizer) et al; 17) CNTF derivatives such as axokine (Regeneron), and other compounds disclosed in WO94/09134, WO98/22128, WO99/43813 et al; 18) growth hormone secretion receptor agonists such as NN703, hexarelin, MK-0677, SM-130686, CP-424,391, L-692,429, L-163,255, and compounds disclosed in USP 6,358,951, US Patent Application Nos. 2002/049196, 2002/022637, WO01/56592, WO02/32888 et al; 19) serotonin receptor-2C agonists such as BVT933, DPCA37215, IK264, PNU22394, WAY161503, R-1065, YM348, and other compounds disclosed in USP 3,914,250, WO02/36596, WO02/48124, WO02/10169, WO01/66548, WO02/44152, WO02/51844, WO02/40456 and WO02/40457 et al; 20) melanocortin-3 receptor agonists; 21) melanocortin-4 receptor agonists such as CHIR86036 (Chiron), ME-10142, ME-10145 (Melacure), and other compounds disclosed in WO99/64002, WO00/74679, WO01/991752, WO01/74844, WO01/70708, WO01/70337, WO01/91752, WO02/059095, WO02/059107, WO02/059108, WO02/059117, WO02/12166, WO02/11715, WO02/12178, WO02/15909, WO02/068387, WO02/068388, WO02/067869, WO03/007949 and WO03/009847 et al; 22) monoamine resorption inhibitors such as sibutramine (Meridia^{™}/Reductil^{™}) and its salts, and other derivatives disclosed in USP 4,746,680, USP 4,806,570, USP 5,436,272, US Patent Application No. 2002/0006964, WO01/27068 and WO01/62341 et al; 23) serotonin re-uptake inhibitors such as dexfenfluramine, fluoxetine, and other compounds disclosed in USP 6,365,633, WO01/27060 and WO01/162341 et al; 24) glucagon-like peptide-1 agonists; 25) topiramate (Topimax^{™}); 26) phytopharm compound 57 (e.g., CP644,673); 27) acetyl CoA carboxylase-2 (ACC2) inhibitors; 28) β-adrenalin receptor-3 agonists such as AD9677/TAK677 (Dai-Nippon Pharmaceutical/Takeda Chemical), CL-316,243, SB418790, BRL-37344, L-796568, BMS-196085, BRL-35135A, CGP12177A, BTA-243, W427353, Trecadrine, Zeneca D7114, SR59119A, and other compounds disclosed in USP 5,705,515, USP 5,451,677, WO01/74782 and WO02/32897 et al; 29) diacylglycerol acyltransferase-1 inhibitors; 30) diacylglycerol acyltransferase-2 inhibitors, 31) fatty acid synthesis inhibitors such as carulenin, C75; 32) phosphodiesterase inhibitors such as theophylline, pentoxiphylline zaprinast, sildenafil, amrinone, milrinone, cilostamide, rolipram and cilomilast et al; 33) thyroid hormone-β agonists such as KB-2611 (KaroBio BMS), and other compounds disclosed in WO02/15845, JP-A-2000-256190 et al; 34) UCP (uncoupling protein)-1, 2, or 3 activators such as phytanic acid, 4-[(E)-2-(5,6,7,8-tetrahydro-5,5,8,8-tetramethyl-2-naphthalenyl)-1-propenyl]benzoic acid (TTNPB), retinoic acid, and other compounds disclosed in WO99/00123 et al; 35) acylestrogens such as oleoylestrone, and other compounds disclosed in del Mar-Grasa, M. et al., Obesity Research, 9:202-9 (2001) et al; 36) glucocorticoid antagonists; 37) 11-β-hydroxysteroid dehydrogenase-1 inhibitors such as BVT3498, BVT2733, and other compounds disclosed in WO01/90091, WO01/90090, WO01/90092 et al; 38) stearoyl-CoA desaturase-1 inhibitors; 39) dipeptidyl peptidase-IV inhibitors such as isoleucine thiazolidide, valine pyrrolidide, NVP-DPP728, AF237, P93/01, TSL225, TMC-2A/2B/2C, FE999011, P9310/K364, VIP0177, SDZ274-444, and other compounds disclosed in WO03/004498, WO03/004496, EP1258476, WO02/083128, WO02/062764, WO03/000250, WO03/002530, WO03/002531, WO03/002553, WO03/002593, WO03/000180 and WO03/000181 et al; 40) lipase inhibitors such as tetrahydroliptatin (Orlistat/Xenical^{™}), Triton WR1339, RHC80267, lipstatin, teasaponin, diethylumbelliferyl phosphate, FL-386, WAY-121898, Bay-N-3176, valilactone, esteracin, ebelactone A, ebelactone B, RHC80267, and other compounds disclosed in WO01/77094, USP 4,598,089, USP 4,452,813, USP 5,512,565, USP 5,391,571, USP 5,602,151, USP 4,405,644, USP 4,189,438 and USP 4,242,453 et al; 41) fatty acid transporter inhibitors; 42) dicarboxylate transporter inhibitors; 43) glucose transporter inhibitors; 44) phosphate transporter inhibitors.

Those combined drugs are obtained by combining a compound of the invention with one, two or more of the above co-drugs. Furthermore, the combined drugs are useful for prevention or treatment of metabolic disorders, when combined with one, two or more drugs selected from the group consisting of remedy for diabetes and remedy for hyperlipidemia. Combinations containing, in particular, remedy for hypertension and anti-obesity agent are useful for prevention or treatment of metabolic disorders with synergistic effect, when remedy for diabetes and/or remedy for hyperlipidemia are added thereto.

On the other hand, the compound of the invention may be combined with an antipsychotic. An antipsychotic, especially an atypical antipsychotic is known to have a side effect of body weight increase; and the compound of the invention, when combined with such an antipsychotic, is useful for retarding the side effect. The antipsychotic includes, for example, olanzapine, Risperidone, quetiapine, Ziprasidone, aripiprazole, Paliperidone, Clozapine et al. Using an antipsychotic, as combined with a compound of the invention, may improve the level of metabolic parameters such as the level of blood pressure, glucose and lipid level that may be elevated by the antipsychotic. The above-mentioned methods may apply to the conditions of dose, administration subj ect, administration route and administration form.

### BEST MODE FOR CARRYING OUT THE INVENTION

The invention is described more concretely with reference to the following Examples, to which, however, the invention should not be limited. The compounds of the invention may be produced with reference to the methods described in WO2008/038692 and to known techniques. As silica gel for column, used was Wakogel^{™} C-200 (Wako Pure Chemical Industries); as a filled silica gel column, used was FLASH+^{™} cartridge, KP-Sil or FPNH, FLASH12+M, FLASH25+S, FLASH25+M, FLASH40+M (Biotage Japan), TC-C18 (Agilent), Extend-C18 (Zorbax); and as a partitioning thin-layer chromatogram, used was Kieselgel 60F254 (Merck). For mass spectrometry, used was QuattroII (Micromass). For ¹H-NMR, used was JNM-AL400 (JEOL) or MERCURYvx400 (VARIAN) and ^{UNITY}INOVA400 (VARIAN); and for mass spectrometry, used was ZQ2000 (Waters).

### EXAMPLES

### Reference Example 1 - Production of compound of formula (IX) (hereinafter referred to as "oxime (IX)") through stereoselective oximation with metal catalyst:

### Reference Example 1-1:

### Production of (E)-(3,4-difluorophenyl)[5-(hydroxymethyl)-2-pyridinyl]methanone O-(2-hydroxy-2-methylpropyl)oxime

25 % sodium methoxide/methanol solution (50 mL) was added to a methanol solution (500 mL) of O-(2-hydroxy-2-methylpropyl)hydroxylamine hydrochloride (28.8 g) and stirred for 30 minutes. A solution previously prepared by dissolving (3,4-difluorophenyl)[5-(hydroxymethyl)-2-phenyl]methanone (25.4 g) and copper(I) triflate-toluene complex (274 mg) in methanol (500 mL) followed by stirring it for 30 minutes was added to the above, and stirred at room temperature for 13 hours, then aqueous 28 % ammonia was added to the reaction liquid, and methanol was evaporated off under reduced pressure. This was extracted with ethyl acetate, washed twice with aqueous 28 % ammonia, then water and saturated brine in that order, dried with sodium sulfate, concentrated under reduced pressure, and further dried in vacuum to give the entitled compound (32.3 g) as a colorless oil.
ESI-MS Found: m/z 337[M+H]⁺

A compound of formula (VII) was used and reacted under the condition shown in the above Reference Example 1, the following oximes (IX) were produced.

[Table 2]

**Table 2 - Production of Oximes (IX)**

| Reference Example | R²O-NH₂ **(VII)** | 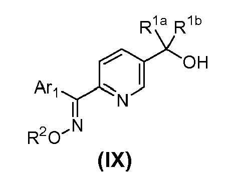 | **ESI-MS** |
|---|---|---|---|
| 1-2 | EtONH₂·HCl | 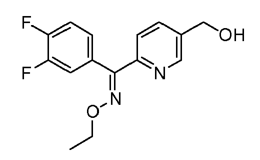 | 293 [M+H]⁺ |
| 1-3 | FCH₂ONH₂·HCl | 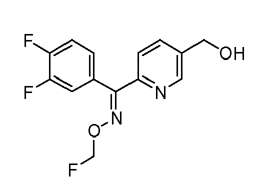 | 297 [M+H]⁺ |
| 1-4 | MeSO₂CH₂ONH₂·HCl | 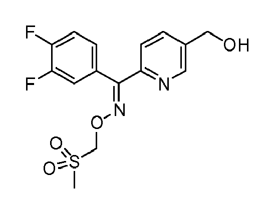 | 357 [M+H]⁺ |

### Reference Example 2 - Production of compound of formula (VIII) (hereinafter referred to as "amine (VIII)"):

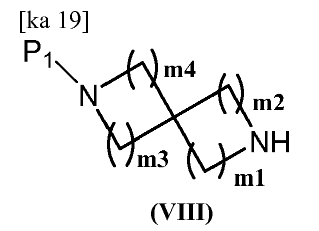

### Reference Example 2-1:

### 1,1-Dimethylethyl 7-(phenylmethyl)-2,7-diazaspiro[4.4]nonane-2-carboxylate

At 0°C, di-tert-butyl dicarbonate (0.64 mL) was added to a chloroform solution (2.0 mL) of 2-(phenylmethyl)-2,7-diazaspiro[4.4]nonane (500 mg), diisopropylethylamine (0.64 mL) and DMAP (28.2 mg), and stirred at room temperature for 18 hours. Aqueous sodium hydrogencarbonate solution was added to the reaction liquid, and extracted with chloroform. The organic layer was washed with saturated brine, dried with sodium sulfate, and concentrated under reduced pressure. The resulting residue was purified through silica gel column chromatography (chloroform/methanol = 100/0 to 95/5) to give the entitled compound (635 mg) as a brown oil. ESI-MS Found: m/z 317[M+H]⁺

### Reference Example 2-2:

### 1,1-Dimethylethyl 2,7-diazaspiro[4.4]nonane-2-carboxylate

20 % palladium hydroxide-carbon (141 mg) was added to a methanol solution (10 mL) of the compound (635 mg) obtained in Reference Example 2-1, and stirred in a hydrogen atmosphere at room temperature for 18 hours. The reaction liquid was filtered through Celite, and the filtrate was concentrated under reduced pressure to give the entitled compound (458 mg) as a pale yellow oil.
ESI-MS Found: m/z 227[M+H]⁺

### Reference Example 2-3:

### 1,1-Dimethylethyl 2,6-diazaspiro[3.4]octane-6-carboxylate

The entitled compound was obtained according to the same operation as in Reference Example 2-2 but using 1,1-dimethylethyl 2-(phenylmethyl)-2,6-diazaspiro[3.4]octane-carboxylate.
ESI-MS Found: m/z 213[M+H]⁺

### Reference Example 2-4:

### 2-(Phenylmethyl)-2,6-diazaspiro[3.4]octane

At 0°C, trifluoroacetic acid (3.0 mL) was added to a chloroform solution (3.0 mL) of 1,1-dimethylethyl 2-(phenylmethyl)-2,6-diazaspiro[3.4]octane-carboxylate (300 mg), and stirred at room temperature for 30 minutes. The reaction solution was evaporated with toluene, then aqueous 2 N sodium hydroxide solution was added to the residue, and extracted with chloroform. The organic layer was dried with sodium sulfate, and then concentrated under reduced pressure to give the entitled compound (100 mg).
ESI-MS Found: m/z 203[M+H]⁺

### Reference Example 3 - Production of compound of formula (III-a) (hereinafter referred to as "protected arylamine (III-a)"):

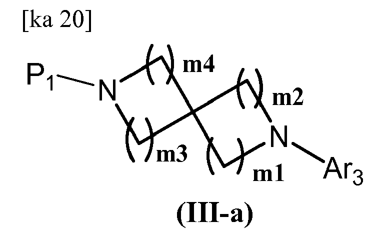

### Reference Example 3-1:

### 2-(6-Fluoro-3-pyridinyl)-7-(phenylmethyl)-2,7-diazaspiro[4.4]nonane

Pd₂ (dba)₃ (43.2 mg) was added to a toluene (2.0 mL) solution of 2-(phenylmethyl)-2,7-diazaspiro[4.4]nonane (100.0 mg), 5-bromo-2-fluoropyridine (81.0 mg), (R)-(+)-BINAP (43.2 mg) and sodium tert-butoxide (62.2 mg), and stirred at 130°C for 30 minutes under irradiation with microwaves. Aqueous sodium hydrogencarbonate solution was added to the reaction liquid, and extracted with ethyl acetate. The organic layer was washed with saturated brine, dried with sodium sulfate, and concentrated under reduced pressure. The resulting residue was purified through silica gel column chromatography (hexane/ethyl acetate = 10/1 to 7/3) to give the entitled compound (67.1 mg) as a pale green oil.
ESI-MS Found: m/z 312[M+H]⁺

Various amines (VIII) and compounds of formula (V) were used and reacted under the condition shown in the above-mentioned Reference Example 3-1 to give protected arylamines (III-a) mentioned below.

[Table 3]

**Table 3 - Production of Protected Arylamines (IIIa)**

| Reference Example | 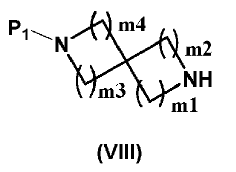 | **Ar₃·X** **(V)** | 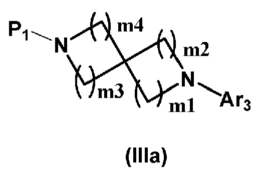 | ESI-MS |
|---|---|---|---|---|
| 3-2 | 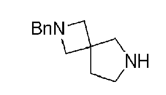 | 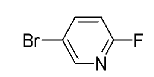 | 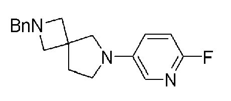 | 298 [M+H]⁺ |
| 3-3 | 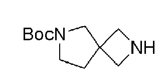 | 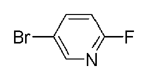 | 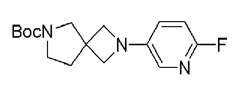 | 308 [M+H]⁺ |
| 3-4 | 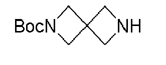 | 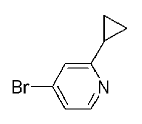 | 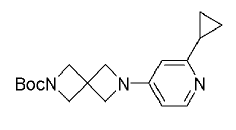 | 316 [M+H]⁺ |
| 3-5 | 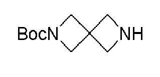 | 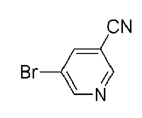 | 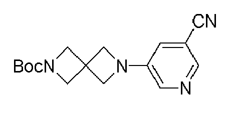 | 301 [M+H]⁺ |
| 3-6 | 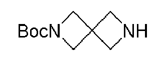 | 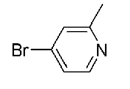 | 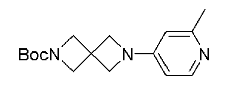 | 290 [M+H]⁺ |
| 3-7 | 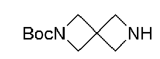 | 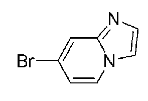 | 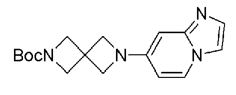 | 315 [M+H]⁺ |

### Reference Example 4 - Production of compound of formula (III) (hereinafter referred to as "arylamine (III)"):

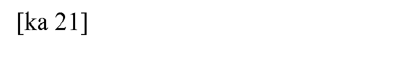

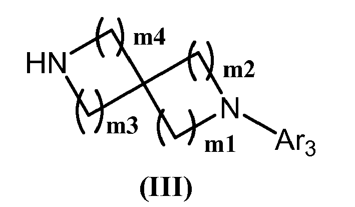

### Reference Example 4-1:

### 2-(6-Fluoro-3-pyridinyl)-2,7-diazaspiro[4.4]nonane

20 % palladium hydroxide-carbon (50.0 mg) was added to a methanol solution (5.0 mL) of the compound (67.1 mg) of the compound obtained in Reference Example 3-1, and stirred under a hydrogen atmosphere at room temperature for 18 hours. The reaction liquid was filtered through Celite, and the filtrate was concentrated under reduced pressure. The resulting residue was dried to give the entitled compound (47.0 mg) as a colorless amorphous substance. ESI-MS Found: m/z 222[M+H]⁺

Various protected arylamines (III-a) were used and deprotected under the same condition as in the above-mentioned Reference Example 2-4 or 4-1 to give the following arylamines (III).

[Table 4]

**Table 4 - Production of Arylamines (III)**

| Reference Example | 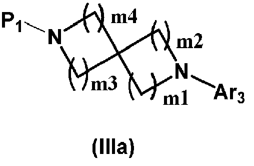 | Conditions* | 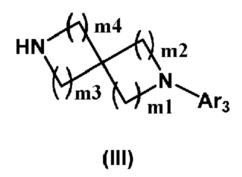 | ESI-MS |
|---|---|---|---|---|
| 4-2 | 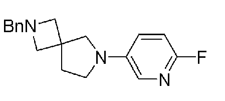 | **A** | 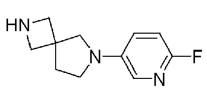 | 208 [M+H]⁺ |
| 4-3 | 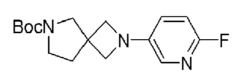 | **B** | 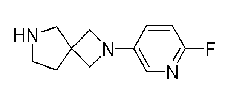 | 208 [M+H]⁺ |
| 4-4 | 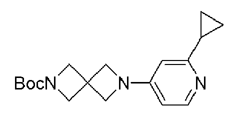 | **B** | 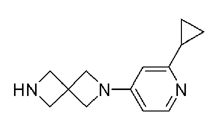 | 216 [M+H]⁺ |
| 4-5 | 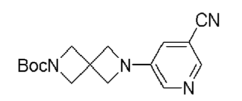 | **B** | 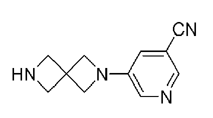 | 201 [M+H]⁺ |
| 4-6 | 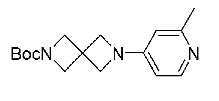 | **B** | 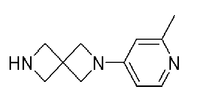 | 190 [M+H]⁺ |
| 4-7 | 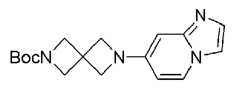 | **B** | 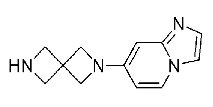 | 215 [M+H]⁺ |

| | | | | |
|---|---|---|---|---|
| *Condition A : H₂, Pd(OH)₂ / MeOH, Condition B : TFA / CHCl₃ | | | | |

### Reference Example 5 - Production of compound of formula (II) (hereinafter referred to as "compound (II)"):

### Reference Example 5-1:

### (6-{(E)-(3,4-Difluorophenyl)[(ethyloxy)imino]methyl}-3-pyridinyl)methyl methanesulfonate

At 0°C, methanesulfonyl chloride (0.64 mL) was added to an ethyl acetate solution (75 mL) of (E)-(3,4-difluorophenyl)[5-(hydroxymethyl)-2-pyridinyl]methanone O-ethyloxime (7.46 g) and triethylamine (2.17 mL), and stirred at room temperature for 1 hour. The reaction liquid was filtered through Celite, and the filtrate was concentrated under reduced pressure. The resulting residue was purified through silica gel column chromatography (hexane/ethyl acetate = 100/0 to 60/40) to give the entitled compound (7.06 g) as a brown solid.
ESI-MS Found: m/z 371 [M+H]⁺

### Reference Example 5-2:

### [6-((E)-(3,4-difluorophenyl){[(2-hydroxy-2-methylpropyl)oxy]imino}methyl)-3-pyridinyl]methyl methanesulfonate

A crude product (1.01 g) of the entitled compound was obtained as a brown oily substance according to the same process as in Reference Example 5-1 but using the alcohol (814 mg) obtained in Reference Example 1-1.
ESI-MS Found: m/z 415[M+H]⁺

### Reference Example 6 - Production of compound of formula (IV-a) (hereinafter referred to as "compound (IV-a)"):

### Reference Example 6-1:

### 1,1-Dimethylethyl 7-[(6-{[(E)-(3,4-difluorophenyl)[(ethyloxy)imino]methyl}-3-pyridinyl)methyl]-2,7-diazaspiro[4.4]nonane-2-carboxylate

Sodium iodide (397 mg) was added to a dimethylformamide solution (5.0 mL) of the compound (200 mg) obtained in Reference Example 2-2, the compound (316 mg) obtained in Reference Example 5-1 and diisopropylethylamine (0.77 mL), and stirred at 80°C for 15 hours. Saturated sodium hydrogencarbonate solution was added to the reaction liquid, and extracted with ethyl acetate. The organic layer was washed with saturated brine, dried with sodium sulfate, and concentrated under reduced pressure. The resulting residue was purified through silica gel column chromatography (chloroform/methanol = 100/0 to 97/3) to give the entitled compound (237 mg) as a brown oil.
ESI-MS Found: m/z 501 [M+H]⁺

Compounds of formula (IV-a) were obtained according to the same process as in the above-mentioned Reference Example 6-1 and using compounds (II) obtained in Reference Example 5 and Compounds of formula (VI-b).

[Table 5]

**Table 5 - Production of Compounds (IV-a)**

| Reference Example | 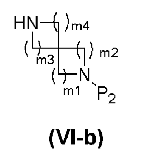 | 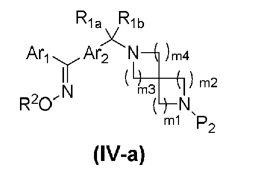 | ESI-MS |
|---|---|---|---|
| 6-2 | 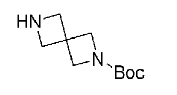 | 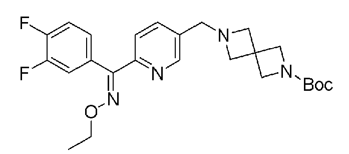 | 473 [M+H]⁺ |
| 6-3 | 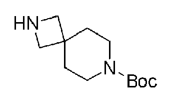 | 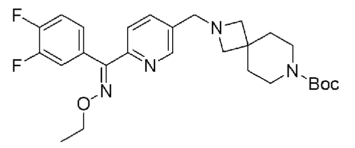 | 501 [M+H]⁺ |
| 6-4 | 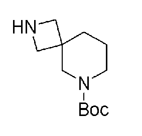 | 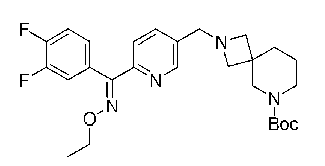 | 501 [M+H]⁺ |
| 6-5 | 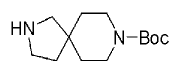 | 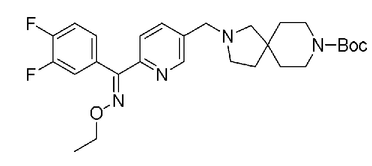 | 515 [M+H]⁺ |
| 6-6 | 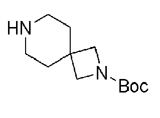 | 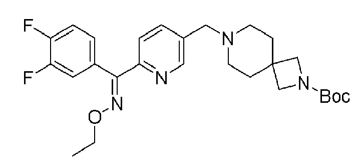 | 501 [M+H]⁺ |

### Reference Example 7 - Production of compound of formula (IV):

### Reference Example 7-1:

### (E)-[5-(2,7-diazaspiro[4.4]non-2-ylmethyl)-2-pyridinyl](3,4-difluorophenyl)methanone O-ethyloxime

At 0°C, trifluoroacetic acid (5.0 mL) was added to a chloroform solution (5.0 mL) of the compound (273 mg) obtained in Reference Example 6-1, and stirred at room temperature for 30 minutes. The reaction solution was evaporated with toluene, then saturated sodium hydrogencarbonate solution was added to the residue, and extracted with ethyl acetate. The organic layer was washed with saturated brine, then dried with sodium sulfate, and concentrated under reduced pressure. The resulting residue was purified through silica gel chromatography (chloroform/methanol = 100/0 to 97/3) to give the entitled compound (118 mg) as a brown oil. ESI-MS Found: m/z 401 [M+H]⁺

### Reference Example 7-2:

### (E)-[5-(2,6-diazaspiro[3.3]hept-2-ylmethyl)-2-pyridinyl](3,4-difluorophenyl)methanone O-ethyloxime

According to the same process as in Reference Example 7-1 but using the compound (75.0 mg) obtained in Reference Example 6-2, the entitled compound (68.0 mg) was obtained as a pale yellow oily substance.
ESI-MS Found: m/z 373[M+H]⁺

### Example 1:

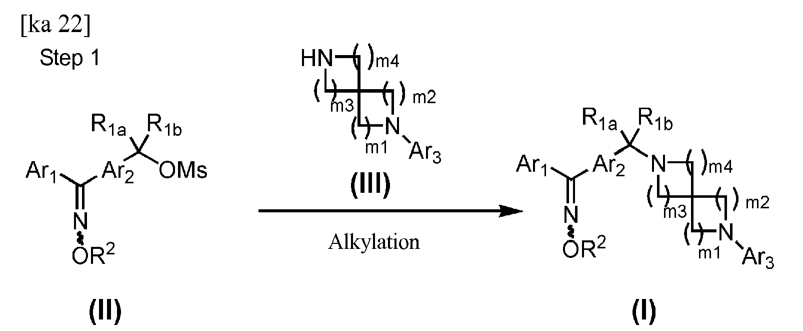

### Example 1-1:

### (E)-(3,4-difluorophenyl)(5-{[7-(6-fluoro-3-pyridinyl)-2,7-diazaspiro[4.4]non-2-yl]methyl}-2-pyridinyl)methanone O-ethyloxime

Diisopropylethylamine (186.0 µL) and sodium iodide (96.0 mg) were added to a DMF (2.0 mL) solution of the compound (79.0 mg) obtained in Reference Example 5-1 and the compound (47.1 mg) obtained in Reference Example 4-1, and stirred at 120°C for 5 minutes under irradiation with microwaves. Aqueous sodium hydrogencarbonate solution was added to the reaction liquid, and extracted with ethyl acetate. The organic layer was washed with saturated brine, dried with sodium sulfate, and concentrated under reduced pressure. The resulting residue was purified through high-performance liquid chromatography (YMC-Pack^{™} Pro C-18, water (0.1 % TFA)/acetonitrile (0.1 % TFA) = 90/10 to 50/50) to give the entitled compound (37.4 mg) as a pale yellow oil.
¹ H-NMR (400 MHz, CDCl₃, δ ppm): 1.32 (3H, t, J = 7.1 Hz), 1.85-2.04 (4H, m), 2.46 (1H, d, J = 8.8 Hz), 2.55-2.77 (3H, m), 3.17 (1H, d, J = 8.8 Hz), 3.25-3.33 (3H, m), 3.65 (2H, s), 4.30 (2H, q, J = 7.1 Hz), 6.77 (1H, dd, J = 3.2, 9.0 Hz), 6.86-6.92 (1H, m), 7.10-7.35 (3H, m), 7.38-7.40 (1H, m), 7.71 (1H, dd, J = 7.9, 1.9 Hz), 7.78 (1H, d, J = 7.9 Hz), 8.51 (1H, d, J = 1.9 Hz). ESI-MS Found: m/z 496[M+H]⁺

### Example 1-2:

### (E)-(3,4-difluorophenyl){5-[(7-phenyl-2,7-diazaspiro[4.4]non-2-yl)methyl]-2-pyridinyl}methanone O-ethyloxime

The entitled compound was obtained according to the same process as in Example 1-1 but using the compound obtained in Reference Example 5-1 and 2-phenyl-2,7-diazaspiro[4.4]nonane.
¹ H-NMR (400 MHz, CDCl₃, δ ppm): 1.32 (3H, t, J = 7.2 Hz), 1.80-2.05 (4H, m), 2.48 (1H, d, J = 9.6 Hz), 2.55-2.80 (3H, m), 3.19 (1H, d, J = 9.2 Hz)3.26-3.40 (3H, m), 3.65 (2H, s), 4.29 (2H, q, J = 7.2 Hz), 6.48-6.55 (2H, m), 6.62-6.68 (1H, m), 7.10-7.35 (5H, m), 7.72 (1H, dd, J = 1.8, 7.6 Hz), 7.78 (1H, d, J = 7.6 Hz), 8.51 (1H, d, J = 1.8 Hz).
ESI-MS Found: m/z 477[M+H]⁺

### Example 1-3:

### (E)-(5-{[7-(2-chlorophenyl)-2,7-diazaspiro[4.4]non-2-yl]methyl}-2-pyridinyl)(3,4-difluorophenyl)methanone O-ethyloxime

The entitled compound was obtained according to the same process as in Example 1-1 but using the compound obtained in Reference Example 5-1 and 2-(2-chlorophenyl)-2,7-diazaspiro[4.4]nonane. ¹ H-NMR (400 MHz, CDCl₃, δ ppm): 1.33 (3H, t, J = 7.1 Hz), 1.79-2.00 (4H, m), 2.45-2.51 (1H, m), 2.58-2.73 (3H, m), 3.28-3.32 (1H, m), 3.36-3.51 (3H, m), 3.64 (2H, s), 4.30 (2H, q, J = 7.1 Hz), 6.72-6.82 (2H, m), 7.09-7.34 (5H, m), 7.68-7.80 (2H, m), 8.51 (1H, s).
ESI-MS Found: m/z 512[M+H]⁺

### Example 1-4:

### (E)-(5-{[7-(3-chlorophenyl)-2,7-diazaspiro[4.4]non-2-yl]methyl}-2-pyridinyl)(3,4-difluorophenyl)methanone O-ethyloxime

The entitled compound was obtained according to the same process as in Example 1-1 but using the compound obtained in Reference Example 5-1 and 2-(3-chlorophenyl)-2,7-diazaspiro[4.4]nonane.
¹ H-NMR (400 MHz, CDCl₃, δ ppm): 1.32 (3H, t, J = 7.1 Hz), 1.80-2.05 (4H, m), 2.43-2.48 (1H, m), 2.54-2.75 (3H, m), 3.16-3.20 (1H, m), 3.23-3.37 (3H, m), 3.64 (2H, s), 4.30 (2H, q, J = 7.1 Hz), 6.35-6.39 (1H, m), 6.47-6.48 (1H, m), 6.59-6.63 (1H, m), 7.07-7.34 (4H, m), 7.68-7.80 (2H, m), 8.51 (1H, s).
ESI-MS Found: m/z 512[M+H]⁺

### Example 1-5:

### (E)-(5-{[7-(4-chlorophenyl)-2,7-diazaspiro[4.4]non-2-yl]methyl}-2-pyridinyl)(3,4-difluorophenyl)methanone O-ethyloxime

The entitled compound was obtained according to the same process as in Example 1-1 but using the compound obtained in Reference Example 5-1 and 2-(4-chlorophenyl)-2,7-diazaspiro[4.4]nonane. ¹ H-NMR (400 MHz, CDCl₃, δ ppm): 1.32 (3H, t, J = 7.1 Hz), 1.78-2.07 (4H, m), 2.43-2.48 (1H, m), 2.54-2.75 (3H, m), 3.14-3.18 (1H, m), 3.22-3.34 (3H, m), 3.64 (2H, s), 4.30 (2H, q, J = 7.1 Hz), 6.41 (2H, d, J = 9.3 Hz), 7.11-7.34 (5H, m), 7.67-7.79 (2H, m), 8.51 (1H, s).
ESI-MS Found: m/z 512[M+H]⁺

### Example 1-6:

### (E)-(3,4-difluorophenyl)[5-({7-[4-(methyloxy)phenyl]-2,7-diazaspiro[4.4]non-2-yl}methyl)-2-pyridinyl]methanone O-ethyloxime

The entitled compound was obtained according to the same process as in Example 1-1 but using the compound obtained in Reference Example 5-1 and 2-[4-(methyloxy)phenyl]-2,7-diazaspiro[4.4]nonane.
¹ H-NMR (400 MHz, CDCl₃, δ ppm): 1.32 (3H, t, J = 7.1 Hz), 1.78-2.05 (4H, m), 2.43-2.49 (1H, m), 2.55-2.75 (3H, m), 3.13-3.17 (1H, m), 3.22-3.32 (3H, m), 3.64 (2H, s), 3.75 (3H, s), 4.30 (2H, q, J = 7.1 Hz), 6.47 (2H, d, J = 8.3 Hz), 6.84 (2H, d, J = 8.3 Hz), 7.11-7.34 (3H, m), 7.68-7.79 (2H, m), 8.51 (1H, s).
ESI-MS Found: m/z 507[M+H]⁺

### Example 1-7:

### 5-{6-[(6-{(E)-(3,4-difluorophenyl)[(ethyloxy)imino]methyl}-3-pyridinyl)methyl]-2,6-diazaspiro[3.3]hept-2-yl}-3-pyridinecarbonitrile

The entitled compound was obtained as trifluoroacetate according to the same process as in Example 1-1 but using the compound obtained in Reference Example 5-1 and the compound obtained in Reference Example 4-5.
¹ H-NMR (400 MHz, CDCl₃ , δ ppm): 1.29 (3H, t, J = 7.6 Hz), 4.11-4.23 (4H, m), 4.25 (2H, q, J = 7.2 Hz), 4.32-4.40 (4H, m), 4.38 (2H, s), 7.11 (1H, s), 7.17 (1H, s), 7.26-7.38 (2H, m), 7.91-8.09 (3H, m), 8.21 (1H, s), 8.58 (1H, s).
ESI-MS Found: m/z 475[M+H]⁺

### Example 1-8:

### 5-{6-[(6-{(E)-(3,4-difluorophenyl)[(ethylxy)imino]methyl}-3-pyriainyl)methy]-2,6-diazaspiro[3.3]hept-2-yl}-3-pyridinecarboxamide

The entitled compound was obtained as trifluoroacetate, as a side product in Example 1-7.
¹ H-NMR (400 MHz, MeOD, δ ppm): 1.30 (3H, t, J = 7.2 Hz), 4.28-4.35 (6H, m), 4.40-4.46 (4H, m), 4.48 (2H, s), 7.12-7.18 (1H, m), 7.28-7.36 (2H, m), 7.55 (1H, s), 7.95-8.05 (3H, m), 8.40 (1H, s), 8.60 (1H, s).
ESI-MS Found: m/z 493[M+H]⁺

### Example 1-9:

### (E)-(5-{[6-(2-cyclopropyl-4-pyridinyl)-2,6-diazaspiro[3.3]hept-2-yl]methyl}-2-pyridinyl)(3,4-difluorophenyl)methanone O-ethyloxime

The entitled compound was obtained as trifluoroacetate according to the same process as in Example 1-1 but using the compound obtained in Reference Example 5-1 and the compound obtained in Reference Example 4-4.
¹ H-NMR (400 MHz, CDCl₃, δ ppm): 1.00-1.10 (2H, m), 1.20-1.26 (2H, m), 1.29 (3H, t, J = 7.2 Hz), 2.00-2.10 (1H, m), 4.30 (2H, q, J = 7.2 Hz), 4.38-4.47 (8H, m), 4.48 (2H, s), 6.23 (1H, s), 6.48 (1H, dd, J = 6.8, 1.6 Hz), 7.06-7.12 (1H, m), 7.26-7.36 (2H, m), 7.90 (1H, d, J = 6.8 Hz), 8.58 (1H, s).
ESI-MS Found: m/z 490[M+H]⁺

### Example 1-10:

### (E)-(3,4-difluorophenyl)(5-{[6-(6-fluoro-3-pyridinyl)-2,6-diazaspiro[3.4]oct-2-yl]methyl}-2-pyridinyl)methanone O-ethyloxime

The entitled compound was obtained as trifluoroacetate according to the same process as in Example 1-1 but using the compound obtained in Reference Example 5-1 and the compound obtained in Reference Example 4-2.
¹ H-NMR (400 MHz, MeOD, δ ppm): 1.29 (3H, t, J = 7.6 Hz), 2.38 (2H, d, J = 6.8 Hz), 3.38 (2H, m), 3.57 (2H, br s), 4.30 (2H, q, J = 7.6 Hz), 4.16-4.31 (4H, m), 4.52 (2H, s), 6.89 (1H, dd, J = 9.2, 2.8 Hz), 7.11-7.28 (2H, m), 7.25-7.35 (2H, m), 7.40-7.44 (1H, m), 7.95-8.02 (2H, m), 8.59 (1H, d, J = 1.2 Hz).
ESI-MS Found: m/z 482[M+H]⁺

### Example 1-11:

### (E)-(3,4-difluorophenyl)(5-{[2-(6-fluoro-3-pyridinyl)-2,6-diazaspiro[3.4]oct-6-yl]methyl}-2-pyridinyl)methanone O-ethyloxime

The entitled compound was obtained as trifluoroacetate according to the same process as in Example 1-1 but using the compound obtained in Reference Example 5-1 and the compound obtained in Reference Example 4-3. ¹ H-NMR (400 MHz, MeOD, δ ppm): 1.29 (3H, t, J = 7.6 Hz), 2.47-2.58 (2H, m), 3.39-3.73 (4H, m), 3.85-3.94 (4H, m), 4.28 (2H, q, J = 7.6 Hz), 4.50 (2H, s), 6.89 (1H, dd, J = 8.8, 2.8 Hz), 7.07-7.16 (2H, m), 7.28-7.35 (3H, m), 8.02 (2H, br s), 8.62 (1H, m).
ESI-MS Found: m/z 482[M+H]⁺

### Example 1-12:

### 5-(6-{[6-((E)-(3,4-difluorophenyl){[(2-hydroxy-2-methylpropyl)oxy]imino}methyl)-3-pyridinyl]methyl}-2,6-diazaspiro[3.3]hept-2-yl)-3-pyridinecarbonitrile

The entitled compound was obtained as trifluoroacetate according to the same process as in Example 1-1 but using the compound obtained in Reference Example 5-2 and the compound obtained in Reference Example 4-5. ¹ H-NMR (400 MHz, MeOD, δ ppm): 1.25 (6H, s), 4.15 (4H, s), 4.45 (4H, br s), 4.50 (2H, s), 7.15-7.25 (2H, m), 7.30-7.40 (2H, m), 7.96-8.06 (3H, m), 8.20 (1H, s), 8.59 (1H, s).
ESI-MS Found: m/z 519[M+H]⁺

### Example 1-13:

### (E)-(3,4-difluorophenyl)(5-{[6-(2-methyl-4-pyridinyl)-2,6-diazaspiro[3.3]hept-2-yl]methyl}-2-pyridinyl)methanone O-(2-hydroxy-2-methylpropyl)oxime

The entitled compound was obtained as trifluoroacetate according to the same process as in Example 1-1 but using the compound obtained in Reference Example 5-2 and the compound obtained in Reference Example 4-6. ¹ H-NMR (400 MHz, MeOD, δ ppm): 1.18 (6H, s), 2.48 (3H, s), 4.12 (2H, s), 4.19-4.38 (10H, m), 6.43-6.55 (2H, m), 7.12-7.21 (1H, m), 7.26-7.42 (2H, m), 7.92 (1H, d, J = 7.6 Hz), 7.98 (2H, m), 8.59 (1H, s).
ESI-MS Found: m/z 508[M+H]⁺

### Example 1-14:

### (E)-(3,4-difluorophenyl){5-[(6-imidazo[1,2-a]pyridin-7-yl-2,6-diazaspiro[3.3]hept-2-yl)methyl]-2-pyridinyl}methanone O-(2-hydroxy-2-methylpropyl)oxime

The entitled compound was obtained as trifluoroacetate according to the same process as in Example 1-1 but using the compound obtained in Reference Example 5-2 and the compound obtained in Reference Example 4-7.
¹ H-NMR (400 MHz, MeOD, δ ppm): 1.18 (6H, s), 4.13 (2H, s), 4.26-4.40 (4H, m), 4.40-4.50 (6H, m), 6.33 (1H, s), 6.70 (1H, dd, J = 7.6, 2.0 Hz), 7.13-7.19 (1H, m), 7.26-7.45 (2H, m), 7.57 (1H, s), 7.72 (1H, d, J = 2.0 Hz), 7.95-8.03 (2H, m), 8.38 (1H, d, J = 7.6 Hz), 8.59 (1H, s). ESI-MS Found: m/z 533[M+H]⁺

### Example 2:

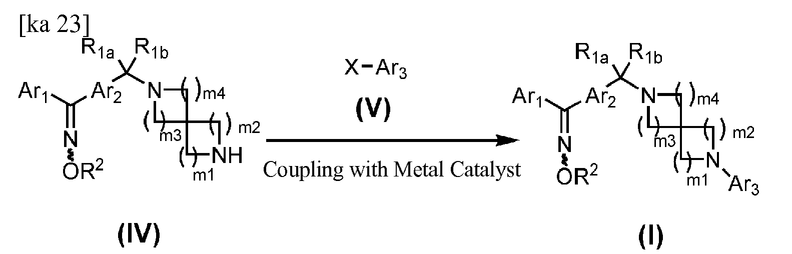

### Example 2-1:

### (E)-(3,4-difluorophenyl){5-[(7-pyrazolo[1,5-b]pyridazin-3-yl-2,7-diazaspiro[4.4]non-2-yl)methyl]-2-pyridinyl}methanone O-ethyloxime

The compound (53.4 mg) obtained in Reference Example 7-1, 3-bromopyrazolo[1,5-b]pyridazine (31.7 mg), (R)-(+)-BINAP (12.5 mg) and sodium tert-butoxide (18.0 mg) were dissolved in toluene (10.0 mL), then Pd₂(dba)₃ (6.1 mg) was added thereto, and stirred for 12 hours in a nitrogen atmosphere with heating under reflux. Aqueous sodium hydrogencarbonate solution was added to the reaction liquid, and extracted with ethyl acetate. The organic layer was washed with saturated brine, dried with sodium sulfate, and concentrated under reduced pressure. The resulting residue was purified through high-performance liquid chromatography (YMC-Pack^{™} Pro C-18, water (0.1 % TFA)/acetonitrile (0.1 % TFA) = 90/10 to 50/50) to give the entitled compound (3.8 mg) as an orange oil. ¹ H-NMR (400 MHz, CDCl₃, δ ppm): 1.32 (3H, t, J = 7.0 Hz), 1.98-2.05 (4H, m), 2.62-2.65 (4H, m), 3.26 (1H, d, J = 8.6 Hz), 3.35-3.44 (3H, m), 3.66 (2H, s), 4.30 (2H, q, J = 7.0 Hz), 6.58 (1H, dd, J = 4.1, 9.2 Hz), 7.16-7.35 (3H, m), 7.42 (1H, s), 7.76-7.78 (2H, m), 7.92-7.94 (1H, m), 8.01-8.03 (1H, m), 8.52-8.52 (1H, m).
ESI-MS Found: m/z 518[M+H]⁺

### Example 2-2:

### (E)-(3,4-difluorophenyl)(5-{[7-(2-pyrazinyl)-2,7-diazaspiro[4.4]non-2-yl]methyl}-2-pyridinyl)methanone O-ethyloxime

The entitled compound was obtained as trifluoroacetate according to the same process as in Example 2-1 but using the compound obtained in Reference Example 7-1 and 2-iodopyrazine. ¹ H-NMR (400 MHz, MeOD, δ ppm): 1.31 (3H, t, J = 7.2 Hz), 2.03-2.30 (4H, m), 3.40-3.70 (8H, m), 4.30 (2H, q, J = 7.2 Hz), 4.55 (2H, s), 7.12-7.18 (1H, m), 7.29-7.34 (2H, m), 7.80 (1H, s), 7.95-8.15 (4H, m), 8.64 (1H, s).
ESI-MS Found: m/z 479[M+H]⁺

### Example 2-3:

### (E)-(3,4-difluorophenyl)(5-{[7-(4-pyrazinyl)-2,7-diazaspiro[4.4]non-2-yl]methyl}-2-pyridinyl)methanone O-ethyloxime

The entitled compound was obtained as trifluoroacetate according to the same process as in Example 2-1 but using the compound obtained in Reference Example 7-1 and 4-bromopyrazine. ¹ H-NMR (400 MHz, MeOD, δ ppm): 1.35 (3H, t, J = 7.2 Hz), 2.13-2.50 (4H, m), 3.46-3.80 (6H, m), 3.88 (2H, s), 4.31 (2H, q, J = 7.2 Hz), 4.61 (2H, s), 6.95-7.43 (4H, m), 7.93-8.15 (2H, m), 8.51 (2H, s), 8.55-8.73 (2H, m).
ESI-MS Found: m/z 479[M+H]⁺

### Example 2-4:

### (E)-(3,4-difluorophenyl){5-[(7-[1,2,4]triazolo[4,3-b]pyridazin-6-yl-2,7-diazaspiro[4.4]non-2-yl)methyl]-2-pyridinyl}methanone O-ethyloxime

The entitled compound was obtained as trifluoroacetate according to the same process as in Example 2-1 but using the compound obtained in Reference Example 7-1 and 6-chloro[1,2,4]triazolo[4,3-b]pyridazine. ¹ H-NMR (400 MHz, CDCl₃, δ ppm): 1.35 (3H, t, J = 7.2 Hz), 2.20-2.30 (4H, m), 3.36-3.70 (8H, m), 4.31 (2H, q, J = 7.2 Hz), 4.38 (2H, s), 6.70 (1H, s), 7.23 (1H, m), 7.23-7.28 (2H, m), 8.04 (2H, m), 8.13 (1H, s), 8.58 (1H, s), 8.61 (1H, s).
ESI-MS Found: m/z 519[M+H]⁺

### Example 2-5:

### (E)-(5-{[7-(5-cyclopropyl-1,3,4-thiadiazol-2-yl)-2,7-diazaspiro[4.4]non-2-yl]methyl}-2-pyridinyl)(3,4-difluorophenyl)methanone O-ethyloxime

The entitled compound was obtained as trifluoroacetate according to the same process as in Example 2-1 but using the compound obtained in Reference Example 7-1 and 2-bromo-5-cyclopropyl-1,2,3-thiadiazole. ¹ H-NMR (400 MHz, CDCl₃, δ ppm): 1.00-1.08 (2H, m), 1.20-1.25 (2H, m), 1.40 (3H, t, J = 7.2 Hz), 2.12-2.40 (5H, m), 3.30-4.09 (8H, m), 4.25-4.38 (4H, m), 7.02-7.31 (3H, m), 7.90-8.15 (2H, m), 8.85 (1H, m).
ESI-MS Found: m/z 525[M+H]⁺

### Example 2-6:

### (E)-(3,4-difluorophenyl)(5-{[6-(6-fluoro-3-pyridinyl)-2,6-diazaspiro[3.3]hept-2-yl]methyl}-2-pyridinyl)methanone O-ethyloxime

The entitled compound was obtained according to the same process as in Example 2-1 but using the compound obtained in Reference Example 7-2 and 5-bromo-2-fluoropyridine.
¹ H-NMR (400 MHz, CDCl₃, δ ppm): 1.33 (3H, t, J = 7.1 Hz), 3.43 (4H, s), 3.63 (2H, s), 3.96 (4H, s), 4.30 (2H, q, J = 7.2 Hz), 6.76 (1H, dd, J = 2.9, 8.8 Hz), 6.83-6.87 (1H, m), 7.14-7.35 (4H, m), 7.67 (1H, dd, J = 2.2, 8.2 Hz), 7.79 (1H, d, J = 8.2 Hz), 8.46 (1H, d, J = 1.5 Hz). ESI-MS Found: m/z 468[M+H]⁺

### Example 2-7:

### (E)-(3,4-difluorophenyl){5-[(6-phenyl-2,6-diazaspiro[3.3]hept-2-yl)methyl]-2-pyridinyl}methanone O-ethyloxime

The entitled compound was obtained as trifluoroacetate according to the same process as in Example 2-1 but using the compound obtained in Reference Example 7-2 and bromobenzene.
¹ H-NMR (400 MHz, CDCl₃, δ ppm): 1.35 (3H, t, J = 7.2 Hz), 4.05 (4H, s), 4.15-4.40 (8H, m), 6.48 (2H, d, J = 7.6 Hz), 6.78-6.85 (1H, m), 7.10 (1H, s), 7.25-7.36 (4H, m), 7.93-8.02 (2H, m), 8.62 (1H, s).
ESI-MS Found: m/z 449[M+H]⁺

### Example 2-8:

### (E)-(3,4-difluorophenyl)(5-{[6-(2-fluorophenyl)-2,6-diazaspiro[3.3]hept-2-yl]methyl}-2-pyridinyl)methanone O-ethyloxime

The entitled compound was obtained as trifluoroacetate according to the same process as in Example 2-1 but using the compound obtained in Reference Example 7-2 and 1-bromo-2-fluorobenzene.
¹ H-NMR (400 MHz, CDCl₃, δ ppm): 1.28 (3H, t, J = 7.2 Hz), 4.10 (4H, br s), 4.15-4.53 (8H, m), 6.41-6.49 (1H, m), 6.70-6.85 (1H, m), 6.86-6.95 (2H, m), 7.02 (1H, s), 7.15-7.30 (2H, m), 7.84 (1H, d, J = 8.0 Hz), 8.02 (1H, d, J = 7.6 Hz), 8.72 (1H, s).
ESI-MS Found: m/z 467[M+H]⁺

### Example 2-9:

### (E)-(3,4-difluorophenyl)(5-{[6-(3-fluorophenyl)-2,6-diazaspiro[3.3]hept-2-yl]methyl}-2-pyridinyl)methanone O-ethyloxime

The entitled compound was obtained as trifluoroacetate according to the same process as in Example 2-1 but using the compound obtained in Reference Example 7-2 and 1-bromo-3-fluorobenzene. ¹ H-NMR (400 MHz, CDCl₃, δ ppm): 1.26 (3H, t, J = 7.6 Hz), 4.00 (4H, br s), 4.30 (8H, m), 6.13 (1H, d, J = 8.0 Hz), 6.44 (1H, dd, J = 8.4, 8.0 Hz), 7.13-7.22 (5H, m), 7.88 (1H, d, J = 8.0 Hz), 7.96 (1H, d, J = 8.4 Hz), 8.62 (1H, s).
ESI-MS Found: m/z 467[M+H]⁺

### Example 2-10:

### (E)-(3,4-difluorophenyl)(5-{[6-(4-fluorophenyl)-2,6-diazaspiro[3.3]hept-2-yl]methyl}-2-pyridinyl)methanone O-ethyloxime

The entitled compound was obtained as trifluoroacetate according to the same process as in Example 2-1 but using the compound obtained in Reference Example 7-2 and 1-bromo-4-fluorobenzene.
¹ H-NMR (400 MHz, MeOD, δ ppm): 1.31 (3H, t, J = 7.6 Hz), 3.90-4.08 (4H, m), 4.29 (2H, q, J = 7.6 Hz), 4.35-4.49 (4H, m), 4.51 (2H, s), 6.46 (2H, d, J = 6.4 Hz), 6.94 (2H, m), 7.12-7.16 (1H, m), 7.31 (2H, dd, J = 6.4, 4.0 Hz), 8.00 (2H, m), 8.59 (1H, s).
ESI-MS Found: m/z 467[M+H]⁺

### Example 2-11:

### (E)-(3,4-difluorophenyl)(5-{[6-(2-pyridinyl)-2,6-diazaspiro[3.3]hept-2-yl]methyl}-2-pyridinyl)methanone O-ethyloxime

The entitled compound was obtained according to the same process as in Example 2-1 but using the compound obtained in Reference Example 7-2 and 2-bromopyridine. ¹ H-NMR (400 MHz, DMSO-d₆, δ ppm): 1.25 (3H, t, J = 7.1 Hz), 3.33 (4H, s), 3.58 (2H, s), 3.98 (4H, s), 4.22 (2H, q, J = 7.1 Hz), 6.35 (1H, d, J = 8.3 Hz), 6.58-6.63 (1H, m), 7.12-7.18 (1H, m), 7.39-7.52 (3H, m), 7.77 (1H, dd, J = 8.1, 2.2 Hz), 7.87 (1H, d, J = 8.1 Hz), 8.04 (1H, dd, J = 4.9, 1.0 Hz), 8.39 (1H, d, J = 1.5 Hz).
APCI-MS Found: m/z 450[M+H]⁺

### Example 2-12:

### (E)-(3,4-difluorophenyl)(5-{[6-(3-pyridinyl)-2,6-diazaspiro[3.3]hept-2-yl]methyl}-2-pyridinyl)methanone O-ethyloxime

The entitled compound was obtained according to the same process as in Example 2-1 but using the compound obtained in Reference Example 7-2 and 3-bromopyridine.
¹ H-NMR (400 MHz, DMSO-d₆, δ ppm): 1.24 (3H, t, J = 7.1 Hz), 3.34 (4H, s), 3.59 (2H, s), 3.93 (4H, s), 4.22 (2H, q, J = 7.1 Hz), 6.77-6.82 (1H, m), 7.11-7.18 (1H, m), 7.39-7.52 (3H, m), 7.77 (1H, dd, J = 8.1, 2.0 Hz), 7.80 (1H, d, J = 3.0 Hz), 7.87 (1H, d, J = 8.1 Hz), 7.90 (1H, dd, J = 4.4, 1.2 Hz), 8.39 (1H, d, J = 1.5 Hz).
APCI-MS Found: m/z 450[M+H]⁺

### Example 2-13:

### (E)-(3,4-difluorophenyl)(5-{[6-(4-pyridinyl)-2,6-diazaspiro[3.3]hept-2-yl]methyl}-2-pyridinyl)methanone O-ethyloxime

The entitled compound was obtained according to the same process as in Example 2-1 but using the compound obtained in Reference Example 7-2 and 4-bromopyridine.
¹ H-NMR (400 MHz, DMSO-d₆, δ ppm): 1.25 (3H, t, J = 7.1 Hz), 3.34 (4H, s), 3.58 (2H, s), 3.97 (4H, s), 4.22 (2H, q, J = 7.1 Hz), 6.30-6.34 (2H, m), 7.12-7.18 (1H, m), 7.39-7.52 (2H, m),
7.76 (1H, dd, J = 8.1, 2.2 Hz), 7.87 (1H, d, J = 8.1 Hz), 7.87 (1H, d, J = 8.1 Hz), 8.08-8.12 (2H, m), 8.39 (1H, d, J = 1.45 Hz).
APCI-MS Found: m/z 450[M+H]⁺

### Example 2-14:

### 4-{6-[(6-{(E)-(3,4-difluorophenyl)[(ethyloxy)imino]methyl}-3-pyridinyl)methyl]-2,6-diazaspiro[3.3]hept-2-yl}benzonitrile

The entitled compound was obtained according to the same process as in Example 2-1 but using the compound obtained in Reference Example 7-2 and 4-bromobenzonitrile. ¹ H-NMR (400 MHz, DMSO-d₆, δ ppm): 1.24 (3H, t, J = 7.1 Hz), 3.34 (4H, s), 3.58 (2H, s), 4.01 (4H, s), 4.22 (2H, q, J = 7.1 Hz), 6.45 (2H, d, J = 8.8 Hz), 7.12-7.18 (1H, m), 7.36-7.52 (2H, m), 7.52 (2H, d, J = 8.8 Hz), 7.76 (1H, dd, J = 8.1, 2.2 Hz), 7.87 (1H, d, J = 8.1 Hz), 8.39 (1H, d, J = 1.5 Hz).
APCI-MS Found: m/z 474[M+H]⁺

### Example 2-15:

### (E)-(3,4-difluorophenyl)(5-{[6-(3-fluoro-2-pyridinyl)-2,6-diazaspiro[3.3]hept-2-yl]methyl}-2-pyridinyl)methanone O-ethyloxime

The entitled compound was obtained according to the same process as in Example 2-1 but using the compound obtained in Reference Example 7-2 and 2-bromo-3-fluoropyridine.
¹ H-NMR (400 MHz, DMSO-d₆, δ ppm): 1.25 (3H, t, J = 7.1 Hz), 3.35 (4H, br s), 3.59 (2H, br s), 4.12 (4H, d, J = 1.7 Hz), 4.22 (2H, q, J = 7.1 Hz), 6.65-6.70 (1H, m), 7.12-7.18 (1H, m), 7.35-7.52 (3H, m), 7.77 (1H, dd, J = 8.1, 2.0 Hz), 7.87 (1H, d, J = 8.1 Hz), 7.87-7.90 (1H, m), 8.39 (1H, s).
APCI-MS Found: m/z 468[M+H]⁺

### Example 2-16:

### (E)-(3,4-difluorophenyl)(5-{[6-(3-fluoro-4-pyridinyl)-2,6-diazaspiro[3.3]hept-2-yl]methyl}-2-pyridinyl)methanone O-ethyloxime

The entitled compound was obtained according to the same process as in Example 2-1 but using the compound obtained in Reference Example 7-2 and 4-bromo-3-fluoropyridine. ¹ H-NMR (400 MHz, DMSO-d₆, δ ppm): 1.25 (3H, t, J = 7.1 Hz), 3.42 (4H, br s), 3.64 (2H, br s), 4.15 (4H, d, J = 1.7 Hz), 4.22 (2H, q, J = 7.1 Hz), 6.47 (1H, dd, J = 8.8, 5.4 Hz), 7.12-7.18 (1H, m), 7.39-7.52 (2H, m), 7.77 (1H, dd, J = 8.1, 2.0 Hz), 7.88 (1H, d, J = 8.1 Hz), 7.99 (1H, d, J = 5.4 Hz), 8.11 (1H, d, J = 4.7 Hz), 8.40 (1H, d, J = 1.5 Hz).
APCI-MS Found: m/z 468[M+H]⁺

### Example 2-17:

### (E)-(3,4-difluorophenyl)(5-{[6-(2-fluoro-4-pyridinyl)-2,6-diazaspiro[3.3]hept-2-yl]methyl}-2-pyridinyl)methanone O-ethyloxime

The entitled compound was obtained as trifluoroacetate according to the same process as in Example 2-1 but using the compound obtained in Reference Example 7-2 and 4-bromo-2-fluoropyridine.
¹ H-NMR (400 MHz, MeOD, δ ppm): 1.32 (3H, t, J = 7.6 Hz), 4.15-4.28 (4H, m), 4.32 (2H, q, J = 7.6 Hz), 4.40-4.56 (4H, m), 4.49 (2H, s), 6.94-7.03 (1H, m), 7.10-7.18 (2H, m), 7.26-7.38 (2H, m), 7.52 (1H, d, J = 3.2 Hz), 7.99 (1H, d, J = 5.6 Hz), 8.04 (1H, d, J = 8.4 Hz), 8.59 (1H, s).
ESI-MS Found: m/z 468[M+H]⁺

### Example 2-18:

### (E)-(3,4-difluorophenyl)(5-{[6-(5-fluoro-3-pyridinyl)-2,6-diazaspiro[3.3]hept-2-yl]methyl}-2-pyridinyl)methanone O-ethyloxime

The entitled compound was obtained as trifluoroacetateaccording to the same process as in Example 2-1 but using the compound obtained in Reference Example 7-2 and 3-bromo-5-fluoropyridine.
¹ H-NMR (400 MHz, MeOD, δ ppm): 1.31 (3H, t, J = 7.6 Hz), 4.20-4.30 (4H, m), 4.32 (2H, q, J = 7.6 Hz), 4.31-4.50 (4H, s), 4.49 (2H, s), 6.91 (1H, d, J = 8.8 Hz), 7.12-7.16 (1H, m), 7.27-7.35 (2H, m), 7.72 (1H, s), 7.90 (1H, s), 7.98 (1H, d, J = 2.4 Hz), 8.02 (1H, d, J = 8.0 Hz), 8.59 (1H, s).
ESI-MS Found: m/z 468[M+H]⁺

### (E)-(3,4-difluorophenyl)[5-({6-[5-(methyloxy)-3-pyridinyl]-2,6-diazaspiro[3.3]hept-2-yl}methyl)-2-pyridinyl]methanone O-ethyloxime

The entitled compound was obtained according to the same process as in Example 2-1 but using the compound obtained in Reference Example 7-2 and 3-bromo-5-(methyloxy)pyridine. ¹ H-NMR (400 MHz, DMSO-d₆, δ ppm): 1.25 (3H, t, J = 7.1 Hz), 3.33 (4H, s), 3.59 (2H, s), 3.76 (3H, s), 3.93 (4H, s), 4.22 (2H, d, J = 7.1 Hz), 6.35 (1H, dd, J = 2.2, 2.2 Hz), 7.12-7.18 (1H, m), 7.39-7.52 (2H, m), 7.41 (1H, d, J = 2.2 Hz), 7.63 (1H, d, J = 2.2 Hz), 7.77 (1H, dd, J = 8.1, 2.2 Hz), 7.87 (1H, d, J = 8.1 Hz), 8.39 (1H, d, J = 1.5 Hz).
APCI-MS Found: m/z 480[M+H]⁺

### Example 2-20:

### (E)-[5-({6-[5-(difluoromethyl)-3-pyridinyl]-2,6-diazaspiro[3.3]hept-2-yl}methyl)-2-byridinyl](3,4-difluorophenyl)methanone O-ethyloxime

The entitled compound was obtained as trifluoroacetate according to the same process as in Example 2-1 but using the compound obtained in Reference Example 7-2 and 3-bromo-5-(difluoromethyl)pyridine. ¹ H-NMR (300 MHz, CDCl₃, δ ppm): 1.25 (3H, t, J = 6.9 Hz), 3.45-3.48 (4H, m), 3.55 (2H, s), 3.91-4.08 (4H, m), 4.28 (2H, q, J = 6.9 Hz), 6.53 (1H, t, J = 74.4 Hz), 6.73 (1H, s), 7.03-7.30 (3H, m), 7.60 (1H, d, J = 8.0 Hz), 7.73 (1H, d, J = 8.4 Hz), 7.83 (1H, s), 8.03 (1H, s), 8.40 (1H, s).
ESI-MS Found: m/z 500[M+H]⁺

### Example 2-21:

### (E)-(3,4-difluorophenyl)[5-({6-[5-(trifluoromethyl)-3-pyriainyl]-2,6-diazaspiro[3.3]hept-2-yl}methyl)-2-pyridinyl]methanone O-ethyloxime

The entitled compound was obtained as trifluoroacetate according to the same process as in Example 2-1 but using the compound obtained in Reference Example 7-2 and 3-bromo-5-(trifluoromethyl)pyridine.
¹ H-NMR (400 MHz, MeOD, δ ppm): 1.31 (3H, t, J = 7.2 Hz), 4.27 (4H, m), 4.31 (2H, q, J = 7.2 Hz), 4.46 (4H, m), 4.50 (2H, s), 7.12-7.18 (1H, m), 7.26-7.35 (3H, m), 8.00-8.06 (3H, m), 8.22-8.26 (1H, m), 8.59 (1H, s).
ESI-MS Found: m/z 518[M+H]⁺

### Example 2-22:

### (E)-(5-{[6-(5-cyclopropyl-3-pyridinyl)-2,6-diazaspiro[3.3]hept-2-yl]methyl}-2-pyridinyl)(3,4-difluorophenyl)methanone O-ethyloxime

The entitled compound was obtained as trifluoroacetate according to the same process as in Example 2-1 but using the compound obtained in Reference Example 7-2 and 3-bromo-5-cyclopropylpyridine. ¹ H-NMR (400 MHz, CDCl₃, δ ppm): 0.68-0.78 (2H, m), 1.10-1.22 (2H, m), 1.32 (3H, t, J = 7.6 Hz), 1.81-1.93 (1H, m), 4.04-4.54 (12H, m), 6.81 (1H, s), 6.95-7.05 (1H, m), 7.12-7.22 (2H, m), 7.58-7.70 (2H, m), 7.80-7.90 (1H, m), 8.05-8.20 (1H, m), 8.70-8.87 (1H, m).
ESI-MS Found: m/z 490[M+H]⁺

### Example 2-23:

### (E)-(3,4-difluorophenyl)[5-({6-[5-(1-hydroxycyclopropyl)-3-pyridinyl]-2,6-diazaspiro[3.3]hept-2-yl}methyl)-2-pyridinyl]methanone O-ethyloxime

The entitled compound was obtained as trifluoroacetate according to the same process as in Example 2-1 but using the compound obtained in Reference Example 7-2 and 3-bromo-5-(1-{[(1,1-dimethylethyl)(dimethyl)silyl]oxy}cyclopropyl)pyridine, followed by treating the product with hydrogen fluoride in acetonitrile. ¹ H-NMR (400 MHz, CDCl₃, δ ppm): 1.16-1.22 (2H, m), 1.30 (3H, t, J = 7.2 Hz), 1.33 (2H, m), 4.23-4.33 (6H, m), 4.44 (4H, br s), 4.48 (2H, s), 7.10-7.16 (1H, m), 7.22 (1H, s), 7.25-7.35 (2H, m), 7.75 (1H, s), 7.92 (1H, s), 7.94-8.03 (2H, m), 8.58 (1H, s).
ESI-MS Found: m/z 506[M+H]⁺

### Example 2-24:

### (E)-(3,4-difluorophenyl)(5-{[6-(2-methyl-4-pyridinyl)-2,6-diazaspiro[3.3]hept-2-yl]methyl}-2-pyridinyl)methanone O-ethyloxime

The entitled compound was obtained according to the same process as in Example 2-1 but using the compound obtained in Reference Example 7-2 and 4-bromo-2-methylpyridine.
¹ H-NMR (400 MHz, DMSO-d₆, δ ppm): 1.25 (3H, t, J = 7.1 Hz), 2.28 (3H, s), 2.28 (3H, s), 3.33 (4H, s), 3.58 (2H, s), 3.94 (4H, s), 4.22 (2H, q, J = 7.1 Hz), 6.15 (1H, dd, J = 5.6, 2.2 Hz), 6.18 (1H, d, J = 2.0 Hz), 7.12-7.18 (1H, m), 7.39-7.52 (2H, m), 7.76 (1H, dd, J = 8.1, 2.0 Hz), 7.86 (1H, d, J = 8.1 Hz), 7.97 (1H, d, J = 5.4 Hz), 8.39 (1H, d, J = 1.5 Hz).
APCI-MS Found: m/z 464[M+H]⁺

### Example 2-25:

### (E)-(3,4-difluorophenyl)[5-({6-[2-(methyloxy)-4-pyridinyl]-2,6-diazaspiro[3.3]hept-2-yl}methyl)-2-pyridinyl]methanone O-ethyloxime

The entitled compound was obtained as trifluoroacetate according to the same process as in Example 2-1 but using the compound obtained in Reference Example 7-2 and 4-bromo-2-(methyloxy)pyridine. ¹ H-NMR (400 MHz, MeOD, δ ppm): 1.30 (3H, t, J = 7.6 Hz), 4.05 (3H, s), 4.27 (2H, q, J = 7.6 Hz), 4.38-4.53 (10H, m), 5.97 (1H, s), 6.70 (1H, d, J = 5.2 Hz), 7.08-7.16 (1H, m), 7.24-7.33 (2H, m), 7.70 (1H, d, J = 3.2 Hz), 8.02 (2H, m), 8.59 (1H, s).
ESI-MS Found: m/z 480[M+H]⁺

### Example 2-26:

### (E)-(3,4-difluorophenyl)(5-{[6-(5-pyrimidinyl)-2,6-diazaspiro[3.3]hept-2-yl]methyl}-2-pyridinyl)methanone O-ethyloxime

The entitled compound was obtained according to the same process as in Example 2-1 but using the compound obtained in Reference Example 7-2 and 5-bromopyrimidine.
¹ H-NMR (400 MHz, DMSO-d₆, δ ppm): 1.25 (3H, t, J = 7.1 Hz), 3.34 (4H, s), 3.58 (2H, s), 4.01 (4H, s), 4.22 (2H, q, J = 7.1 Hz), 7.12-7.18 (1H, m), 7.39-7.52 (2H, m), 7.77 (1H, dd, J = 8.1, 2.2 Hz), 7.87 (1H, d, J = 8.1 Hz), 8.01 (2H, s), 8.39 (1H, d, J = 1.5 Hz), 8.52 (1H, s). APCI-MS Found: m/z 451[M+H]⁺

### Example 2-27:

### (E)-(3,4-difluorophenyl)(5-{[6-(3-pyridazinyl)-2,6-diazaspiro[3.3]hept-2-yl]methyl}-2-pyridinyl)methanone O-ethyloxime

The entitled compound was obtained as trifluoroacetate according to the same process as in Example 2-1 but using the compound obtained in Reference Example 7-2 and 3-bromopyridazine.
¹ H-NMR (400 MHz, MeOD, δ ppm): 1.29 (3H, t, J = 7.2 Hz), 4.30 (2H, q, J = 7.2 Hz), 4.42-4.58 (10H, m), 7.15 (1H, m), 7.28-7.39 (3H, m), 7.77 (1H, m), 7.96-8.03 (2H, m), 8.50 (1H, s), 8.60 (1H, s).
ESI-MS Found: m/z 451[M+H]⁺

### Example 2-28:

### 4-{6-[(6-{(E)-(3,4-difluorophenyl)[(ethyloxy)imino]methyl}-3-pyridinyl)methyl]-2,6-diazaspiro[3.3]hept-2-yl}-2(1H)-pyridinone

The entitled compound was obtained as trifluoroacetate according to the same process as in Example 2-1 but using the compound obtained in Reference Example 7-2 and 4-iodo-2(1H)-pyridinone.
¹ H-NMR (400 MHz, MeOD, δ ppm): 1.29 (3H, t, J = 7.6 Hz), 4.30 (2H, q, J = 7.6 Hz), 4.30-4.44 (4H, m), 4.40-4.55 (6H, m), 5.72 (1H, s), 6.23 (1H, d, J = 7.6 Hz), 7.10-7.16 (1H, m), 7.26-7.38 (2H, m), 7.60 (1H, d, J = 7.6 Hz), 8.00 (2H, m), 8.60 (1H, s).
ESI-MS Found: m/z 466[M+H]⁺

### Example 2-29:

### 6-{6-[(6-{(E)-(3,4-difluorophenyl)[(ethyloxy)imino]methyl}-3-pyridinyl)methyl]-2,6-diazaspiro[3.3]hept-2-yl}-1-methyl-2(1H)-pyridinone

The entitled compound was obtained as trifluoroacetate according to the same process as in Example 2-1 but using the compound obtained in Reference Example 7-2 and 6-bromo-1-methyl-2(1H)-pyridinone.
¹ H-NMR (400 MHz, MeOD, δ ppm): 1.31 (3H, t, J = 7.6 Hz), 3.44 (3H, s), 4.20-4.36 (4H, m), 4.30 (2H, q, J = 7.6 Hz), 4.35-4.52 (4H, m), 4.48 (2H, s), 7.16 (1H, m), 7.28-7.35 (3H, m), 7.37-7.43 (1H, m), 7.93-8.17 (3H, m), 8.59 (1H, s).
ESI-MS Found: m/z 480[M+H]⁺

### Example 2-30:

### (E)-(3,4-difluorophenyl)(5-{[6-(3-isothiazolyl)-2,6-diazaspiro[3.3]hept-2-yl]methyl}-2-pyridinyl)methanone O-ethyloxime

The entitled compound was obtained as trifluoroacetate according to the same process as in Example 2-1 but using the compound obtained in Reference Example 7-2 and 2-bromoisothiazole. ¹ H-NMR (400 MHz, MeOD, δ ppm): 1.31 (3H, t, J = 7.6 Hz), 4.15 (4H, m), 4.32 (2H, q, J = 7.6 Hz), 4.33-4.49 (4H, m), 4.48 (2H, s), 6.55 (1H, s), 7.29 (1H, m), 7.34 (2H, m), 8.00 (2H, m), 8.59 (1H, s), 8.65 (1H, s).
ESI-MS Found: m/z 456[M+H]⁺

### Example 2-31:

### (E)-(3,4-difluorophenyl)(5-{[6-(1,2,5-thiadiazol-3-yl)-2,6-diazaspiro[3.3]hept-2-yl]methyl}-2-pyridinyl)methanone O-ethyloxime

The entitled compound was obtained according to the same process as in Example 2-1 but using the compound obtained in Reference Example 7-2 and 3-bromo-1,2,5-thiadiazole.
¹ H-NMR (400 MHz, DMSO-d₆, δ ppm): 1.25 (3H, t, J = 7.1 Hz), 3.35 (4H, br s), 3.58 (2H, br s), 4.16 (4H, s), 4.22 (2H, q, J = 7.1 Hz), 7.12-7.18 (1H, m), 7.39-7.52 (2H, m), 7.73-7.81 (1H, m), 7.84-7.90 (1H, m), 8.12 (1H, s), 8.39 (1H, s).
APCI-MS Found: m/z 457[M+H]⁺

### Example 2-32:

### (E)-(3,4-difluorophenyl)(5-{[6-(1,5-naphthyridin-4-yl)-2,6-diazaspiro[3.3]hept-2-yl]methyl}-2-pyridinyl)methanone O-ethyloxime

The entitled compound was obtained according to the same process as in Example 2-1 but using the compound obtained in Reference Example 7-2 and 4-bromo-1,5-naphthyridine. ¹ H-NMR (400 MHz, DMSO-d₆, δ ppm): 1.25 (3H, t, J = 7.1 Hz), 3.41 (4H, s), 3.62 (2H, s), 4.22 (2H, q, J = 7.1 Hz), 4.50 (4H, br s), 6.37 (1H, d, J = 5.4 Hz), 7.13-7.18 (1H, m), 7.39-7.52 (2H, m), 7.61 (1H, dd, J = 8.6,1.7 Hz), 8.39 (1H, d, J = 5.4 Hz), 8.41 (1H, d, J = 1.5 Hz), 8.70 (1H, dd, J = 4.2, 1.7 Hz).
APCI-MS Found: m/z 501[M+H]⁺

### Example 2-33:

### (E)-(3,4-difluorophenyl)(5-{[6-(5-quinoxalinyl)-2,6-diazaspiro[3.3]hept-2-yl]methyl}-2-pyridinyl)methanone O-ethyloxime

The entitled compound was obtained according to the same process as in Example 2-1 but using the compound obtained in Reference Example 7-2 and 5-bromoquinoxaline. ¹ H-NMR (400 MHz, DMSO-d₆, δ ppm): 1.25 (3H, t, J = 7.1 Hz), 3.39 (4H, s), 3.61 (2H, s), 4.22 (2H, q, J = 7.1 Hz), 4.34 (4H, br s), 6.58 (1H, d, J = 7.6 Hz), 7.13-7.18 (1H, m), 7.29 (1H, d, J = 8.3 Hz), 7.39-7.52 (2H, m), 7.59 (1H, dd, J = 8.1, 8.1 Hz), 7.76-7.82 (1H, m), 7.85-7.90 (1H, m), 8.41 (1H, br s), 8.70 (1H, d, J = 1.7 Hz), 8.81 (1H, d, J = 1.7 Hz).
APCI-MS Found: m/z 501[M+H]⁺

### Example 2-34:

### (E)-(3,4-difluoropheny)(5-{[6-(7-quinazolinyl)-2,6-diazaspiro[3.3]hept-2-yl]methyl}-2-pyridinyl)methanone O-ethyloxime

The entitled compound was obtained according to the same process as in Example 2-1 but using the compound obtained in Reference Example 7-2 and 7-bromoquinazoline. ¹ H-NMR (400 MHz, DMSO-d₆, δ ppm): 1.25 (3H, t, J = 7.1 Hz), 3.40 (4H, br s), 3.63 (2H, br s), 4.15 (4H, s), 4.22 (2H, q, J = 7.1 Hz), 6.56 (1H, d, J = 1.5 Hz), 6.95 (1H, dd, J = 8.8, 2.2 Hz), 7.12-7.18 (1H, m), 7.38-7.52 (2H, m), 7.72-7.82 (1H, m), 7.86 (2H, d, J = 8.8 Hz), 8.35-8.45 (1H, m), 8.94 (1H, s), 9.12 (1H, s).
APCI-MS Found: m/z 501[M+H]⁺

### Example 2-35:

### (E)-(3,4-difluorophenyl){5-[(6-imidazo[1,2-a]pyrimidin-6-yl-2,6-diazaspiro[3.3]hept-2-yl)methyl]-2-pyridinyl}methanone O-ethyloxime

The entitled compound was obtained as trifluoroacetate according to the same process as in Example 2-1 but using the compound obtained in Reference Example 7-2 and 6-bromoimidazo[1,2-a]pyrimidine.
¹ H-NMR (400 MHz, MeOD, δ ppm): 1.30 (3H, t, J = 7.6 Hz), 4.27 (2H, q, J = 7.6 Hz), 4.29-4.50 (10H, m), 6.32-6.35 (1H, m), 7.17-7.20 (1H, m), 7.29-7.37 (2H, m), 7.88-8.05 (4H, m), 8.36-8.40 (1H, m), 8.60 (1H, m).
ESI-MS Found: m/z 490[M+H]⁺

### Example 2-36:

### (E)-(3,4-difluorophenyl){5-[(6-[1,2,4]triazolo[4,3-a]pyridin-7-yl-2,6-diazaspiro[3.3]hept-2-yl)methyl]-2-pyridinyl}methanone O-ethyloxime

The entitled compound was obtained as trifluoroacetate according to the same process as in Example 2-1 but using the compound obtained in Reference Example 7-2 and 7-bromo[1,2,4]triazolo[4,3-a]pyridine. ¹ H-NMR (400 MHz, MeOD, δ ppm): 1.29 (3H, t, J = 7.6 Hz), 4.26 (2H, q, J = 7.6 Hz), 4.37-4.46 (10H, m), 6.30 (1H, s), 6.73 (1H, d, J = 7.6 Hz), 7.10-7.16 (1H, m), 7.26-7.30 (2H, example 2-37m), 7.95 (1H, d, J = 4.8 Hz), 7.98 (1H, s), 8.40 (1H, d, J = 7.6 Hz), 8.56 (1H, s), 8.90 (1H, s).
ESI-MS Found: m/z 490[M+H]⁺

### (E)-(3,4-difluorophenyl){5-[(6-imidazo[1,2-a]pyridin-6-yl-2,6-diazaspiro[3.3]hept-2-yl)methyl] 2-pyridinyl}methanone O-ethyloxime

The entitled compound was obtained as trifluoroacetate according to the same process as in Example 2-1 but using the compound obtained in Reference Example 7-2 and 6-bromoimidazo[1,2-a]pyridine.
¹ H-NMR (400 MHz, MeOD, δ ppm): 1.26 (3H, t, J = 7.6 Hz), 4.18-4.34 (4H, m), 4.33 (2H, q, J = 7.6 Hz), 4.33-4.48 (4H, m), 4.58 (2H, s), 7.15 (1H, m), 7.24-7.35 (2H, m), 7.48 (1H, m), 7.75 (1H, d, J = 7.6 Hz), 7.80-7.93 (2H, m), 8.02 (3H, s), 8.59 (1H, s).
ESI-MS Found: m/z 489[M+H]⁺

### Example 2-38:

### (E)-(3,4-difluorophenyl)(5-{[6-(2-methylimidazo[1,2-a]pyridin-6-yl)-2,6-diazaspiro[3.3]hept-2-yl]methyl}-2-pyridinyl)methanone O-ethyloxime

The entitled compound was obtained as trifluoroacetate according to the same process as in Example 2-1 but using the compound obtained in Reference Example 7-2 and 6-bromo-2-methylimidazo [1,2-a]pyridine. ¹ H-NMR (400 MHz, MeOD, δ ppm): 1.26 (3H, t, J = 7.6 Hz), 2.56 (3H, s), 4.16-4.33 (4H, m), 4.33 (2H, q, J = 7.6 Hz), 4.50-4.64 (4H, m), 4.66 (2H, s), 7.15 (1H, m), 7.25-7.34 (3H, m), 7.65 (1H, m), 7.75 (1H, s), 7.83 (1H, s), 8.02 (2H, m), 8.59 (1H, s).
ESI-MS Found: m/z 503[M+H]⁺

### Example 2-39:

### (E)-(3,4-difluorophenyl)(5-{[6-(2,3-dimethylimidazo[1,2-a]pyridin-6-yl)-2,6-diazaspiro[3.3]hept-2-yl]methyl}-2-pyridinyl)methanone O-ethyloxime

The entitled compound was obtained as trifluoroacetate according to the same process as in Example 2-1 but using the compound obtained in Reference Example 7-2 and 6-bromo-2,3-dimethylimidazo[1,2-a]pyridine. ¹ H-NMR (400 MHz, MeOD, δ ppm): 1.26 (3H, t, J = 7.6 Hz), 2.48 (3H, s), 2.49 (3H, s), 4.15-4.30 (4H, m), 4.33 (2H, q, J = 7.6 Hz), 4.40-4.55 (4H, m), 4.58 (2H, s), 7.15 (1H, m), 7.43 (3H, m), 7.48 (1H, s), 7.65 (1H, s), 8.02 (2H, m), 8.59 (1H, s).
ESI-MS Found: m/z 517[M+H]⁺

### Example 2-40:

### (E)-(3,4-difluorophenyl){5-[(6-imidazo[1,2-a]pyridin-7-yl-2,6-diazaspiro[3.3]hept-2-yl)methyl]-2-pyridinyl}methanone O-ethyloxime

The entitled compound was obtained as trifluoroacetate according to the same process as in Example 2-1 but using the compound obtained in Reference Example 7-2 and 7-bromoimidazo[1,2-a]pyridine.
¹ H-NMR (400 MHz, MeOD, δ ppm): 1.26 (3H, t, J = 7.6 Hz), 4.43 (2H, q, J = 7.6 Hz), 4.31-4.45 (4H, m), 4.40-4.53 (4H, m), 4.58 (2H, s), 6.37 (1H, d, J = 5.6 Hz), 6.66 (1H, d, J = 5.6 Hz), 7.16 (1H, m), 7.48 (2H, m), 7.58 (1H, s), 7.75 (1H, s), 8.02 (2H, m), 8.48 (1H, m), 8.59 (1H, s). ESI-MS Found: m/z 489[M+H]⁺

### Example 2-41:

### (E)-(3,4-difluorophenyl)(5-{[6-(2-methylimidazo[1,2-a]pyridin-7-yl)-2,6-diazaspiro[3.3]hept-2-yl]methyl}-2-pyridinyl)methanone O-ethyloxime

The entitled compound was obtained as trifluoroacetate according to the same process as in Example 2-1 but using the compound obtained in Reference Example 7-2 and 7-bromo-2-methylimidazo [1,2-a]pyridine.
¹ H-NMR (400 MHz, MeOD, δ ppm): 1.33 (3H, t, J = 7.2 Hz), 2.36 (3H, s), 4.26-4.49 (12H, m), 6.27 (1H, d, J = 1.6 Hz), 6.60 (1H, dd, J = 7.2, 2.0 Hz), 7.11-7.14 (1H, m), 7.27-7.34 (2H, m), 7.44 (1H, s), 7.99-8.03 (2H, m), 8.26 (1H, d, J = 7.6 Hz), 8.56-8.58 (1H, m).
ESI-MS Found: m/z 503[M+H]⁺

### Example 2-42:

### (E)-(3,4-difluorophenyl)(5-{[6-(2-ethylimidazo[1,2-a]pyridin-7-yl)-2,6-diazaspiro[3.3]hept-2-yl]methyl}-2-pyridinyl)methanone O-ethyloxime

The entitled compound was obtained as trifluoroacetate according to the same process as in Example 2-1 but using the compound obtained in Reference Example 7-2 and 7-bromo-2-ethylimidazo[1,2-a]pyridine. ¹ H-NMR (400 MHz, MeOD, δ ppm): 1.29-1.31 (6H, m), 2.67 (2H, q, J = 7.6 Hz), 4.18-4.45 (12H, m), 6.19 (1H, s), 6.53 (1H, dd, J = 7.2, 2.0 Hz), 7.20-7.10 (1H, m), 7.19-7.26 (2H, m), 7.39 (1H, s), 7.92 (2H, m), 8.19 (1H, d, J = 7.6 Hz), 8.42-8.55 (1H, m).
ESI-MS Found: m/z 517[M+H]⁺

### Example 2-43:

### (E)-(3,4-difluorophenyl)(5-{[6-(3-ethylimidazo[1,2-a]pyridin-7-yl)-2,6-diazaspiro[3.3]hept-2-yl]methyl}-2-pyridinyl)methanone O-ethyloxime

The entitled compound was obtained as trifluoroacetate according to the same process as in Example 2-1 but using the compound obtained in Reference Example 7-2 and 7-bromo-3-ethylimidazo[1,2-a]pyridine. ¹ H-NMR (400 MHz, MeOD, δ ppm): 1.29 (3H, t, J = 7.2 Hz), 1.35 (3H, t, J = 7.2 Hz), 2.88 (2H, q, J = 7.2 Hz), 4.27 (2H, q, J = 7.2 Hz), 4.31-4.48 (10H, m), 6.33 (1H, s), 6.70 (1H, d, J = 7.2 Hz), 7.10-7.18 (1H, m), 7.26-7.36 (3H, m), 7.90-8.08 (2H, m), 8.30 (1H, d, J = 7.6 Hz), 8.56 (1H, s).
ESI-MS Found: m/z 517[M+H]⁺

### Example 2-44:

### (E)-(3,4-difluorophenyl)(5-{[6-(3-propylimidazo[1,2-a]pyridin-7-yl)-2,6-diazaspiro[3.3]hept-2-yl]methyl}-2-pyridinyl)methanone O-ethyloxime

The entitled compound was obtained as trifluoroacetate according to the same process as in Example 2-1 but using the compound obtained in Reference Example 7-2 and 7-bromo-3-propylimidazo[1,2-a]pyridine. ¹ H-NMR (400 MHz, MeOD, δ ppm): 1.05 (3H, t, J = 7.2 Hz), 1.30 (3H, t, J = 6.8 Hz), 1.75 (2H, m), 2.81 (2H, m), 4.27 (2H, q, J = 7.2 Hz), 4.35 (4H, br s), 4.46 (4H, br s), 4.49 (2H, br s), 6.33 (1H, s), 6.70 (1H, d, J = 7.6 Hz), 7.10-7.18 (1H, m), 7.26-7.48 (3H, m), 8.00 (2H, m), 8.29 (1H, d, J = 7.6 Hz), 8.49 (1H, s).
ESI-MS Found: m/z 531[M+H]⁺

### Example 2-45:

### (E)-(3,4-difluorophenyl)(5-{[6-(2,3-dimethylimidazo[1,2-a]pyridin-7-yl)-2,6-diazaspiro[3.3]hept-2-yl]methyl}-2-pyridinyl)methanone O-ethyloxime

The entitled compound was obtained as trifluoroacetate according to the same process as in Example 2-1 but using the compound obtained in Reference Example 7-2 and 7-bromo-2,3-dimethylimidazo[1,2-a]pyridine. ¹ H-NMR (400 MHz, CDCl₃, δ ppm): 1.33 (3H, t, J = 7.2 Hz), 2.25 (3H, s), 2.25 (3H, s), 4.20 (2H, q, J = 7.2 Hz), 4.30-4.54 (10H, m), 6.26-6.53 (2H, m), 6.98-7.25 (3H, m), 7.63-7.75 (1H, m), 7.78-8.03 (2H, m), 8.48-8.70 (1H, m).
ESI-MS Found: m/z 517[M+H]⁺

### Example 2-46:

### (E)-(3,4-difluorophenyl)(5-{[6-(6,7,8,9-tetrahydropyrido[1,2-a]benzimidazol-3-yl)-2,6-diazaspiro[3.3]hept-2-yl]methyl}-2-pyridinyl)methanone O-ethyloxime

The entitled compound was obtained as trifluoroacetate according to the same process as in Example 2-1 but using the compound obtained in Reference Example 7-2 and 3-bromo-6,7,8,9-tetrahydropyrido[1, 2-a]benzimidazole. ¹ H-NMR (400 MHz, MeOD, δ ppm): 1.27-1.31 (7H, m), 1.92-2.02 (3H, m), 2.69-2.74 (2H, m), 4.26-4.48 (12H, m), 6.33 (1H, d, J = 2.4 Hz), 6.64-6.66 (1H, m), 7.11-7.15 (1H, m), 7.27-7.34 (2H, m), 7.96-8.05 (2H, m), 8.17 (1H, d, J = 7.6 Hz), 8.58 (1H, s).
ESI-MS Found: m/z 543[M+H]⁺

### Example 2-47:

### (E)-[5-({6-[2-(difluoromethyl)imidazo[1,2-a]pyridin-7-yl]-2,6-diazaspiro[3.3]hept-2-yl}methyl)-2-pyridinyl](3,4-difluorophenyl)methanone O-ethyloxime

The entitled compound was obtained as trifluoroacetate according to the same process as in Example 2-1 but using the compound obtained in Reference Example 7-2 and 7-bromo-2-(difluoromethyl)imidazo[1.2-a]pyridine.
¹ H-NMR (400 MHz, CDCl₃, δ ppm): 1.29 (3H, t, J = 7.2 Hz), 3.50 (4H, br s), 3.70 (2H, s), 4.05 (4H, br s), 4.26 (2H, q, J = 7.2 Hz), 6.18 (1H, s), 6.38 (1H, dd, J = 7.6, 2.0 Hz), 6.77 (1H, t, J = 55.6 Hz), 7.08-7.18 (1H, m), 7.23-7.40 (2H, m), 8.17 (1H, d, J = 7.6 Hz), 8.20-8.35 (3H, m), 8.42 (1H, s).
ESI-MS Found: m/z 539[M+H]⁺

### Example 2-48:

### (E)-(3,4-difluorophenyl)(5-{[7-(6-fluoro-3-pyridinyl)-2,7-diazaspiro[3.5]non-2-yl]methyl}-2-pyridinyl)methanone O-ethyloxime

The entitled compound was obtained as trifluoroacetate according to the same process as in Example 2-1, for which, however, the compound obtained in Reference Example 6-3 was treated in the same manner as in Reference Example 7-1 and then reacted with 5-bromo-2-fluoropyridine. ¹ H-NMR (400 MHz, CDCl₃, δ ppm): 1.36 (3H, t, J = 7.6 Hz), 1.70-2.03 (4H, m), 2.90-3.09 (4H, m), 3.38-3.65 (2H, m), 4.00-4.33 (6H, m), 6.78 (1H, s), 6.98-7.30 (4H, m), 7.68 (1H, s), 7.73-7.95 (2H, m), 8.51 (1H, s).
ESI-MS Found: m/z 496[M+H]⁺

### Example 2-49:

### (E)-(3,4-difluorophenyl)(5-{[6-(6-fluoro-3-pyridinyl)-2,6-diazaspiro[3.5]non-2-yl]methyl}-2-pyridinyl)methanone O-ethyloxime

The entitled compound was obtained as trifluoroacetate according to the same process as in Example 2-1, for which, however, the compound obtained in Reference Example 6-4 was treated in the same manner as in Reference Example 7-1 and then reacted with 5-bromo-2-fluoropyridine. ¹ H-NMR (400 MHz, CDCl₃, δ ppm): 1.29 (3H, t, J = 7.6 Hz), 1.70-1.83 (4H, m), 3.30 (2H, m), 3.33 (2H, m), 4.13-4.46 (8H, m), 6.83-6.88 (1H, m), 7.06-7.09 (1H, m), 7.19-7.28 (2H, m), 7.45 (1H, s), 7.77 (1H, s), 7.90 (1H, d, J = 8.4 Hz), 8.07 (1H, d, J = 8.4 Hz), 8.75 (1H, s).
ESI-MS Found: m/z 496[M+H]⁺

### Example 2-50:

### (E)-(3,4-difluorophenyl)(5-{[8-(6-fluoro-3-pyridinyl)-2,8-diazaspiro[4.5]dec-2-yl]methyl}-2-pyridinyl)methanone O-ethyloxime

The entitled compound was obtained as trifluoroacetate according to the same process as in Example 2-1, for which, however, the compound obtained in Reference Example 6-5 was treated in the same manner as in Reference Example 7-1 and then reacted with 5-bromo-2-fluoropyridine. ¹ H-NMR (400 MHz, CDCl₃, δ ppm): 1.29 (3H, t, J = 7.2 Hz), 1.75-1.85 (4H, m), 2.02-2.08 (2H, m), 2.95-3.18 (5H, m), 3.20-3.75 (3H, m), 4.31 (2H, q, J = 7.2 Hz), 4.48 (2H, s), 6.75-6.86 (2H, m), 6.95-7.05 (2H, m), 7.40-7.45 (1H, m), 7.75-7.80 (2H, m), 8.20-8.27 (1H, m), 9.15 (1H, s).
ESI-MS Found: m/z 510[M+H]⁺

### Example 2-51:

### (E)-(3,4-difluorophenyl)(5-{[2-(6-fluoro-3-pyridinyl)-2,7-diazaspiro[3.5]non-7-yl]methyl}-2-pyridinyl)methanone O-ethyloxime

The entitled compound was obtained as trifluoroacetate according to the same process as in Example 2-1, for which, however, the compound obtained in Reference Example 6-6 was treated in the same manner as in Reference Example 7-1 and then reacted with 5-bromo-2-fluoropyridine. ¹ H-NMR (400 MHz, CDCl₃, δ ppm): 1.29 (3H, t, J = 7.6 Hz), 2.12 (4H, m), 2.61-2.94 (2H, m), 3.40-3.59 (2H, m), 3.64 (4H, s), 4.26 (2H, q, J = 7.6 Hz), 4.31 (2H, s), 6.76-6.84 (2H, m), 7.03 (1H, d, J = 4.4 Hz), 7.16-7.21 (2H, m), 7.30 (1H, s), 7.83 (1H, d, J = 8.0 Hz), 8.13 (1H, d, J = 7.6 Hz), 8.79 (1H, s).
ESI-MS Found: m/z 496[M+H]⁺

In addition to the above-mentioned Examples, the following compounds were produced in the same manner as above.

### Example 2-52:

5-[6-({6-[(E)-{[(2-hydroxy-2-methylpropyl)oxy]imino}(3,4,5-trifluorophenyl)methyl]-3-pyridinyl}methyl)-2,6-diazaspiro[3.3]hept-2-yl]-3-pyridinecarbonitrile
¹ H-NMR (400 MHz, MeOD, δ ppm): 1.17 (6H, s), 4.14 (2H, s), 4.20 (4H, br s), 4.42 (4H, br s), 4.47 (2H, s), 7.19 (3H, m), 7.92-8.04 (2H, m), 8.05-8.10 (1H, m), 8.18 (1H, s), 8.56 (1H, s). ESI-MS Found: m/z 537[M+H]⁺

### Example 2-53:

5-(6-{[6-((E)-(4-chloro-3,5-difluorophenyl)([(2-hydroxy-2-methylpropyl)oxy]imino}methyl)-3-pyridinyl)methyl)-2,6-diazaspiro[3.3]hept-2-yl)-3-pyridinecarbonitrile ¹ H-NMR (400 MHz, MeOD, δ ppm): 1.18 (6H, s), 4.14 (2H, s), 4.22 (4H, br s), 4.42 (4H, br s), 4.49 (2H, s), 7.17 (1H, s), 7.18-7.22 (2H, m), 7.92-8.02 (2H, m), 8.05-8.10 (1H, m), 8.20 (2H, s), 8.64 (1H, s).
ESI-MS Found: m/z 553[M+H]⁺

### Example 2-54:

### 5-(6-{(1R) or (1S)-1-[6-((E)-(4-chloro-3,5-difluorophenyl)([(2-hydroxy-2-methylpropyl)oxy]imino}methyl)-3-pyridinyl]ethyl}-2,6-diazaspiro[3.3]hept-2-yl)-3-pyridinecarbonitrile

After the racemic compound was prepared, this was optically resolved under the condition mentioned below to give two enantiomers.
Supercritical Fluid Chromatography (SFC), CHIRALPAK AD-H (methanol, 0.1 % diethylamine, 40 % carbon dioxide), 2nd eluate enantiomer:
¹ HNMR (400 MHz, CDCl₃ , δ ppm): 1.18 (9H, s), 2.94 (1H, m), 3.45 (4H, br s), 3.99 (4H, br s), 4.09 (2H, s), 6.78 (1H, s), 6.99 (2H, d, J = 7.2 Hz), 7.77 (2H, m), 7.90 (1H, d, J = 2.8 Hz), 8.16 (1H, s), 8.42 (1H, s).
ESI-MS Found: m/z 567[M+H]⁺

### Example 2-55:

### (E)-(3,4-difluorophenyl)(5-{[(5R) or (5S)-7-(2-methyl-4-pyridinyl)-2,7-diazaspiro[4.4]non-2-yl]methyl}-2-pyridinyl)methanone O-(2-hydroxy-2-methylpropyl)oxime

After the racemic compound was prepared, this was optically resolved under the condition mentioned below to give two enantiomers.

CHIRALPAK AD-H (hexane/ethanol/diethylamine = 60/60/0.04), 2nd eluate enantiomer: ¹ H-NMR (400 MHz, MeOD, δ ppm): 1.17 (6H, s), 2.21-2.28 (4H, m), 2.50 (3H, s), 3.61-3.65 (8H, m), 4.13 (2H, s), 4.54 (2H, s), 6.62-6.74 (2H, m), 7.17-7.21 (3H, m), 7.99 (2H, m), 8.05-8.63 (1H, m), 8.54 (1H, s).
ESI-MS Found: m/z 536[M+H]⁺

### INDUSTRIAL APPLICABILITY

The compounds of the invention have an MCH-1R antagonistic effect and are useful as a preventive or remedy for metabolic disorders such as obesity, diabetes, hormone disorder, hyperlipidemia, gout, fatty liver, hepatitis, and cirrhosis; cardiovascular disorders such as stenocardia, acute/congestive heart failure, myocardial infarction, coronary atherosclerosis, hypertension, renal diseases and electrolyte abnormality; central and peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence and alcoholism; reproductive disorders such as infertility, preterm labor and sexual dysfunction; other digestive disorders, respiratory disorders, cancer or pigmentation.

## Claims

1. A compound of a formula (I) or a pharmaceutically-acceptable salt thereof: 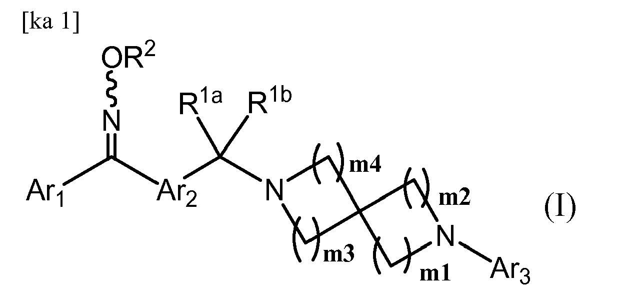 [wherein,
R^{1 a} and R^{1 b} each independently represent a hydrogen atom, or a C₁₋₆ alkyl optionally substituted with a halogen or a hydroxy, or R^{1 a} and R^{1 b}, taken together, form a cyclopropyl;
R² represents a hydrogen atom, a C₁₋₆ alkyl or a C₃₋₆ cycloalkyl, wherein the alkyl or the cycloalkyl may be optionally substituted with a substituent selected from a group consisting of a halogen, a hydroxy, a C₁₋₆ alkyloxy, a C₁₋₆ alkyloxycarbonyl, a C₁₋₆ alkylsulfonyl, a mono(C₁₋₆ alkyl)amino, a di(C₁₋₆ alkyl)amino, a carbamoyl, a mono(C₁₋₆ alkyl)carbamoyl, a di(C₁₋₆ alkyl)carbamoyl and cyano;
Ar₁ represents a 6-membered aromatic carbocyclic group optionally mono- to tetra-substituted with a substituent selected from the group α, or represents a 6-membered aromatic nitrogen-containing heterocyclic group optionally mono- to tetra-substituted with a substituent selected from the group α,
Ar₂ represents a group to be formed by removing two hydrogen atoms from a 6-membered aromatic carbon ring, a 6-membered aromatic nitrogen-containing hetero ring, a 5-membered aromatic hetero ring or a pyridone ring, wherein the 6-membered aromatic carbon ring, the 6-membered aromatic nitrogen-containing hetero ring, the 5-membered aromatic hetero ring or the pyridone ring may be optionally substituted with a substituent selected from the group α;
Ar₃ represents a mono- or bi-cyclic aromatic carbocyclic group or aromatic heterocyclic group, or a pyridone, wherein the aromatic carbon ring or the aromatic hetero ring may form a fused ring with a non-aromatic cyclic hydrocarbon or a non-aromatic hetero ring, and wherein the aromatic carbocyclic group, the aromatic heterocyclic group or the pyridone may be optionally mono- to tetra-substituted with a substituent selected from a halogen, a C₁₋₆ alkyl, a halo-C₁₋₆ alkyl, a hydroxy-C₁₋₆ alkyl, a C₁₋₆ alkyloxy, a halo-C₁₋₆ alkyloxy, a C₃₋₆ cycloalkyl, a hydroxy-C₃₋₆ cycloalkyl, a cyano, a carbamoyl, a mono-C₁₋₆ alkylcarbamoyl, a di-C₁₋₆ alkylcarbamoyl, a C₁₋₆ alkylsulfonyl and a sulfonylamide;
m1, m2, m3 and m4 each independently indicate 0, 1, 2, 3 or 4, provided that the total of m1 and m2 is from 2 to 6, and the total of m3 and m4 is from 2 to 6, and any -CH₂- forming the spiro ring may be replaced by -O- and/or -C(O)-;
Substituents of group α:
a halogen, a cyano, a hydroxy, an amino, a mono-C₁₋₆ alkylamino, a di-C₁₋₆ alkylamino, a C₁₋₆ alkyl, a halo-C₁₋₆ alkyl, a C₁₋₆ alkyloxy, a halo-C₁₋₆ alkyloxy, a C₁₋₆ alkyloxy-C₁₋₆ alkyl, a C₁₋₆ alkyloxycarbonyl, a C₁₋₆ alkyloxycarbonylamino, a C₁₋₆ alkyloxycarbonyl(C₁₋₆ alkyl)amino, a C₁₋₆ alkylcarbonyl, a C₁₋₆ alkylcarbonyloxy, a C₁₋₆ alkylcarbonylamino, a C₁₋₆ alkylcarbonyl(C₁₋₆ alkyl)amino, a carbamoyl, a mono-C₁₋₆ alkylcarbamoyl, a di-C₁₋₆ alkylcarbamoyl, a carbamoylamino, a mono-C₁₋₆ alkylcarbamoylamino, a di-C₁₋₆ alkylcarbamoylamino, a mono-C₁₋₆ alkylcarbamoyl(C₁₋₆ alkyl)amino, a di-C₁₋₆ alkylcarbamoyl(C₁₋₆ alkyl)amino, a carbamoyloxy, a mono-C₁₋₆ alkylcarbamoyloxy, a di-C₁₋₆ alkylcarbamoyloxy, a C₁₋₆ alkylsulfonyl, a C₁₋₆ alkylsulonylamino, a C₁₋₆ atkylsulfonyl(C₁₋₆ alkyl)amino, a sulfamoyl, a mono-C₁₋₆ alkylsulfamoyl, a di-C₁₋₆ alkylsulfamoyl, a sulfamoylamino, a mono-C₁₋₆ alkylsulfamoylamino, a di-C₁₋₆ alkylsulfamoylamino, a mono-C₁₋₆ alkylsulfamoyl(C₁₋₆ alkyl)amino, and a di-C₁₋₆ alkylsulfamoyl(C₁₋₆ alkyl)amino].

2. The compound or the pharmaceutically-acceptable salt thereof as claimed in claim 1, wherein R^{1 a} and R^{1 b} each are independently a hydrogen atom or a methyl.

3. The compound or the pharmaceutically-acceptable salt thereof as claimed in claim 1 or 2, wherein Ar₁ is a 6-membered aromatic carbocyclic group substituted with from 1 to 3 fluorine atoms or chlorine atoms, or a 6-membered aromatic nitrogen-containing heterocyclic group substituted with from 1 to 3 fluorine atoms or chlorine atoms.

4. The compound or the pharmaceutically-acceptable salt thereof as claimed in claim 3, wherein Ar₁ is a phenyl substituted with from 1 to 3 fluorine atoms or chlorine atoms, or a pyridinyl substituted with from 1 to 3 fluorine atoms or chlorine atoms.

5. The compound or the pharmaceutically-acceptable salt thereof as claimed in any of claims 1 to 4, wherein Ar₂ is a group to be formed by removing two hydrogen atoms from a benzene ring, a pyridine ring, a pyrimidine ring, a pyrazine ring, a pyridazine ring or a pyridone ring, wherein the group may be optionally substituted with a substituent selected from the group α.

6. The compound or the pharmaceutically-acceptable salt thereof as claimed in claim 5, wherein Ar₂ is 1,4-phenylenediyl, 3-methoxyphenylene-1,4-diyl, 3-methanesulfonylphenylene-1,4-diyl, 2-fluorophenylene-1,4-diyl, 3-fluorophenylene-1,4-diyl, 2-methylphenylene-1,4-diyl, 3-methylphenylene-1,4-diyl, pyridine-2,5-diyl, pyrimidine-2,5-diyl, pyrazine-2,5-diyl, pyridazine-3,6-diyl, thiophene-2,5-diyl or pyridonediyl.

7. The compound or the pharmaceutically-acceptable salt thereof as claimed in any of claims 1 to 6, wherein Ar₃ is selected from the following group:

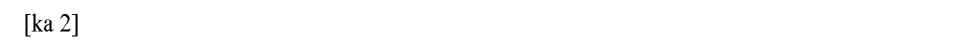

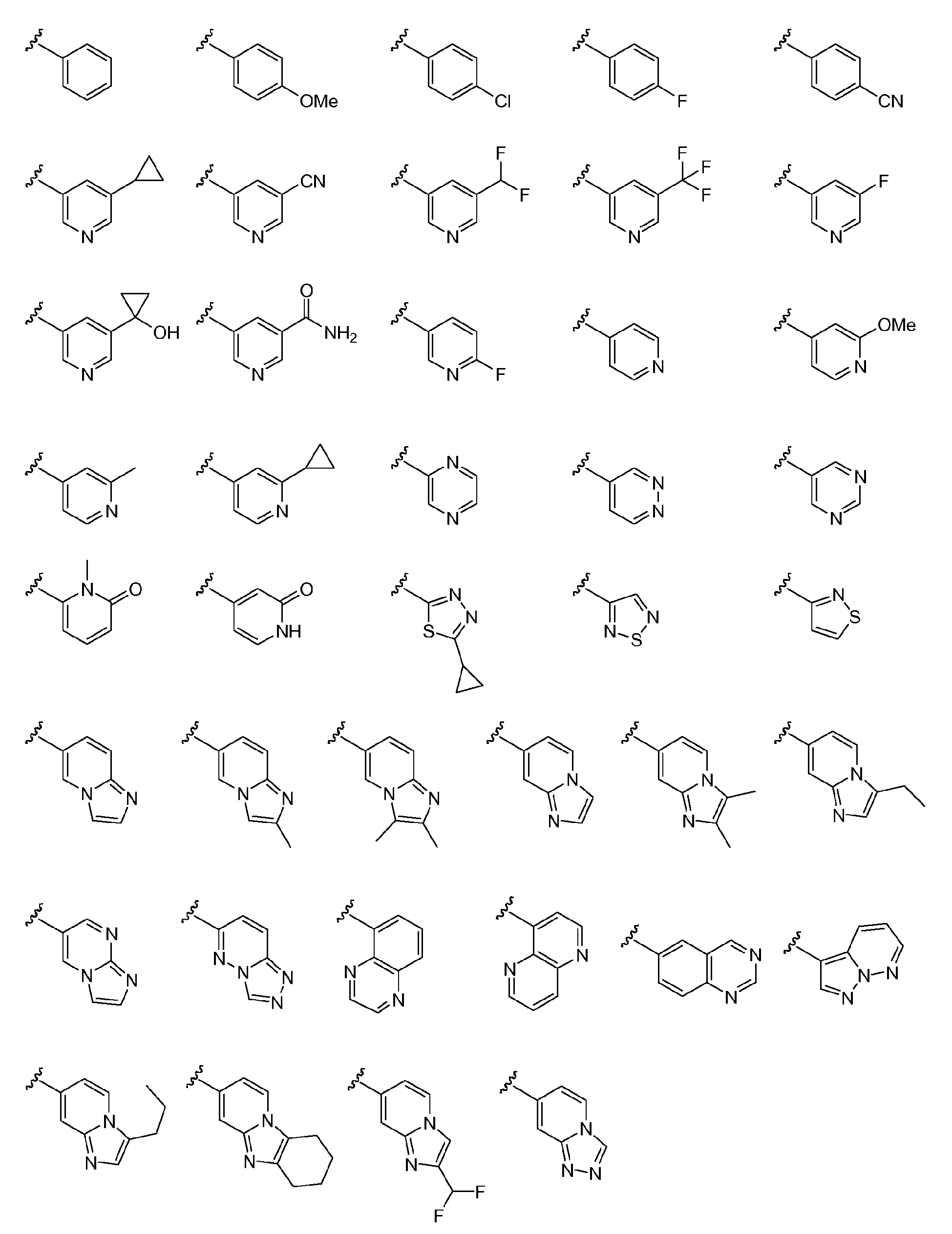

8. The compound or the pharmaceutically-acceptable salt thereof as claimed in claim 7, wherein Ar₃ is selected from the following group:

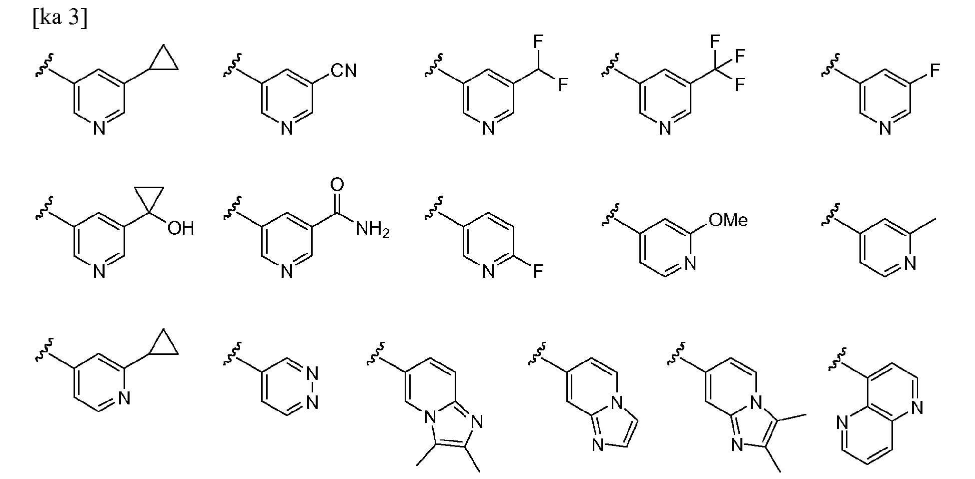

9. The compound or the pharmaceutically-acceptable salt thereof as claimed in any of claims 1 to 8, wherein R² is a hydrogen atom, methyl, ethyl, n-propyl, isopropyl, 2-fluoroethyl, 2,2-difluoroethyl, 2-hydroxyethyl, dimethylcarbamoylmethyl, difluoromethyl, 2-hydroxy-2-methylpropyl, methanesulfonylmethyl or cyanomethyl.

10. The compound or the pharmaceutically-acceptable salt thereof as claimed in claim 9, wherein R² is a hydrogen atom, methyl, ethyl, 2-fluoroethyl, difluoromethyl, 2-hydroxy-2-methylpropyl, methanesulfonylmethyl or cyanomethyl.

11. The compound or the pharmaceutically-acceptable salt thereof as claimed in any of claims 1 to 10, wherein the group of a formula (A): 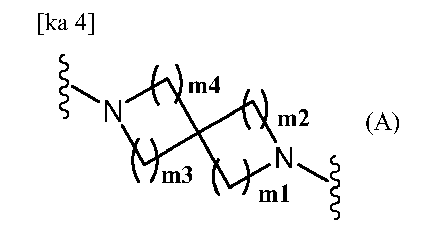 in formula (I) is selected from the following group:

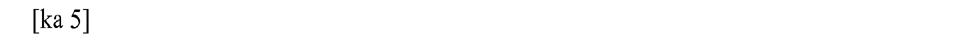

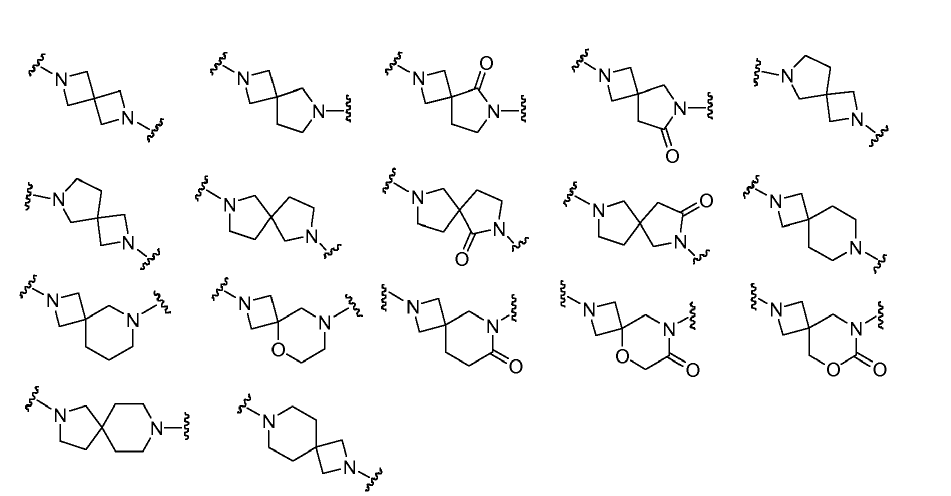

12. The compound or the pharmaceutically-acceptable salt thereof as claimed in claim 11, wherein the group of formula (A) is selected from the following group:

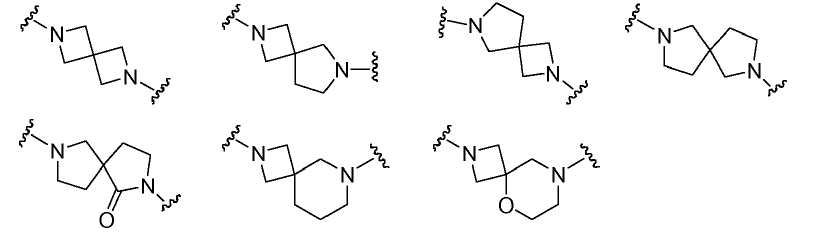

13. The compound or the pharmaceutically-acceptable salt thereof as claimed in claim 1, wherein the compound of formula (I) is selected from the following group:
(E)-(3,4-difluorophenyl){5-[(7-phenyl-2,7-diazaspiro[4.4]non-2-yl)methyl]-2-pyridinyl}methanone O-ethyloxime,
5-{6-[(6-{(E)-(3,4-difluorophenyl)[(ethoxy)imino]methyl}-3-pyridinyl)methyl]-2,6-diazaspiro[3.3]hept-2-yl}-3-pyridinecarbonitrile,
(E)-(5-{[6-(2-cyclopropyl-4-pyridinyl)-2,6-diazaspiro[3.3]hept-2-yl]methyl}-2-pyridinyl)-(3,4-difluorophenyl)methanone O-ethyloxime,
(E)-(3,4-difluorophenyl)(5-{[6-(6-fluoro-3-pyridinyl)-2,6-diazaspiro[3.4]oct-2-yl]methyl}-2-pyridinyl)methanone O-ethyloxime,
(E)-(3,4-difluorophenyl)(5-{[2-(6-fluoro-3-pyridinyl)-2,6-diazaspiro[3.4]oct-6-yl]methyl}-2-pyridinyl)methanone O-ethyloxime,
5-(6-{[6-((E)-(3,4-difluorophenyl){[(2-hydroxy-2-methylpropyl)oxy]imino}methyl)-3-pyridinyl]methyl}-2,6-diazaspiro[3.3]hept-2-yl)-3-pyridinecarbonitrile, (E)-(3,4-difluorophenyl){5-[(6-imidazo[1.2-a]pyridin-7-yl-2,6-diazaspiro[3.3]hept-2-yl)methyl]-2-pyridinyl}methanone O-(2-hydroxy-2-methylpropyl)oxime,
(E)-(3,4-difluorophenyl){5-[(7-pyrazolo[1,5-b]pyridazin-3-yl-2,7-diazaspiro[4.4]non-2-yl)methyl]-2-pyridinyl}methanone O-ethyloxime,
(E)-(3,4-difluorophenyl)(5-{[7-(4-pyridazinyl)-2,7-diazaspiro[4.4]non-2-yl]methyl}-2-pyridinyl)methanone O-ethyloxime,
(E)-(3,4-difluorophenyl)(5-{[6-(3-pyridinyl)-2,6-diazaspiro[3.3]hept-2-yl]methyl}-2-pyridinyl)methanone O-ethyloxime,
(E)-(3,4-difluorophenyl)(5-{[6-(4-pyridinyl)-2,6-diazaspiro[3.3]hept-2-yl]methyl}-2-pyridinyl)methanone O-ethyloxime,
(E)-(3,4-difluorophenyl)(5-{[6-(5-fluoro-3-pyridinyl)-2,6-diazaspiro[3.3]hept-2-yl]methyl}-2-pyridinyl)methanone O-ethyloxime,
(E)-[5-({6-[5-(difluoromethyl)-3-pyridinyl]-2,6-diazaspiro[3.3]hept-2-yl}methyl)-2-pyridinyl](3,4-difluorophenyl)methanone O-ethyloxime,
(E)-(5-{[6-(5-cyclopropyl-3-pyridinyl)-2,6-diazaspiro[3.3]hept-2-yl]methyl}-2-pyridinyl)(3,4-difluorophenyl)methanone O-ethyloxime,
(E)-(3,4-difluorophenyl)[5-({6-[5-(1-hydroxycyclopropyl)-3-pyridinyl]-2,6-diazaspiro[3.3]hept-2-yl}methyl)-2-pyridinyl]methanone O-ethyloxime,
(E)-(3,4-difluorophenyl)(5-{[6-(2-methyl-4-pyridinyl)-2,6-diazaspiro[3.3]hept-2-yl]methyl}-2-pyridinyl)methanone O-ethyloxime,
(E)-(3,4-difluorophenyl)[5-({6-[2-(methyloxy)-4-pyridinyl]-2,6-diazaspiro[3.3]hept-2-yl}methyl)-2-pyridinyl]methanone O-ethyloxime,
(E)-(3,4-difluorophenyl)(5-{[6-(3-isothiazolyl)-2,6-diazaspiro[3.3]hept-2-yl]methyl}-2-pyridinyl)methanone O-ethyloxime,
(E)-(3,4-difluorophenyl)(5-{[6-(1,5-naphthyridin-4-yl)-2,6-diazaspiro[3.3]hept-2-yl]methyl}-2-pyridinyl)methanone O-ethyloxime,
(E)-(3,4-difluorophenyl){5-[(6-imidazo[1,2-a]pyridin-6-yl-2,6-diazaspiro[3.3]hept-2-yl)methyl]-2-pyridinyl}methanone O-ethyloxime,
(E)-(3,4-difluorophenyl)(5-{[6-(2,3-dimethylimidazo[1,2-a]pyridin-6-yl)-2,6-diazaspiro[3.3]hept-2-yl]methyl}-2-pyridinyl)methanone O-ethyloxime,
(E)-(3,4-difluorophenyl){5-[(6-imidazo[1,2-a]pyridin-7-yl-2,6-diazaspiro[3.3]hept-2-ylmethyl]-2-pyridinyl}methanone O-ethyloxime,
(E)-(3,4-difluorophenyl)(5-{[6-(3-propylimidazo[1,2-a]pyridin-7-yl)-2,6-diazaspiro[3.3]hept-2-yl]methyl}-2-pyridinyl)methanone O-ethyloxime,
(E)-(3,4-difluorophenyl)(5-{[6-(6,7,8,9-tetrahydropyrido[1,2-a]benzimidazol-3-yl)-2,6-diazaspiro[3.3]hept-2-yl]methyl}-2-pyridinyl)methanone O-ethyloxime, 5-(6-{[6-((E)-(4-chloro-3,5-difluorophenyl){[(2-hydroxy-2-methylpropyl)oxy]imino}methyl)-3-pyridinyl]methyl}-2,6-diazaspiro[3.3]hept-2-yl)-3-pyridinecarbonitrile,
(E)-(3,4-difluorophenyl)(5-{[(5R) or (5S)-7-(2-methyl-4-pyridinyl)-2,7-diazaspiro[4.4]non-2-yl]methyl}-2-pyridinyl)methanone O-(2-hydroxy-2-methylpropyl)oxime, and
(E)-(3,4-difluorophenyl)(5-{[6-(6-fluoro-3-pyridinyl)-2,6-diazaspiro[3.5]non-2-yl]methyl}-2-pyridinyl)methanone O-ethyloxime.
14 A melanin concentrating hormone receptor antagonist comprising, as the active ingredient thereof, a compound or the pharmaceutically-acceptable salt thereof of any of claims 1 to 13.
15 A pharmaceutical composition containing a pharmaceutically-acceptable additive and a compound or the pharmaceutically-acceptable salt thereof of any of claims 1 to 13.
16 A preventive or remedy comprising, as the active ingredient thereof, a compound or the pharmaceutically-acceptable salt thereof of any of claims 1 to 13 for bulimia, obesity, diabetes, fatty liver, depression or anxiety.
